# EUROPEAN PATENT SPECIFICATION

(11) **EP 3 873 607 B1**
(45) Date of publication and mention of the grant of the patent: **29.11.2023**
(21) Application number: 19797382.9
(22) Date of filing: 28.10.2019
(51) Int. Cl.: A61P 31/18, A61K 31/416, A61K 31/517, C07D 403/14, C07D 405/14

(54) **QUINAZOLINYL-INDAZOLE DERIVATIVES AND THEIR USE AS INHIBITORS OF HUMAN IMMUNODEFICIENCY VIRUS REPLICATION**
CHINAZOLINYLINDAZOLDERIVATE UND IHRE VERWENDUNG ALS INHIBITOREN DER REPLIKATION DES MENSCHLICHEN IMMUNDEFIZIENZVIRUS
DÉRIVÉS DE QUINAZOLINYLE-INDAZOLE ET LEUR UTILISATION EN TANT QU'INHIBITEURS DE LA RÉPLICATION DU VIRUS DE L'IMMUNODÉFICIENCE HUMAINE

(30) Priority: 29.10.2018 US 201862751733 P
(43) Date of publication of application: 08.09.2021
(73) Proprietor: VIIV Healthcare UK (No.5) Limited, Stevenage SG1 2NY (GB)
(72) Inventor: BOWSHER, Michael S., Branford, Connecticut 06405 (US); GILLIS, Eric P, Branford, Connecticut 06405 (US); PARCELLA, Kyle E., Branford, Connecticut 06405 (US)
(74) Representative: Fowler, Gavin James
(86) International application number: PCT/IB2019/059238
(87) International publication number: WO 2020/089778

(56) References cited:
- WO-A1-2013/006738
- WO-A1-2016/033243
- WO-A1-2018/203235
- WO-A1-2019/198024

## Description

### FIELD OF THE INVENTION

The invention relates to compounds, compositions, and methods for the treatment of human immunodeficiency virus (HIV) infection. More particularly, the invention provides novel Capsid inhibitors, pharmaceutical compositions containing such compounds, and methods for using these compounds in the treatment of HIV infection. The invention also relates to methods for making the compounds hereinafter described.

### BACKGROUND OF THE INVENTION

Acquired immunodeficiency syndrome (AIDS) is the result of infection by HIV. HIV continues to be a major global public health issue. In 2015, an estimated 36.7 million people were living with HIV (including 1.8 million children) - a global HIV prevalence of 0.8%. The vast majority of this number live in low- and middle- income countries. In the same year, 1.1 million people died of AIDS-related illnesses.

Current therapy for HIV-infected individuals consists of a combination of approved anti-retroviral agents. Close to four dozen drugs are currently approved for HIV infection, either as single agents, fixed dose combinations or single tablet regimens; the latter two containing 2-4 approved agents. These agents belong to a number of different classes, targeting either a viral enzyme or the function of a viral protein during the virus replication cycle. Thus, agents are classified as either nucleotide reverse transcriptase inhibitors (NRTIs), non-nucleotide reverse transcriptase inhibitors (NNRTIs), protease inhibitors (PIs), integrase strand transfer inhibitors (INSTIs), or entry inhibitors (one, maraviroc, targets the host CCR5 protein, while the other, enfuvirtide, is a peptide that targets the gp41 region of the viral gp160 protein). In addition, a pharmacokinetic enhancer (cobicistat or ritonavir) can be used in combinations with antiretroviral agents (ARVs) that require boosting.

Despite the armamentarium of agents and drug combinations, there remains a medical need for new anti-retroviral agents. High viral heterogeneity, drug-associated toxicity, tolerability problems, and poor adherence can all lead to treatment failure and may result in the selection of viruses with mutations that confer resistance to one or more antiretroviral agents or even multiple drugs from an entire class (Beyrer, C., Pozniak A. HIV drug resistance - an emerging threat to epidemic control. N. Engl. J. Med. 2017, 377, 1605-1607; Gupta, R. K., Gregson J., et al. HIV-1 drug resistance before initiation or re-initiation of first-line antiretroviral therapy in low-income and middle-income countries: a systematic review and meta-regression analysis. Lancet Infect. Dis. 2017, 18, 346-355; Zazzi, M., Hu, H., Prosperi, M. The global burden of HIV-1 drug resistance in the past 20 years. PeerJ. 2018, DOI 10.7717/peerj.4848). As a result, new drugs are needed that are easier to take, have high genetic barriers to the development of resistance and have improved safety over current agents. In this panoply of choices, novel mechanisms of action (MOAs) that can be used as part of the preferred antiretroviral therapy (ART) can still have a major role to play since they should be effective against viruses resistant to current agents.

Certain potentially therapeutic compounds have now been described in the art and set forth in Blair, Wade S. et.al. Antimicrobial Agents and Chemotherapy (2009), 53(12), 5080-5087, Blair, Wade S. et al. PLoS Pathogens (2010), 6(12), e1001220, Thenin-Houssier, Suzie; Valente, Susana T. Current HIV Research, 2016, 14, 270-282, and PCT Patent applications with the following numbers: WO 2012065062, WO 2013006738, WO 2013006792, WO 2014110296, WO 2014110297, WO 2014110298, WO 2014134566, WO 2015130964, WO2015130966, WO 2016033243, WO2018035359, WO2018203235, WO 2019161017, and WO 2019161280.

What is now needed in the art are additional compounds which are novel and useful in the treatment of HIV. Additionally, these compounds should provide advantages for pharmaceutical uses, for example, with regard to one or more of their mechanisms of action, binding, inhibition efficacy, target selectivity, solubility, safety profiles, bioavailability or reduced frequency of dosing. Also needed are new formulations and methods of treatment which utilize these compounds.

### SUMMARY OF THE INVENTION

Briefly, in one aspect, the present invention discloses A compound of Formula I, or a pharmaceutically acceptable salt thereof: 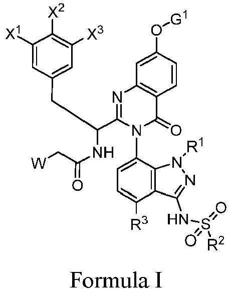 wherein:
G¹ is C₆-C₈alkyl optionally substituted with 1-3 fluorines; or G¹ is one of the following: 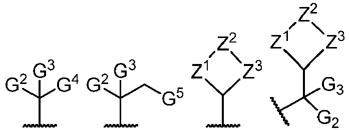
Z¹ is -C₁-C₂alkylene;
Z² is -O-, -S(O₂)-, or -CH₂-;
Z³ is -C₁-C₂alkylene;
G² and G³ are independently selected from H and -CH₃;
G⁴ is phenyl, pyridine, pyrimidine or pyrazine;
G⁵ is G⁴, -OG⁴, -O(C₁-C₂ alkyl optionally substituted with 1-3 fluorines), or G⁵ is one of the following: 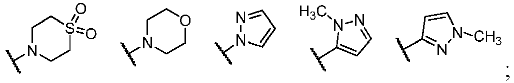
R¹ is hydrogen, C₁-C₃alkyl optionally substituted with 1-3 fluorines, or cyclopropyl optionally substituted with 1-2 fluorines;
R² is C₁-C₂alkyl optionally substituted with 1-3 fluorines, or C₃-C₄ cycloalkyl optionally substituted with 1-2 fluorines;
R³ is hydrogen, Cl, F, CH₃, or OCH₃;
W is selected from: 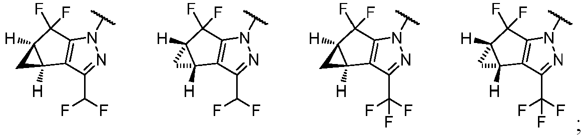
X¹, X² and X³ are independently selected from H, F, and Cl, or one of the group X¹, X² and X³ is selected from -CN, -OCH₃, -CH₃, -CH₂F, -CHF₂, and -CF₃.

In another aspect, the present invention discloses a composition comprising a compound of Formula I or a pharmaceutically acceptable salt thereof.

In another aspect, the present invention discloses a method of treating HIV infection comprising administering a composition comprising a compound of Formula I or a pharmaceutically acceptable salt thereof to a patient.

In another aspect, the present invention discloses a compound of Formula I or pharmaceutically acceptable salt thereof for use in therapy.

In another aspect, the present invention discloses a compound of Formula I or pharmaceutically acceptable salt thereof for use in treating HIV infection.

In another aspect, the present invention discloses the use of a compound of Formula I or pharmaceutically acceptable salt thereof in the manufacture of a medicament for the treatment of HIV infection.

### DETAILED DESCRIPTION OF THE INVENTION

In one embodiment, the present invention discloses compounds of Formula I and pharmaceutically acceptable salts thereof wherein W is the following:

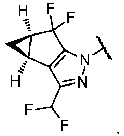

In one embodiment, the present invention discloses compounds of Formula I and pharmaceutically acceptable salts thereof wherein W is the following:

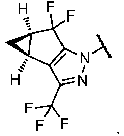

In one embodiment, the present invention discloses compounds of Formula I and pharmaceutically acceptable salts thereof wherein R¹ is -CH₃, -CH₂CHF₂, or -CH₂CF₃; R² is - CH₃ or cyclopropyl; and R³ is H, Cl or CH₃. In another embodiment, the present invention discloses compounds of Formula I and pharmaceutically acceptable salts thereof wherein R¹ is -CH₃; R² is -CH₃; and R³ is Cl.

In one embodiment, the present invention discloses compounds of Formula I and pharmaceutically acceptable salts thereof wherein X¹, X², and X³ are independently selected from H or F. In another embodiment, the present invention discloses compounds of Formula I and pharmaceutically acceptable salts thereof wherein X¹ is F, X² is H, and X³ is F.

In one embodiment, the present invention discloses compounds of Formula I and pharmaceutically acceptable salts wherein G² and G³ are H.

In one embodiment, the present invention discloses compounds of Formula I and pharmaceutically acceptable salts thereof wherein G₁ is the following: 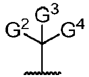 wherein G⁴ is pyridine or pyrimidine.

In one embodiment, the present invention discloses compounds of Formula I and pharmaceutically acceptable salts thereof wherein G₁ is the following:

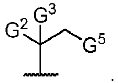

In one embodiment, the present invention discloses compounds of Formula I and pharmaceutically acceptable salts wherein G₁ is the following: 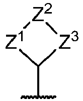 wherein Z² is -O- or -(CH₂)-.

In one embodiment, the present invention discloses compounds of Formula I and pharmaceutically acceptable salts thereof wherein G₁ is the following: 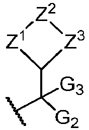 wherein Z² is -(CH₂)-.

In one embodiment, the present invention discloses compounds of Formula I and pharmaceutically acceptable salts thereof wherein G¹ is C₆-C₈alkyl optionally substituted with 1-3 fluorines.

In one embodiment, the present invention discloses compounds of Formula I and pharmaceutically acceptable salts thereof wherein G¹ is one of the following:

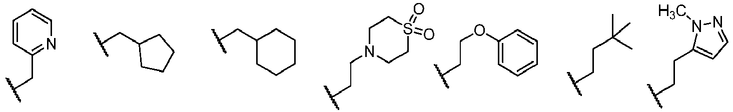

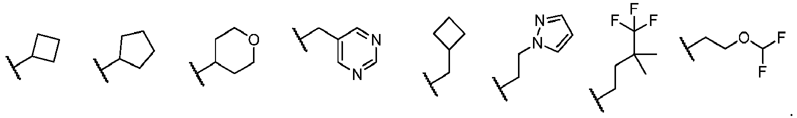

In one embodiment, the present invention discloses compounds of Formula I and pharmaceutically acceptable salts thereof wherein the stereochemistry is as depicted below:

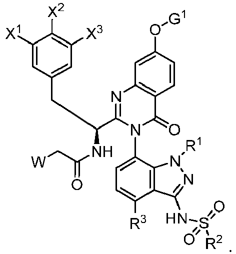

In one embodiment, the present invention discloses compounds of Formula I and pharmaceutically acceptable salts thereof wherein the stereochemistry is as depicted below:

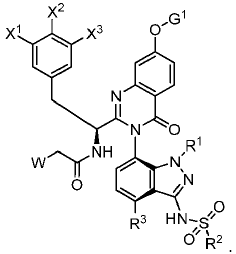

In one embodiment, the present invention discloses compounds and salts selected from the group consisting of: 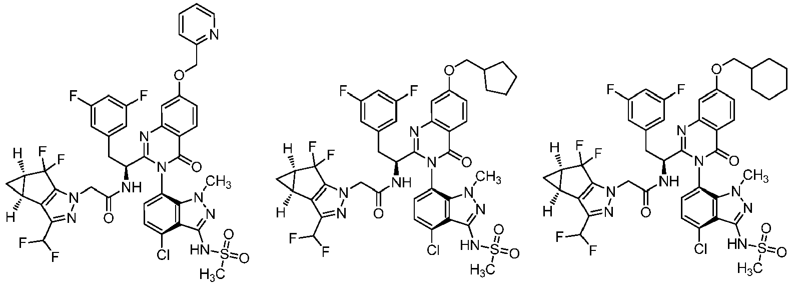 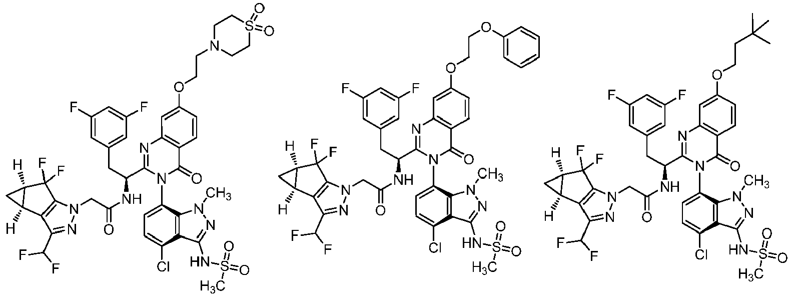 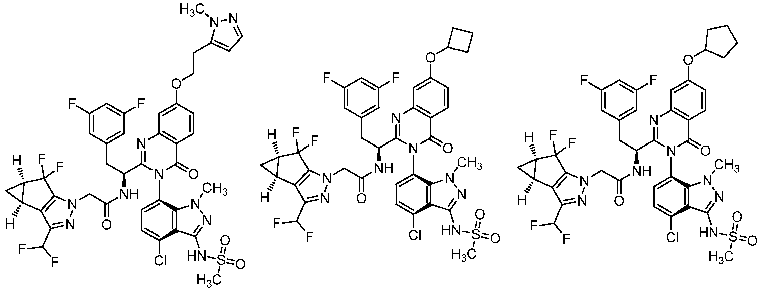 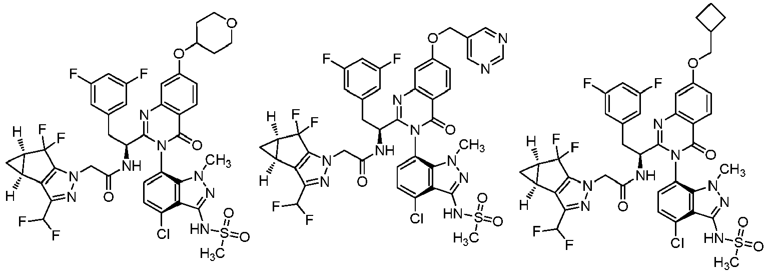 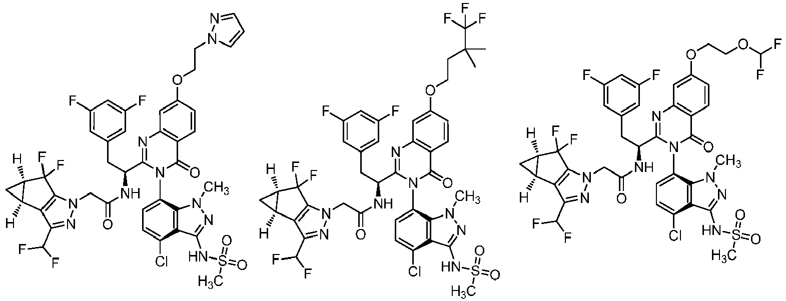 and pharmaceutically acceptable salts thereof.

The salts of compounds of formula I are pharmaceutically acceptable. Such salts may be acid addition salts or base addition salts. For a review of suitable pharmaceutically acceptable salts see Berge et al, J. Pharm, Sci., 66, 1-19, 1977. In an embodiment, acid addition salts are selected from the hydrochloride, hydrobromide, hydroiodide, sulphate, bisulfate, nitrate, phosphate, hydrogen phosphate, acetate, benzoate, succinate, saccharate, fumarate, maleate, lactate, citrate, tartrate, gluconate, camsylate, methanesulfonate, ethanesulfonate, benzenesulfonate, p-toluenesulfonate and pamoate. In an embodiment, base addition salts include metal salts (such as sodium, potassium, aluminium, calcium, magnesium and zinc) and ammonium salts (such as isopropylamine, diethylamine, diethanolamine salts). Other salts (such as trifluoroacetates and oxalates) may be used in the manufacture of compounds of formula I and their pharmaceutically acceptable salts, and are included within the scope of the invention. All possible stoichiometric and non-stoichiometric forms of the salts of compounds of formula I are included within the scope of the invention. Acid and base addition salts may be prepared by the skilled chemist, by treating a compound of formula I with the appropriate acid or base in a suitable solvent, followed by crystallization and filtration.

Some of the compounds of the invention exist in stereoisomeric forms. The invention includes all stereoisomeric forms of the compounds including enantiomers and diastereromers including atropisomers. The term homochiral is used as a descriptor, per accepted convention, to describe a structure which is a single stereoisomer. Absolute stereochemistry was not assigned in all cases. Thus the compound is drawn at the chiral center as unspecified but labelled as homochiral and in the procedures it is identified by its properties such as for example first eluting off a normal or chiral column per the conventions of chemists. It should be noted that the provided experimental procedures teach how to make the exact compound even if not drawn with absolute configuration. Methods of making and separating stereoisomers are known in the art. The invention includes all tautomeric forms of the compounds. The invention includes atropisomers and rotational isomers.

For the compounds of Formula I, the scope of any instance of a variable substituent can be used independently with the scope of any other instance of a variable substituent. As such, the invention includes combinations of the different aspects. In some examples, the stereochemistry of all the centers were not unambiguously assigned so they can be referred to as diastereomer 1 and diastereomer 2 or enantiomer 1 or enantiomer 2 etc. and these are understood by chemists skilled in the art. In other cases, atropisomers can be observed and these are understood to convert at slow or fast rates or even not at all depending on the conditions for handling the compound. These are referred to as mixtures of atropisomers where they interconvert at ambient temperatures or as atropisomer 1 and atropisomer 2 where they were isolated. Since the compounds are identified by their properties rather than exact structural assignment from a crystal structure, it is understood in the art that where not specified, atropisomers are covered and inferred to be covered by the chemical structure.

In the method of this invention, preferred routes of administration are oral and by injection to deliver subcutaneously (SC) or intramuscularly (IM). Therefore, preferred pharmaceutical compositions include composition suitable for oral administration (for example tablets) and formulations suitable for injection.

The compounds of this invention are believed to act as Capsid Inhibitors.

The compounds of the present invention and their salts, solvates, or other pharmaceutically acceptable derivatives thereof, may be employed alone or in combination with other therapeutic agents. The compounds of the present invention and any other pharmaceutically active agent(s) may be administered together or separately and, when administered separately, administration may occur simultaneously or sequentially, in any order. The amounts of the compounds of the present invention and the other pharmaceutically active agent(s) and the relative timings of administration will be selected in order to achieve the desired combined therapeutic effect. The administration in combination of a compound of the present invention and salts, solvates, or other pharmaceutically acceptable derivatives thereof with other treatment agents may be in combination by administration concomitantly in: (1) a unitary pharmaceutical composition including multiple compounds; or (2) separate pharmaceutical compositions each including one of the compounds. Alternatively, the combination may be administered separately in a sequential manner wherein one treatment agent is administered first and the other second or vice versa, and the different agents could be administered on different schedules if appropriate. Such sequential administration may be close in time or remote in time. The amounts of the compound(s) of Formula I or salts thereof and the other pharmaceutically active agent(s) and the relative timings of administration will be selected in order to achieve the desired combined therapeutic effect.

As such, the compounds of the present invention may be used in combination with one or more agents useful in the prevention or treatment of HIV.

### EXAMPLES

### General Procedure A:

In the below procedure, N-((S)-1-(3-(4-chloro-3-(N-(4-methoxybenzyl)methylsulfonamido)-1-methyl-1H-indazol-7-yl)-7-hydroxy-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide ("acetamide") is the limiting reagent and all equivalents are in reference to this amount. To a stirred solution of N-((S)-1-(3-(4-chloro-3-(N-(4-methoxybenzyl)methylsulfonamido)-1-methyl-1H-indazol-7-yl)-7-hydroxy-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (1 equiv, typically 25 mg), the indicated alcohol (3 equiv), and triphenylphosphine (3 equiv) in THF (volume necessary to achieve a 0.11M concentration in acetamide) was added a solution of diisopropyl (E)-diazene-1,2-dicarboxylate ("DIAD", 3 equiv) in THF (16.1 mg DIAD/0.25 mL THF, 5.1 M). The total reaction volume was approximately 0.50 mL when conducted using 25 mg of acetamide. The solution was stirred at room temperature either overnight (~18h), 72 h (time not optimized), or until the reaction was deemed complete by LCMS. The volatiles were evaporated under a N₂ (g) stream and the resulting residue was then placed under high vacuum for 10 minutes. The crude residue was taken up in DCM:TFA (1:1) to achieve a concentration of 0.1 1M based on the acetamide input used above. To the solution was added triflic acid (3 equiv) and resulting solution was stirred at room temperature for 10 minutes. The reaction volatiles were evaporated under reduced pressure. The residue was taken up in EtOAc and washed with aq. K₃PO₄ (0.75 M). The organic layer was isolated and then concentrated under a stream of N₂ (g). The resulting residue was subjected to HPLC purification to afford the indicated product.

### LCMS Method F:

Column: Acquity BEH C18, 2.1 × 30 mm, 1.7 µm particles; Solvent A = 0.1% Formic acid in 100% Water. Solvent B = 0.1% Formic Acid in 100% Acetonitrile. Flow Rate = 0.8 mL/min. Start % B = 5. Final % B = 95. Gradient Time = 1.7 min, then a 0.2 min hold at 95% B. Wavelength = 215 and 254 nm.

### HPLC purification:

HPLC purification was performed using one of the conditions indicated below, optionally followed by a second HPLC purification using a different condition indicated below. Based on analytical HPLC data obtained on the crude reaction mixture, the purification condition was optimized for each target compound by modifying the initial Solvent A: Solvent B ratio, the gradient time, the final Solvent A: Solvent B ratio, and the hold time at the final Solvent A: Solvent B concentration.

HPLC Condition A: Column: Zorbax Eclipse Plus C18, 21.2 × 100 mm, 5 µm particles; Solvent A = 0.1% Formic Acid in 100% Water. Solvent B = Acetonitrile. Flow Rate = 40 mL/min. Wavelength = 215 and 254 nm. ESI+ Range: 150 to 1500 dalton.

HPLC Condition B: Column: Sunfire prep C18 OBD, 30 × 100 mm, 5 µm particles; Solvent A: water:MeCN 95:5 w/ 0.1% TFA, Solvent B: MeCN:water 95:5 w/ 0.1% TFA. Flow Rate = 42 mL/min. Wavelength = 220 and 254 nm.

HPLC Condition C: Column: Waters Xterra C18, 19 × 100 mm, 10 µm particles; Solvent A = 0.1% NH4OH in 100% Water. Solvent B = Acetonitrile. Flow Rate = 40 mL/min. Wavelength = 215 and 254 nm. ESI + Range: 150 to 1500 dalton.

### Preparation of 3-bromo-6-chloro-2-fluorobenzaldehyde

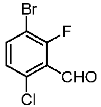

To a stirred solution of 1-bromo-4-chloro-2-fluorobenzene (200 g, 0.955 mol, 1.0 equiv.) in anhydrous THF (2.0 L) was added a solution of LDA in THF (2.0 M, 620 mL, 1.24 mol, 1.3 equiv.) at -50 °C. The reaction mixture was allowed to warm to -20 °C and was stirred for 1 h. The mixture was cooled to -50 °C and slowly to the mixture was added DMF (184.8 mL, 2.48 mol, 2.6 equiv.) maintaining a temperature of -50 °C. The mixture was allowed to warm to 0 °C and was stirred for 30-45 min at the same temperature (0 °C). The mixture was quenched via the slow addition of ice cold water (2.0 L). The reaction mixture was diluted with ethyl acetate (2.0 L) and stirred for 15 min at room tempreature. The organic layer was separated and reserved; the aqueous layer was extracted with ethyl acetate (2 × 1.0 L). The combined organic layers were washed with water (2 × 1.0 L); 1.0 N HCl (1.0 L) and then 15% NaCl solution (2.0 L). The organic solution was dried over Na₂SO₄; filtered; and then concentrated *in vacuo.* The resultant crude solid was used directly in the next step without further purification. Yield for the crude product: 210.0 g (93%).

### Preparation of 3-bromo-6-chloro-2-fluorobenzonitrile

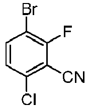

To a stirred solution of 3-bromo-6-chloro-2-fluorobenzaldehyde (210.0 g, 0.89 mol, 1.0 equiv.) in water (2.1 L) at room temperature was added hydroxylamine-O-sulfonic acid (175.15 g, 1.55 mol, 1.75 equiv.). The reaction mixture was heated to 50 °C and stirred for 18 h). The mixture was cooled to room temperature and stirred for 1-1.5 h. The solids were isolated via filtration and were then washed with water. The wet solid was dried under vacuum at 50 °C for 12-15 h to afford 3-bromo-6-chloro-2-fluorobenzaldehyde, 190.0 g (91%).

### Preparation of 7-bromo-4-chloro-1H-indazol-3-amine

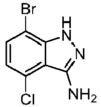

To a 3 L three neck round bottom flask fitted with a water-cooled condenser, a thermometer and a mechanical stirrer was added 3-bromo-6-chloro-2-fluorobenzonitrile (100 g, 427 mmol) and ethanol (500 mL). To the solution was added hydrazine hydrate (104 ml, 2133 mmol) at room temperature. The solution was heated to 80 °C and was maintained at that temperature for 1 h upon which the mixture became a homogeneous solution and LCMS analysis indicated the reaction was complete. The solution was allowed to cool to 45 °C and then water (1L) was added slowly to produce a white ppt. as a thick slurry. Following the addition the mixture was stirred for 30 minutes. The solids were isolated via filtration. The solids were washed with water (1L) and then dried under vacuum at 45 °C to afford 7-bromo-4-chloro-1H-indazol-3-amine as a pale orange solid, 103 g (98%). ¹H NMR (400MHz, DMSO-d6): *δ* 12.21 (bs, 1H), 7.41 (d, *J=* 7.8 Hz, 1H), 6.84 (d, *J=* 7.8 Hz, 1H), 5.34 (bs, 2H) ppm.

### Preparation of 7-bromo-4-chloro-1-methyl-1H-indazol-3-amine

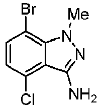

To a solution of 3-bromo-6-chloro-2-fluorobenzonitrile (360.0 g, 1.55 mol, 1.0 equiv.) in ethanol (1.08 L) was added methylhydrazine sulphate (1.11 kg, 7.73 mol, 5.0 equiv.) followed by the addition of triethylamine (1.3 L, 9.3 mol, 6.0 equiv.) at 25-35 °C. The reaction mixture was heated to 110 °C and maintained at that temperature for 15 h. The mixture was cooled to room temperature and to the mixture was added water (3.0 L). The mixture was stirred at room temperature for 1 h. The solids were isolated via filtration and were washed with water. The wet solid was dried under vacuum at 50 °C for 12-15 hours. The material was subjected to silica gel column chromatography (hexanes:EtOAc 90:10 → 60:40) to afford 7-bromo-4-chloro-1-methyl-1H-indazol-3-amine as a pale yellow solid, 185.0 g (46 %).

### Preparation of 7-bromo-4-chloro-1-(2,2-difluoroethyl)-1H-indazol-3-amine

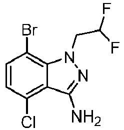

To a stirred solution of 7-bromo-4-chloro-1*H*-indazol-3-amine (128.0 g, 0.52 mol, 1.0 equiv.) in dry THF (1.92 L) at 0 °C was added *^{t}*BuOK (76 g, 0.67 mol, 1.3 equiv.) in portions. The reaction mixture was stirred for 10 min at 0 °C; then to the solution was slowly added 2,2-difluoroethyl trifluoro-methanesulfonate (122.5 g, 0.57 mol, 1.1 equiv.) at 0 °C. The mixture was slowly warmed to room temperature and then was stirred for 2 h. The mixture was diluted with ice-cold water (3.0 L) and MTBE (2 × 1.5 L). The organic layer was separated, washed with water (2 × 1.2 L), dried over Na₂SO₄, filtered, and then concentrated *in vacuo.* The resulting crude material was subjected to silica gel chromatography (hexanes:EtOAc 95:5→90:10). Product-containing fractions contaminated with the undesired regioisomer were concentrated and then triturated with DCM (5 mL/g) to afford the pure desired product which was then combined with fractions of the pure material. This process afforded 7-bromo-4-chloro-1-(2,2-difluoroethyl)-1H-indazol-3-amine as a light yellow solid, 110 g (68%). ¹H NMR (DMSO-d₆, 500 MHz) δ 7.55 (d, 1H, J=7.9 Hz), 6.96 (d, 1H, J=7.9 Hz), 6.1-6.5 (m, 1H), 5.62 (s, 2H), 4.94 (dt, 2H, J=3.8, 14.1 Hz).

### Preparation of 7-bromo-4-chloro-1-(2,2,2-trifluoroethyl)-1H-indazol-3-amine

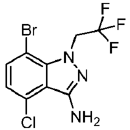

To a stirred solution of 7-bromo-4-chloro-1H-indazol-3-amine (70 g, 284 mmol, 1.0 equiv.) in dry DMF (700 mL) at room temperature was added in portions Cs₂CO₃ (184 g, 568 mmol, 2 equiv.). The reaction mixture was stirred for 10 min at room temperature. To the reaction mixture was added slowly at room temperature 2,2,2-trifluoroethyl trifluoromethanesulfonate (72.5 g, 312 mmol, 1.10 equiv.). After completion of the reaction (monitored by TLC), the mixture was diluted with ice cold water (700 mL) upon which a precipitate was formed.The mixture was allowed to warm to room temperature and then was stirred for 30 minutes at room temperature. The solids were isolated via filtration and then were washed with water (500 mL). The wet product was dissolved in DMF (350 mL) and then was diluted with water (350 mL) at room temperature. The mass was stirred for 30 min., then the solids were collected via filtration and were washed with water (200 mL) followed by hexanes (700 mL). The wet solids were dried under vacuum at 50-55 °C for 18-20 h to afford 7-bromo-4-chloro-1-(2,2,2-trifluoroethyl)-1H-indazol-3-amine (4) as a light yellow solid, 64.0 g (69%).

### Preparation of N-(7-bromo-4-chloro-1-methyl-1H-indazol-3-yl)methanesulfonamide

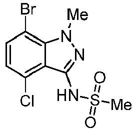

To a solution of 7-bromo-4-chloro-1-methyl-1H-indazol-3-amine (90 g, 0.34 mol, 1.0 equiv.) in CH₂Cl₂ (900 mL) was added diisopropylethylamine ("DIPEA", 180.4 mL, 1.04 mol, 3.0 equiv.) and 4-dimethylaminopyridine ("DMAP", 2.07 g, 0.017 mol, 0.05 equiv.). The mixture was stirred for 5 min, then was cooled to 0 °C and methanesulfonyl chloride (67.7 mL, 0.87 mol, 2.5 equiv.) was added resulting in a noted exotherm. The reaction mixture was warmed to room temperature and stirred at that temperature 3 h upon which a precipitate formed. The mixture was diluted with dichloromethane (1.0 L) and then was washed with water (2.0 L) followed by aq. HCl (1.0M, 1.0 L), and then brine (1.5 L). The organic solution was dried over Na₂SO₄; filtered, and then concentrated *in vacuo.* The crude residue was dissolved in EtOH (1.8 L). To the solution was added aq. NaOH (20%, 650 mL) at room temperature upon which a slight exotherm was noted. The resulting mixture was stirred for 2 h upon which the mixture became a homogeneous solution. The solution was diluted with water (2.0 L) and the pH was adjusted to pH 2-3 using aq. HCl (1.0M, app. 3.0 L). The precipitate that was formed was collected by filtration. The solids were washed with water and then dried *in vacuo* to afford N-(7-bromo-4-chloro-1-methyl-1H-indazol-3-yl)methanesulfonamide as an off-white solid, 96 g (82%). ¹H NMR (500 MHz, CDCl₃) δ 7.48 (d, J=7.9 Hz, 1H), 7.24 (br s, 1H), 6.95 (d, J=7.9 Hz, 1H), 4.38 (s, 3H), 3.42 (s, 3H). LC/MS (M+H)⁺ = 337.80.

### Preparation of N-(7-bromo-4-chloro-1-(2,2-difluoroethyl)-1H-indazol-3-yl)methanesulfonamide

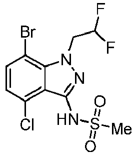

To a stirred solution of 7-bromo-4-chloro-1-(2,2-difluoroethyl)-1H-indazol-3-amine (40.0 g, 0.12 mol, 1.0 equiv.) in dry DCM (400 mL) was added DIPEA (67 mL, 0.38 mol, 3.0 equiv.) and DMAP (0.78 g, 0.0064 mol, 0.05 equiv.). The solution was stirred for 5 min, then the reaction mixture was cooled to 0 °C and to the mixture was slowly added methanesulfonyl chloride (31.0 mL, 0.38 mol, 3.0 equiv.). The reaction mixture was allowed to warm to room temperature and was then stirred for 2 h. After completion of the reaction (monitored by TLC), the mixture was diluted with DCM (2 × 2.5 L) and water (2.0 L). The organic layer was separated and was washed with water (2 × 1.5 L); brine (1.5 L); dried over Na₂SO₄; filtered; and was concentrated *in vacuo.* The residue was dissolved in ethanol (320 mL) and to the solution was aq. NaOH (20% w/w, 320 mL). The reaction mixture was stirred at room temperature for 2 h. After completion of the reaction (monitored by TLC), the mixture was diluted with water (1.0 L) and acidified to pH 2-3 using aq. HCl (1.0 M). The resulting solids were collected via filtration. The solids were triturated with hexanes:EtOAc (95:5, 10 V) and again isolated via filtration. The wet solids were dried under vacuum at 50 °C to afford N-(7-bromo-4-chloro-1-(2,2-difluoroethyl)-1H-indazol-3-yl)methane sulfonamide (5) as a light yellow solid, 45.7 g (91%). ¹H NMR (400 MHz, CDCl₃): δ 7.52 (d, *J=* 8.0 Hz, 1H), 7.41 (bs, 1H), 7.00 (d, *J=* 8.0 Hz, 1H), 6.16 (tt, *J₁* = 4.3 Hz, *J₂* = 8.6 Hz, *J₃* = 55.4 Hz, 1H), 5.15 (td, *J₁* = 4.3 Hz, *J₂* = 12.7 Hz, 2H), 3.41 (s, 3H).

### Preparation of N-(7-bromo-4-chloro-1-(2,2-difluoroethyl)-1H-indazol-3-yl)cyclopropanesulfonamide

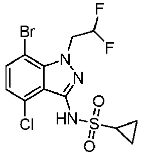

To a stirred solution of 7-bromo-4-chloro-1-(2,2-difluoroethyl)-1H-indazol-3-amine (10 g, 0.032 mol, 1.0 equiv.) in dry pyridine (100 mL) was added cyclopropylsulfonyl chloride (18.1 g, 0.128 mol, 4.0 equiv.). The reaction mixture was stirred at room temperature for 48 h. The mixture was diluted with water (400 mL) and extracted with MTBE (2 × 100 mL). The combined organic layers were washed with water (3 × 300 mL), brine (300 mL), dried over Na₂SO₄, filtered and concentrated *in vacuo.* The resulting crude material was triturated with hexanes (15 V) to obtain *N*-(7-Bromo-4-chloro-1-(2,2-difluoroethyl)-1*H-*indazol-3-yl)cyclopropanesulfonamide as a light-red solid, 11.1 g (82%).

### Preparation of N-(7-bromo-4-chloro-1-(2,2,2-trifluoroethyl)-1H indazol-3-yl)methanesulfonamide

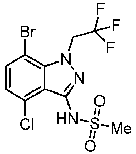

To a stirred solution of 7-bromo-4-chloro-1-(2,2,2-trifluoroethyl)-1H-indazol-3-amine (60 g, 182.64 mmol, 1.0 equiv.) in dry DCM (600 mL, 10 V) was added DIPEA (94.8 ml, 547.92 mmol, 3.0 equiv.) and DMAP (1.11 g, 9.13 mmol, 0.05 equiv.). After being stirring for 15 min the solution was cooled to 0 °C. To the solution was slowly added methanesulfonyl chloride (52.3 g, 456.6 mmol, 3.0 equiv.). The reaction mixture was then allowed to warm to room temperature, and was stirred at room temperature for 2 h. The progress of the reaction (bis-mesylation) was monitored by TLC. After the reaction was determined to be complete the mixture was diluted with DCM (200 mL) and water (200 mL). The organic layer was isolated and washed with water (500 mL), brine (300 mL), dried over Na₂SO₄, filtered and concentrated *in vacuo.* The resulting residue was dissolved in ethanol (600 mL) and to the solution was aq. NaOH (20% w/w, 600 mL). The reaction mixture was stirred for 2 h at room temperature. After completion of the reaction (mono demesylation, monitored by TLC) the solution was diluted with water (300 mL) and acidified to pH 2-3 using aq. HCl (1.0 M). The resulting solids were isolated via filtration and were then washed with water. The solids were dried under vacuum at 50-55 °C. The solid material was further purified by trituration using hexanes:EtOAc (95:5, 15V) to afford *N*-(7-Bromo-4-chloro-1-(2,2,2-trifluoroethyl)-1*H*-indazol-3-yl)methanesulfonamide as a light yellow solid, 55.1 g (75%).

### Preparation of N-(7-bromo-4-chloro-1-methyl-1H-indazol-3-yl)-N-(4-methoxybenzyl)methanesulfonamide

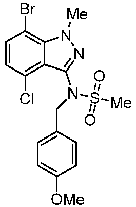

To a mixture of *N*-(7-bromo-4-chloro-1-methyl-1*H*-indazol-3-yl)methanesulfonamide (49 g, 0.144 mol, 1.0 equiv.) in DMF (980 mL) was added 1-(chloromethyl)-4-methoxybenzene (23.54 mL, 0.17 mol, 1.2 equiv.). To the mixture was added cesium carbonate (61.3 g, 0.18 mol, 1.3 equiv.). The mixture was heated to 80 °C and maintained at that temperature for 2 h. After completion of the reaction (monitored by TLC) the mixture was poured into water (2.0 L). The mixture was extracted with EtOAc (2 × 1.5 L). The combined organic layers were washed with brine (1.0 L); dried over Na₂SO₄; filtered and then concentrated in vacuo. The residue was crystallised from hexanes:EtOAc (9:1, 120 mL) to afford the desired product *N*-(7-Bromo-4-chloro-1-methyl-1*H*-indazol-3-yl)-*N*-(4-methoxybenzyl) methane sulfonamide as a white solid. Yield: 62 g (94%). ¹H NMR (500 MHz, CDCl₃) δ 7.44 (d, J=7.9 Hz, 1H), 7.31 (d, J--8.5 Hz, 2H), 6.99 (d, J=7.9 Hz, 1H), 6.84 (d, J=8.5 Hz, 2H), 4.99 (br s, 1H), 4.76 (br s, 1H), 4.40 (s, 3H), 3.80 (s, 3H), 3.01 (s, 3H).

### Preparation of N-(7-bromo-4-chloro-1-(2,2-difluoroethyl)-1H-indazol-3-yl)-N-(4-methoxybenzyl)methanesulfonamide

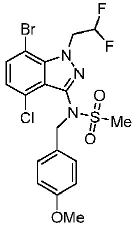

To a stirred solution of *N*-(7-bromo-4-chloro-1-(2,2-difluoroethyl)-1*H*-indazol-3-yl)methanesulfonamide (45.7 g, 0.117 mol, 1.0 equiv.) and 1-(chloromethyl)-4-methoxybenzene (22.1 g, 0.141 mol, 1.2 equiv.) in DMF (460 mL, 10 V) was added cesium carbonate (49.8 g, 0.152 mol, 1.3 equiv.). The reaction mixture was heated to 80 °C and stirred for 2 h at the same temperature. After completion of the reaction (monitored by TLC), the mixture was cooled to room temperature and then poured into water (2.0 L). The mixture was extracted with EtOAc (2 × 1.5 L). The combined organic layers were washed with brine (1.0 L), dried over Na₂SO₄, filtered and concentrated *in vacuo.* The resulting crude material was subjected to silica gel column purification (eluting with hexanes:EtOAc 85:15 → 75:25) to afford N-(7-bromo-4-chloro-1-(2,2-difluoroethyl)-1H-indazol-3-yl)-N-(4-methoxy benzyl)methanesulfonamide as a light yellow solid, 56 g (93%).

### Preparation of N-(7-bromo-4-chloro-1-(2,2-difluoroethyl)-1H-indazol-3-yl)-N-(4-methoxybenzyl)cyclopropanesulfonamide

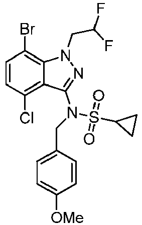

To a stirred mixture of *N*-(7-bromo-4-chloro-1-(2,2-difluoroethyl)-1H-indazol-3-yl)cyclo- propanesulfonamide (15 g, 0.036 mol, 1.0 equiv.) and 1-(chloromethyl)-4-methoxybenzene (6.79 g, 0.043 mol, 1.2 equiv.) in DMF (150 mL) was added cesium carbonate (15.32 g, 0.047 mol, 1.3 equiv.). The reaction mixture was heated to 80 °C and stirred at that temperature for 2 h. After completion of the reaction (monitored by TLC), the mixture was poured into water (300 mL) and the product was extracted with MTBE (2 × 200 mL). The combined organic layers were washed with brine (300 mL), dried over Na₂SO₄, filtered and concentrated *in vacuo.* The resulting crude material was subjected to silica gel column purification (hexanes:EtOAc 80:20 → 75:25) to afford N-(7-Bromo-4-chloro-1-(2,2-difluoroethyl)-1H-indazol-3-yl)-*N*-(4-methoxybenzyl)methanesulfonamide as a gummy liquid, 16.5 g (86%).

### Preparation of N-(7-bromo-4-chloro-1-(2,2,2-trifluoroethyl)-1H-indazol-3-yl)-N-(4-methoxybenzyl)methanesulfonamide

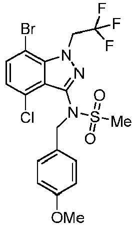

To a stirred solution of *N*-(7-Bromo-4-chloro-1-(2,2,2-trifluoroethyl)-1*H*-indazol-3-yl)methanesulfonamide (6.0 g, 14.77 mmol, 1.0 equiv.) in dry DMF (60 mL, 10 V) at room temperature was added in portions Cs₂CO₃ (6.25 g, 19.20 mmol, 1.3 equiv.). The mixture was stirred for 10 min at room temperature, then to the mixture was slowly added 1-(chloromethyl)-4-methoxybenzene (2.77 g, 17.724 mmol, 1.2 equiv.). The reaction mixture was heated to 80 °C and maintained at that temperature for 2 h. After completion of the reaction (monitored by TLC), the mixture was cooled to room temperature and then was diluted with ice cold water (60 mL) and ethyl acetate (60 mL). The organic layer was isolated; washed with water (40 mL); dried over Na₂SO₄; filtered and concentrated *in vacuo.* The resulting crude material was triturated using hexanes:EtOAc (97:3, 15V) to afford N-(7-bromo-4-chloro-1-(2,2,2-trifluoroethyl)-1*H*-indazol-3-yl)-*N*-(4-methoxybenzyl)methanesulfonamide as a light yellow solid, 7.0 g (90%).

### Preparation of N-(7-amino-4-chloro-1-methyl-1H-indazol-3-yl)-N-(4-methoxybenzyl)methanesulfonamide

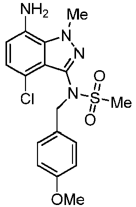

To a stirred solution of *N*-(7-Bromo-4-chloro-1-methyl-1*H-*indazol-3-yl)-*N*-(4-methoxybenzyl) methanesulfonamide (55 g, 0.12 mol, 1.0 equiv.) in NMP (900 mL) at room temperature was added copper (I) iodide (4.57 g, 0.024 mol, 0.2 equiv.), sodium ascorbate (47.4 g, 0.24 mol, 2 equiv.) and (1R, 2R)-*N*₁,*N*₂-dimethylcyclohexane-1,2-diamine (8.52 g, 0.06 mol, 0.5 equiv.) were added at room temperature. Then a solution of sodium azide (23.3 g, 0.36 mol, 3.0 equiv.) in water (182 mL). The mixture was heated to 100 °C and maintained at that temperature for 12 h. The reaction mixture was cooled to room temperature and diluted with ethyl acetate (1.5 L), then filtered through a pad of Celite. The filter pad was extracted with EtOAc (500 mL). The combined filtrate was diluted with water (2.0 L) and the organic layer was isolated and reserved. The aqueous phase was extracted with EtOAc (2 × 1.0 L). The combined organic layers were washed with water (1.0 L); brine (1.0 L); dried over Na₂SO₄; filtered; and concentrated *in vacuo.* The crude material was purified by silica column chromatography (hexanes:EtOAc 100:0→80:20) to afford the title compound, N-(7-Amino-4-chloro-1-methyl-1*H*-indazol-3-yl)-*N*-(4-methoxybenzyl) methanesulfonamide, as an off-white solid, 27.0 g (57%). ¹H NMR (400 MHz, CDCl₃) δ 7.33 - 7.29 (m, 2H), 6.89 (d, J--7.8 Hz, 1H), 6.85 - 6.79 (m, 2H), 6.48 (d, J--7.8 Hz, 1H), 5.11 (br.s, 1H), 4.81 (br.s, 1H), 4.30 (s, 3H), 3.80 (br s, 2H), 3.79 (s, 3H), 2.99 (s, 3H). LC/MS (M+H)⁺ = 395.00.

### Preparation of N-(7-amino-4-chloro-1-(2,2-difluoroethyl)-1H-indazol-3-yl)-N-(4-methoxybenzyl)methanesulfonamide

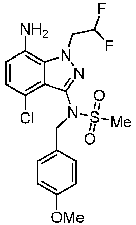

To a stirred solution of *N*-(7-bromo-4-chloro-1-(2,2-difluoroethyl)-1*H-*indazol-3-yl)-N-(4-methoxybenzyl)methanesulfonamide (62 g, 0.12 mol, 1.0 equiv.) in NMP (745 mL) at room temperature was added copper (I) iodide (4.64 g, 0.024 mol, 0.2 equiv.), sodium ascorbate (48.3 g, 0.24 mol, 2 equiv.) and (1R, 2R)-*N*₁,*N*₂-dimethylcyclohexane-1,2-diamine (8.7 g, 0.06 mol, 0.5 equiv.). To the mixture was added a solution of sodium azide (23.8 g, 0.36 mol, 3.0 equiv.) in water (204 mL). The mixture was heated to 100 °C and maintained at that temperature for 15 h. The mixture was cooled to room temperature and was then diluted with ethyl acetate (1.5 L). The mixture was filtered through a pad of Celite and the filter pad was extracted with EtOAc (500 mL). The combined filtrate was diluted with water (2.0 L), organic layer was separated and aqueous layer extracted with EtOAc (2 × 1.0 L). The combined organic layers were washed with water (1.2 L), brine (1.0 L), dried over Na₂SO₄, filtered and then concentrated *in vacuo.* The resulting residue was subjected to silica gel column chromatography (hexanes:EtOAc 100:0→75:25) to afford the title compound, N-(7-amino-4-chloro-1-(2,2-difluoroethyl)-1*H*-indazol-3-yl)-*N*-(4-methoxybenzyl)methanesulfonamide, as an off-white solid, 23.0 g, (44%).

### Preparation of N-(7-amino-4-chloro-1-(2,2-difluoroethyl)-1H-indazol-3-yl)-N-(4-methoxybenzyl)cyclopropanesulfonamide

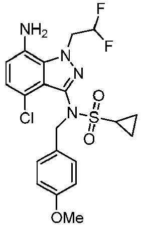

To a stirred solution of *N*-(7-bromo-4-chloro-1-(2,2-difluoroethyl)-1*H*-indazol-3-yl)-N-(4-methoxybenzyl)cyclopropanesulfonamide (32 g, 0.059 mol, 1.0 equiv.) in NMP (512 mL) at room temperature was added copper (I) iodide (2.27 g, 0.012 mol, 0.2 equiv.), sodium ascorbate (23.7 g, 0.12 mol, 2 equiv.) and (1R, 2R)-*N*₁,*N*₂-dimethylcyclohexane-1,2-diamine (4.25 g, 0.03 mol, 0.5 equiv.). To the mixture was added a solution of sodium azide (11.6 g, 0.18 mol, 3.0 equiv.) in water (112 mL). The reaction was heated to 100 °C and stirred for 18 h the same temperature. The mixture was cooled to room temperature and diluted with ethyl acetate (1.2 L). The mixture was filtered through a pad of Celite, extracting with EtOAc (300 mL). The combined filtrate was poured into water (1.5 L) and the organic layer was isolated and reserved. The aqueous layer was extracted with EtOAc (2 × 0.8 L). The combined organic layers were washed with water (0.8 L), brine (0.8 L), dried over Na₂SO₄, filtered and then concentrated *in vacuo.* The crude residue was subjected to silica gel column chromatography (hexanes:EtOAc 100:0→80:20) to afford the title compound, *N*-(7-amino-4-chloro-1-(2,2-difluoroethyl)-1*H*-indazol-3-yl)-*N*-(4-methoxybenzyl)cyclopropanesulfonamide as an off-white solid, 14.2 g (50%).

### Preparation of N-(7-amino-4-chloro-1-(2,2,2-trifluoroethyl)-1H-indazol-3-yl)-N-(4-methoxybenzyl)methanesulfonamide

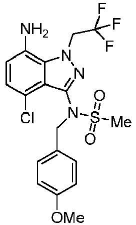

To a stirred solution of *N*-(7-bromo-4-chloro-1-(2,2,2-trifluoroethyl)-1*H*-indazol-3-yl)-N-(4-methoxybenzyl)methanesulfonamide (3 g, 5.69 mmol, 1.0 equiv.) in NMP (45 mL) was added at room tempreature copper (I) iodide (0.22 g, 1.13 mmol, 0.2 equiv.), sodium ascorbate (2.25 g, 11.38 mmol, 2 equiv.) and (1R, 2R)-*N*₁,*N*₂-dimethylcyclohexane-1,2-diamine (0.4 g, 2.84 mmol, 0.5 equiv.). To the mixture was added a solution of sodium azide (1.1 g, 17.07 mmol) in water (15 mL). The mixture was heated to 100 °C and maintained at that tempreature for 13 h. The reaction mixture was cooled to room temperature and was then diluted with ethyl acetate (50 mL). The mixture was filtered through a pad of Celite bed extracting with EtOAc (30 mL). The combined filtrate was poured into water (50 mL) and the organic layer was isolated and reserved. The aqueous phase was extracted with EtOAc (2 × 30 mL). The combined organics were washed with water (50 mL), brine (40 mL), dried over Na₂SO₄, filtered and concentrated *in vacuo.* The resulting residue was subjected to silica gel column chromatography (hexanes:EtOAc 100:0→75:25) to afford the title compound, N-(7-amino-4-chloro-1-(2,2,2-trifluoroethyl)-1*H*-indazol-3-yl)-*N*-(4-methoxybenzyl)methanesulfonamide as an off-white solid, 1.6 g (61%).

### Preparation of bicyclo[3.1.0]hexan-3-ol

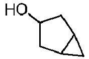

To a stirred solution of cyclopent-3-enol (130 g, 1545 mmol) in DCM (1200 mL) under N₂ atmosphere at 0-5 °C was added dropwise a solution of diethyl zinc in hexane (1.0 M, 3091 mL, 3091 mmol) over a period of 3 h. To the solution at 0 °C was added dropwise a solution of diiodomethane (249 mL, 3091 mmol) in DCM (300 mL) over a period of 1h. The reaction mixture was allowed to warm to 27 °C upon which formation of a white precipitation was observed. The mixture stirred for 16 h. Progress of the reaction was monitored by TLC (SiO₂, 20% EtOAc/pet, Rf = 0.3, UV-inactive, PMA-active). The reaction mixture was quenched via the careful addition of aq. saturated NH₄Cl solution (1.5 L). The mixture was filtered through pad of Celite. The aqueous layer was extracted with DCM (2 × 1L). The combined organic layers were dried over anhydrous Na₂SO₄, filtered and then concentrated under reduced pressure to afford crude bicyclo[3.1.0]hexan-3-ol as red liquid, 180 g. ¹H NMR (400 MHz, CDCl₃) δ = 4.41 - 4.35 (m, 1H), 2.18 - 2.05 (m, 2H), 1.73 (d, *J* = 13.9 Hz, 2H), 1.35 - 1.25 (m, 2H), 1.21 - 1.14 (m, 1H), 0.57 - 0.43 (m, 2H). GCMS: m/z = 98.1).

### Preparation of bicyclo[3.1.0]hexan-3-one

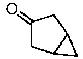

To a stirred solution ofbicyclo[3.1.0]hexan-3-ol (210 g, 2054 mmol) in DCM (5000 mL) under N₂ atmosphere at 0 °C was added portion-wise Dess-Martin periodinane (954 g, 225 mmol). The mixture was allowed to warm to 27 °C and was then stirred for 16 h. Progress of the reaction was monitored by TLC (SiO₂, 20% Acetone/Hex, Rf = 0.3, UV in-active, PMA-active). The reaction mixture was filtered through pad of Celite and the filtrate was washed with aq. NaOH (1N, 8x 1 L). The combined aqueous phases were extracted with DCM (5 × 1 L). The combined organic layers were dried over anhydrous Na₂SO₄, filtered, and then concentrated under reduced pressure (bath temperature: 20 °C) to afford crude bicyclo[3.1.0]hexan-3-one as brown liquid. The liquid was further purified by downward distillation at 70 °C to afford bicyclo[3.1.0]hexan-3-one as a pale yellow viscous liquid, 125 g (62%). ¹H NMR (400 MHz, CDCl₃) δ = 2.61 - 2.54 (m, 2H), 2.17 - 2.12 (m, 2H), 1.54 - 1.46 (m, 2H), 0.92 - 0.86 (m, 1H), -0.01 - -0.08 (m, 1H); GCMS: M/Z = 96.1.

### Preparation of 2-(2,2-difluoroacetyl)bicyclo[3.1.0]hexan-3-one

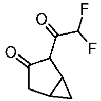

To a stirred solution ofbicyclo[3.1.0]hexan-3-one (125 g, 1274 mmol) in THF (1500 mL) under N₂ atmosphere at -78 °C was added LDA (2.0 M in THF, 0.701 L, 1402 mmol). The solution was stirred for 1 h at -78 °C. To the solution was added slowly over 30 minutes a solution of ethyldifluoroacetate (174 g, 1402 mmol) in THF (300 mL) maintaining a temperature of -78 °C. The reaction mixture was allowed to warm to 27 °C and was then stirred for 1 h. Progress of the reaction was monitored by TLC (SiO₂, 20% Acetone/Hexane, Rf = 0.3, UV -active). The reaction mixture was quenched via the addition of aq. HCl (1N, 2000 mL). The mixture was stirred for 30 min. and then was extracted with EtOAc (3 × 1000 mL). The combined organic layers were washed with brine (1000 mL), dried over anhydrous Na₂SO₄ and filtered. The filtrate was concentrated under reduced pressure to afford 2-(2,2-difluoroacetyl)bicyclo[3.1.0]hexan-3-one as a pale yellow viscous liquid, 180 g (71%). ¹H NMR (400 MHz, CDCl₃) δ = 6.18 (t, *J=* 54.8 Hz, 1H), 2.70 - 2.62 (m, 1H), 2.35 (d, *J=* 19.4 Hz, 1H), 2.14 (br s, 1H), 1.26 - 1.21 (m, 1H), 1.04-1.03 (m, 1H), 0.22-0.21 (m, 1H), LCMS: M/Z = 173.17).

### Preparation of ethyl 2-(3-(difluoromethyl)-3b,4,4a,5-tetrahydro-1H-cyclopropa[3,4]cyclopenta[1,2-c]pyrazol-1-yl)acetate.

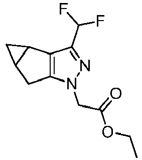

To a stirred solution of 2-(2,2-difluoroacetyl)bicyclo[3.1.0]hexan-3-one (180 g, 910 mmol) in ethanol (2 L) under N₂ atmosphere at 27 °C was added ethyl 2-hydrazinylacetate hydrochloride (422 g, 2729 mmol) followed by sulfuric acid (20 mL, 375 mmol). The mixture was stirred for 30 min. and then was was heated to 100 °C and stirred for 16 h. Progress of the reaction was monitored by TLC (SiO₂, 20% Acetone/Hexane, Rf = 0.3, UV-active). The reaction mixture was concentrated under reduced pressure. The residue was dissolved in EtOAc (2000 mL) and was washed with water (2 × 1 L), brine (1.0 L), dried over anhydrous Na₂SO₄, filtered, and then was concentrated under reduced pressure. The resulting residue was subjected to silica gel column chromatography (pet.:acetone 100:0→98:2) to afford ethyl 2-(3-(difluoromethyl)-3b,4,4a,5-tetrahydro-1H-cyclopropa[3,4]cyclopenta[1,2-c]pyrazol-1-yl)acetate as an off-white solid, 110 g (46%). ¹H NMR (400 MHz, DMSO-d₆) δ = 6.86 (t, *J=* 54.8 Hz, 1H), 4.93 (s, 2H), 4.14 (q, *J=* 7.2 Hz, 2H), 2.88 - 2.79 (m, 1H), 2.76 - 2.68 (m, 1H), 2.14 - 2.04 (m, 2H), 1.19 (t, *J=* 7.2 Hz, 3H), 1.10 - 1.03 (m, 1H), 0.14 (q, *J* = 4.3 Hz, 1H).

### Preparation of ethyl 2-(3-(difluoromethyl)-5-oxo-3b,4,4a,5-tetrahydro-1H-cyclopropa[3,4]cyclopenta[1,2-c]pyrazol-1-yl)acetate.

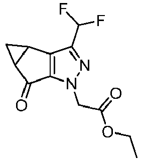

To a stirred solution of ethyl 2-(3-(difluoromethyl)-3b,4,4a,5-tetrahydro-1H-cyclopropa[3,4]cyclopenta[1,2-c]pyrazol-1-yl)acetate (110 g, 422 mmol) and Celite (395 g) in cyclohexane (3.5 L) at 0 °C was added portionwise pyridinium dichromate (794 g, 2110 mmol). To the mixture under nitrogen atmosphere was added dropwise tert-butyl hydroperoxide (355 mL, 2130 mmol) over a period of 10 min. The reaction mixture was warmed to 27 °C and was then stirred at that temperature for 48 h. Progress of the reaction was monitored by TLC (SiO₂, 30% Acetone/pet, Rf = 0.4, UV -active). The reaction mixture was filtered and the filter cake was extracted with EtOAc (1000 mL). The filtrate was washed with saturated aq. Na₂S₂O₃ (2x500 mL); saturated aq. FeSO₄ (300 mL); and then brine (500 mL). The organic layer was dried over anhydrous Na₂SO₄,; filtered and concentrated under reduced pressure to obtain the crude title compound (150 g).

### Preparation of ethyl 2-(3-(difluoromethyl)-4,4a-dihydrospiro[cyclopropa[3,4]cyclopenta[1,2-c]pyrazole-5,2'-[1,3]dithiolane]-1(3bH)-yl)acetate.

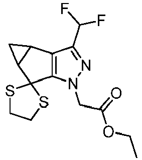

To a stirred solution of ethyl 2-(3-(difluoromethyl)-5-oxo-3b,4,4a,5-tetrahydro-1H-cyclopropa[3,4]cyclopenta[1,2-c]pyrazol-1-yl)acetate (75 g, 269 mmol) in DCM (1500 mL) at 27 °C under nitrogen atmosphere was added ethane-1,2-dithiol (43.0 mL, 511 mmol) followed by the addition of boron trifluoride acetic acid (72.6 mL, 511 mmol). The solution was stirred for 16 h. Progress of the reaction was monitored by TLC (SiO₂, 20% Acetone/Pet, Rf = 0.35, UV -Active). After completion, the reaction mixture was cooled to 0 °C and quenched via the addition of aq. saturated NaHCO₃ (500 mL). The mixture was extracted with DCM (2 × 1000 mL). The combined organics were washed with brine (1000 mL), dried over anhydrous Na₂SO₄, filtered and concentrated under reduced pressure to obtain a brown liquid. This material was subjected to silica gel column chromatography (Pet.:EtOAc 95:5→90:10) to afford ethyl 2-(3-(difluoromethyl)-4,4a-dihydrospiro[cyclopropa[3,4]cyclopenta[1,2-c]pyrazole-5,2'-[1,3]dithiolane]-1(3bH)-yl)acetate as an off-white solid, 80 g (74%). ¹H-NMR (400 MHz, CDCl₃) δ = 6.61 (t, *J=* 55.2 Hz, 1H), 5.00 - 4.85 (m, 2H), 4.29 - 4.19 (m, 2H), 3.55 - 3.46 (m, 4H), 2.63 - 2.53 (m, 1H), 2.49 - 2.38 (m, 1H), 1.30 - 1.24 (m, 4H), 0.65 - 0.60 (m, 1H). LCMS M+H = 346.9.

### Preparation of ethyl 2-(3-(difluoromethyl)-5,5-difluoro-3b,4,4a,5-tetrahydro-1H-cyclopropa[3,4]cyclopenta[1,2-c]pyrazol-1-yl)acetate

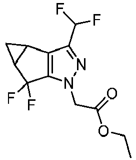

To a stirred solution of 1,3-dibromo-5,5-dimethylimidazolidine-2,4-dione (26.3 g, 92 mmol) in DCM (20 mL) at -70 °C under N₂ atmosphere was added HF-pyridine (2.460 g, 24.83 mmol). The solution was for 30 min. To the solution was added a solution of ethyl 2-(3-(difluoromethyl)-4,4a-dihydrospiro[cyclopropa[3,4]cyclopenta[1,2-c]pyrazole-5,2'-1,3]dithiolane]-1(3bH)-yl)acetate (10 g, 25 mmol) in DCM (20 mL). The reaction mixture was allowed to warm to -40 °C and then was stirred at that temperature for 1 h. Progress of the reaction was monitored by TLC (SiO2, 30% EtOAc/Pet, Rf = 0.3, UV in-active). The reaction mixture was quenched via the addition of aq. sat. NaHCO₃ (200 mL). The mixture was warmed to room temperature and was then extracted with EtOAc (2 × 100 mL). The combined organics were washed with brine (50 mL); dried over anhydrous Na₂SO₄; filtered; and were concentrated under reduced pressure to afford a brown solid. This material was subjected to silica gel column chromatography (Pet.:EtOAc 100:0→75-25) to afford ethyl 2-(3-(difluoromethyl)-5,5-difluoro-3b,4,4a,5-tetrahydro-1H-cyclopropa[3,4]cyclopenta[1,2-c]pyrazol-1-yl)acetate as a pale yellow solid, 8.5 g (91%). ¹H NMR (400 MHz, CDCl₃) δ = 6.62 (t, J = 55.2 Hz, 1H), 4.82 (s, 2H), 4.30 - 4.18 (m, 2H), 2.51 - 2.37 (m, 2H), 1.42 - 1.35 (m, 1H), 1.31 - 1.23 (m, 3H), 1.14 - 1.08 (m, 1H). LCMS M+H = 293.07.

### Preparation of 2-(3-(difluoromethyl)-5, 5-difluoro-3b,4,4a,5-tetrahydro-1H-cyclopropa[3,4]cyclopenta[1,2-c]pyrazol-1-yl)acetic acid

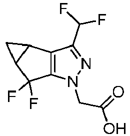

To a stirred solution of ethyl 2-(3-(difluoromethyl)-5,5-difluoro-3b,4,4a,5-tetrahydro-1H-cyclopropa[3,4]cyclopenta[1,2-c]pyrazol-1-yl)acetate (15 g, 50 mmol) in THF (17 mL) and MeOH (66 mL) at 0 °C under N₂ atmosphere was added a solution of LiOH (1.788 g, 74.7 mmol) in water (66 mL). The reaction mixture was allowed to warm to 27 °C and was then stirred for 3 h at that temperature. Progress of the reaction was monitored by TLC (SiO₂, 5% MeOH/DCM, Rf = 0.2, UV Active). After completion, the reaction mixture was concentrated under reduced pressure; diluted with water (50 mL); and washed with EtOAc (2 × 250 mL) to remove impurities. The aqueous layer was adjusted to pH 2-3 using aq. HCl (1M), then was extracted with EtOAc (3 × 1000 mL). The combined organics were dried over anhydrous Na₂SO₄; filtered; and concentrated under reduced pressure to afford 2-(3-(difluoromethyl)-5,5-difluoro-3b,4,4a,5-tetrahydro-1H-cyclopropa[3,4]cyclopenta[1,2-c]pyrazol-1-yl)acetic acid as an off white solid, 14 g (98%). LCMS M+H = 265.15.

### Separation affording 2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetic acid and 2-((3bR,4aS)-3-(difluoromethyl)-5,5-difluoro-3b,4,4a,5-tetrahydro-1H-cyclopropa[3,4]cyclopenta[1,2-c]pyrazol-1-yl)acetic acid

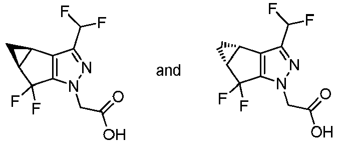

2-(3-(difluoromethyl)-5,5-difluoro-3b,4,4a,5-tetrahydro-1H-cyclopropa[3,4]cyclopenta[1,2-c]pyrazol-1-yl)acetic acid (5.5 g) was dissolved in isopropanol (20 mL). The solution was subjected portion-wise to SFC chiral separation as follows: Instrument = Thar 80; column = Chiralpak IC 30x250mm, 5 micron; solvent A = super critical CO₂; solvent B = isopropanol with 0.5% isopropylamine (v/v); eluent composition = 70%A:30%B; flow-rate = 65 g/min; back-pressure = 100 bar; temperature = 30 °C; injection volume = 2.5 mL; detection = 220 nm. 2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetic acid was collected as peak eluting from 7.5 min. to 14 min; 2-((3bR,4aS)-3-(difluoromethyl)-5,5-difluoro-3b,4,4a,5-tetrahydro-1H-cyclopropa[3,4]cyclopenta[1,2-c]pyrazol-1-yl)acetic acid was collected as a peak eluting from 2.7 min. to 5.8 min. For each enantiomer, the resulting solution was concentrated under reduced pressure and the resulting solids were dissolved in EtOAc, then twice washed with aq. citric acid (1M) followed by water followed by brine. The organic solution was dried over Na₂SO₄; filtered; then concentrated in vacuo to afforded the separated enantiomer in 80-90% recovery.

### Preparation of ethyl 2-(3,5-bis(difluoromethyl)-1H-pyrazol-1-yl)acetate

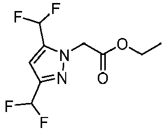

To a stirred solution of 1,1,5,5-tetrafluoropentane-2,4-dione (15 g, 87 mmol) in ethanol (150 mL) under N₂ atmosphere at 26 °C was added sulfuric acid (1.394 mL, 26.2 mmol) followed by ethyl aminoglycinate hydrochloride (16.17 g, 105 mmol). The reaction mixture was heated to 100 °C and then stirred for 3 h at that temperature. Progress of the reaction was monitored by TLC (SiO₂, 30% EtOAc/pet, Rf = 0.4, UV-active). After completion, the reaction mixture was cooled to room temperature and then was concentrated under reduced pressure. The resulting residue was dissolved in water (100 mL) and then extracted with EtOAc (2 × 100 mL). The combined organic layers were washed with brine (50 mL), dried over anhydrous Na₂SO₄, filtered and then concentrated under reduced pressure to afford ethyl 2-(3,5-bis(difluoromethyl)-1H-pyrazol-1-yl)acetate as pale yellow solid, 22.0 g (86 %). ¹H NMR (400 MHz, CDCl₃) δ = 6.91 - 6.52 (m, 3H), 5.03 (s, 2H), 4.30 - 4.20 (m, 2H), 1.32 - 1.25 (m, 3H). LCMS: (M+H) = 255.21, LCMS Purity = 86.6%.

### Preparation of 2-(3,5-bis(difluoromethyl)-1H-pyrazol-1-yl)acetic acid

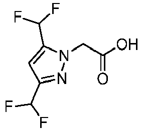

To a stirred solution of ethyl 2-(3,5-bis(difluoromethyl)-1H-pyrazol-1-yl)acetate (22 g, 75 mmol) in THF (50 mL) and methanol (25 mL) under N₂ atmosphere at 0°C was added dropwise a solution of lithium hydroxide (5.41 g, 226 mmol) in water (25 mL). The reaction mixture was allowed to warm to 27 °C and was then stirred for 16 h at that temperature. Progress of the reaction was monitored by TLC (SiO₂, 50% EtOAc/pet, Rf = 0.2, UV-active). After completion, the reaction mixture was concentrated under reduced pressure. The resulting residue was dissolved in water (100 mL) and the solution was adjusted to pH 3 using aq. HCl (2 N). The solution was extracted with EtOAc (4 × 50 mL). The combined organic layers were washed with brine (50 mL), dried over anhydrous Na₂SO₄, filtered, and then concentrated under reduced pressure to afford 2-(3,5-bis(difluoromethyl)-1H-pyrazol-1-yl)acetic as pale yellow solid acid, 15 g (87 %). ¹H NMR (400 MHz, DMSO-d₆) δ = 13.53 - 13.24 (m, 1H), 7.46 - 7.07 (m, 3H), 5.14 (s, 2H). LCMS: (M-H) = 225.15; LCMS Purity = 98.7%.

### Preparation of 1-cyclopropyl-4,4-difluorobutane-1,3-dione

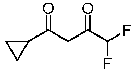

To a stirred solution of 1-cyclopropylethan-1-one (20 g, 238 mmol) in diethyl ether (2000 mL) under N₂ atmosphere at -78 °C was slowly added NaHMDS (119 mL, 238 mmol) over a period of 20 min. The solution was then stirred for 45 min at -78 °C. To the solution was added ethyl 2,2-difluoroacetate (75 mL, 713 mmol). The reaction mixture was slowly warmed to 27 °C and then stirred for 16 h. After completion, the reaction mixture was quenched with water (80 mL) and washed with diethyl ether (100 mL). The aqueous layer was acidified with aq. HCl (1N, 20 mL) and extracted with diethyl ether (2 × 100 mL). The combined organic layers were washed with brine (50 mL), dried over Na₂SO₄, filtered and concentrated under reduced pressure to afford 1-cyclopropyl-4,4-difluorobutane-1,3-dione as pale yellow oil 25 g (65%). ¹H NMR (400 MHz, CDCl₃) δ = 6.07 - 5.87 (m, 2H), 1.84 - 1.75 (m, 1H), 1.28 - 1.19 (m, 2H), 1.10 - 1.05 (m, 2H).

### Preparation of 5-cyclopropyl-3-(difluoromethyl)-1H-pyrazole

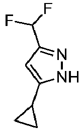

To a stirred solution of 1-cyclopropyl-4,4-difluorobutane-1,3-dione (25 g, 154 mmol) in ethanol (250 mL) at 27 °C was added hydrazine.H₂O (16.13 mL, 385 mmol) followed by dropwise addition of hydrochloric acid (0.18 mL, 5.92 mmol). The reaction mixture was heated to 80 °C and stirred at that temperature for 6 h. The reaction was monitored by TLC (50% EtOAc in pet ether; RF: 0.2; Detection: KMnO₄ active). After completion, the reaction mixture was concentrated under reduced pressure. The resulting residue was dissolved in water (250 mL) and extracted with ethyl acetate (3 × 200 mL). The combined organic layers were washed with brine (50 mL), dried over Na₂SO₄, filtered and concentrated under reduced pressure to afford 5-cyclopropyl-3-(difluoromethyl)-1H-pyrazole as a yellow liquid 20 g (79%).¹H NMR (400 MHz, CDCl₃) δ = 6.79 - 6.49 (m, 1H), 6.24 - 6.08 (m, 1H), 1.96 - 1.82 (m, 1H), 1.09 - 0.91 (m, 2H), 0.79 - 0.56 (m, 2H) LCMS: M+H = 159.11, purity = 96.91%.

### Preparation of ethyl 2-(5-cyclopropyl-3-(difluoromethyl)-1H-pyrazol-1-yl) acetate:

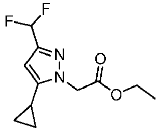

To a stirred solution of 5-cyclopropyl-3-(difluoromethyl)-1H-pyrazole (20 g, 123 mmol) in acetonitrile (200 mL) at 27 °C under N₂ atmosphere was added DIPEA (53.5 mL, 306 mmol) followed by ethyl bromoacetate (27.3 mL, 245 mmol). The reaction mixture was stirred at 65 °C for 48 hr. The progress of the reaction was monitored by TLC (SiO₂, Mobile phase: 30% ethyl acetate in pet ether; RF: 0.5 and KMnO₄ active). After completion, the reaction mixture was diluted with water (20 mL) and extracted with ethyl acetate (2 × 500mL). The combined organic layers were washed with brine solution (500 mL) and dried over anhydrous Na₂SO₄, filtered and concentrated under vacuum to afford the crude compound as brown oil (30 g). This material was subjected to silica gel chromatography (pet.:EtOAc 80:20→70:30) to afford ethyl 2-(5-cyclopropyl-3-(difluoromethyl)-1H-pyrazol-1-yl)acetate as a mixture of regioisomers, 25 g. The material was further purified by HPLC using the following conditions: Column = KROMOSIL PHENYL, 25 × 150 mm, 10 µm; Mobile phase A: 10 mM ammonium bicarbonate in water; Mobile phase B: acetonitrile; flow rate = 25 mL/min; temperature = ambient; Gradient (minute/%B) = 0/10, 2/10, 10/30, 15/30, 15.2/100, 18/100, 18.2/10. Fractions containing the desired product were pooled and then concentrated under reduced pressure to afford an aqueous mixture. This mixture was extracted with ethyl acetate (3 × 100 mL). The combined organics were dried over anhydrous Na₂SO₄, filtered, and concentrated under reduced pressure to afford ethyl 2-(5-cyclopropyl-3-(difluoromethyl)-1H-pyrazol-1-yl) acetate as a pale yellow oil, 2.1 g (24%).¹H NMR (400 MHz, DMSO-d₆) δ = 7.05 - 6.69 (m, 1H), 6.24 - 6.14 (m, 1H), 5.21 - 5.10 (m, 2H), 4.21 - 4.09 (m, 2H), 1.92 - 1.76 (m, 1H), 1.27 - 1.13 (m, 3H), 0.98 - 0.86 (m, 2H), 0.70 - 0.56 (m, 2H). LCMS: M+H = 245.31, purity = 98.89%.

### Preparation of 2-(5-cyclopropyl-3-(difluoromethyl)-1H-pyrazol-1-yl) acetic acid

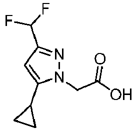

To a stirred solution of ethyl 2-(5-cyclopropyl-3-(difluoromethyl)-1H-pyrazol-1-yl)acetate (2.1 g, 8.60 mmol) in THF:methanol (5 mL:2 mL) at 27 °C was added a solution of LiOH (1.647 g, 68.8 mmol) in water (2 mL). The reaction mixture was stirred at 27 °C for 16 hr. The progress of the reaction was monitored by TLC (SiO₂, ethyl acetate; Rf: 0.1, UV inactive and KMnO₄ active). After completion, the reaction mixture was concentrated under reduced pressure. The resulting aqueous mixture was diluted with water (50 mL) and then washed with ethyl acetate (3 × 50 mL). The aqueous layer was cooled to 0 °C and then adjusted to pH 2 via addition of aq. HCl (2N). The precipitated solid was collected via filtration and then dried under vacuum to afford 2-(5-cyclopropyl-3-(difluoromethyl)-1H-pyrazol-1-yl) acetic acid as an off white solid, 1.3 g (70%). ¹H NMR (400 MHz, DMSO-d₆) δ = 13.27 - 13.10 (m, 1H), 7.02 - 6.72 (m, 1H), 6.21 - 6.10 (m, 1H), 5.08 - 4.93 (m, 2H), 1.86 - 1.77 (m, 1H), 0.97 - 0.87 (m, 2H), 0.71 - 0.58 (m, 2H). LCMS: M+H = 217.20, purity = 99.52%.

### Preparation of tert-butyl (S)-(1-(7-bromo-3-(4-chloro-3-(N-(4-methoxybenzyl)methylsulfonamido)-1-methyl-1H-indazol-7-yl)-4-oxo-3,4-dihydroquinazolin-2-yl)-2-(3,5-difluorophenyl)ethyl)carbamate

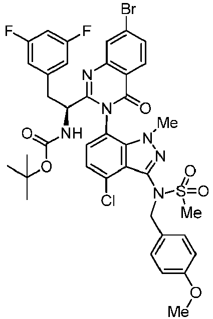

To a solution of (S)-2-((tert-butoxycarbonyl)amino)-3-(3,5-difluorophenyl)propanoic acid (3.82 g, 12.66 mmol), 2-amino-4-bromobenzoic acid (3.01 g, 13.93 mmol) and N-(7-amino-4-chloro-1-methyl-1H-indazol-3-yl)-N-(4-methoxybenzyl)methanesulfonamide (5 g, 12.66 mmol) in pyridine (50 mL) was added diphenyl phosphite (9.80 mL, 50.6 mmol). The resulting mixture was placed on a preheated oil bath (70 °C) and heated at 70 °C for 16 h. The mixture was cooled to room temperature and then concentrated under reduced pressure. The mixture was then diluted with EtOAc (approximately 500 mL) and washed with aqueous citric acid (0.5M, 2 × 50 mL), then aqueous NaOH (1M, 3 × 50 mL), dried over Na₂SO₄, filtered, and concentrated. The residue was then purified via silica gel chromatography (330 g silica gel column, gradient of hexanes:EtOAc 0:100→50:50) to afford tert-butyl (S)-(1-(7-bromo-3-(4-chloro-3-(N-(4-methoxybenzyl)methylsulfonamido)-1-methyl-1H-indazol-7-yl)-4-oxo-3,4-dihydroquinazolin-2-yl)-2-(3,5-difluorophenyl)ethyl)carbamate (6.2 g, 7.22 mmol, 57.1 % yield) as pale yellow solid foam (inseparable mixture of atropisomers). LC/MS: m/z = 801.10 [M-tBu].

### Preparation of (S)-N-(7-(2-(1-amino-2-(3,5-difluorophenyl)ethyl)-7-bromo-4-oxoquinazolin-3(4H)-yl)-4-chloro-1-methyl-1H-indazol-3-yl)methanesulfonamide

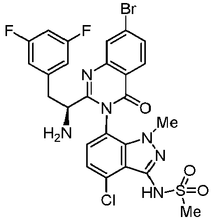

To a stirred solution of tert-butyl (S)-(1-(7-bromo-3-(4-chloro-3-(N-(4-methoxybenzyl)methylsulfonamido)-1-methyl- 1H-indazol-7-yl)-4-oxo-3,4-dihydroquinazolin-2-yl)-2-(3,5-difluorophenyl)ethyl)carbamate (6.2 g, 7.22 mmol) in dichloromethane (DCM) (50 mL) was added trifluoroacetic acid (20 mL, 260 mmol) followed by trifluoromethanesulfonic acid (0.770 mL, 8.67 mmol). The resulting dark red solution was stirred at room temperature for 1 h. LCMS at this point indicates two peaks containing the desired product mass, consistent with the presence of two diastereomeric atropisomers (ratio of approximately 30:70). The mixture was concentrated *in vacuo* and the resulting residue was partitioned between EtOAc (300 mL) and aq. NaOH (1M, 30 mL). The aq. phase was tested and determined to be pH >=8.0. The organic phase was isolated and dried over Na₂SO₄, filtered, and then concentrated *in vacuo.* The residue was purified in three approximately equal portions via C18 chromatography (275 g RediSep Gold Column, Mobile Phase A: 5:95 acetonitrile:water with 0.1 % TFA; Mobile Phase B: 95:5 acetonitrile:water with 0.1 % TFA; gradient of 10-60 %B over 30 min). Fractions containing the major atropisomer (second eluting) were combined, adjusted to pH 8 via addition of aq. 1M NaOH; extracted with ethyl acetate; washed with brine (sat. aq. NaCl); dried over NaiSO₄; filtered; and then concentrated to afford the desired major atropisomer (S)-N-(7-(2-(1-amino-2-(3,5-difluorophenyl)ethyl)-7-bromo-4-oxoquinazolin-3(4H)-yl)-4-chloro-1-methyl-1H-indazol-3-yl)methanesulfonamide (2.4 g, 3.76 mmol, 52% yield). ¹H NMR (500 MHz, DMSO-d₆) δ ppm 8.11 (d, J=8.55 Hz, 1 H), 8.06 (d, J=1.53 Hz, 1 H), 7.81 (dd, *J*=8.55, 1.83 Hz, 1 H), 7.33 (s, 2 H), 6.96 - 7.05 (m, 1 H), 6.75 (br d, J--7.02 Hz, 2 H), 3.67 (s, 3 H), 3.56 (dd, J=7.63, 5.19 Hz, 1 H), 3.25 - 3.29 (m, 1 H), 3.21 (s, 3 H), 2.81 (dd, J=13.43, 8.24 Hz, 1 H). LCMS: m/z = 637.05 [M+H]⁺.

### Preparation of N((S)-1-(7-bromo-3-(4-chloro-1-methyl-3-(methylsulfonamido)-1H-indazol-7-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

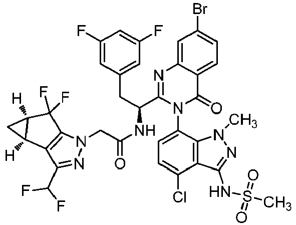

To a solution of (S)-N-(7-(2-(1-amino-2-(3,5-difluorophenyl)ethyl)-7-bromo-4-oxoquinazolin-3(4H)-yl)-4-chloro-1-methyl-1H-indazol-3-yl)methanesulfonamide (2.08 g, 3.26 mmol), 2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetic acid (0.861 g, 3.26 mmol) and diisopropylethylamine ("DIPEA") (1.709 mL, 9.78 mmol) in tetrahydrofuran (THF) (30 mL) was added HATU (1.364 g, 3.59 mmol). The resulting mixture was stirred at room temp for 3 h. To the mixture was added ammonia in methanol (2M, 3 mL). The mixture was stirred at room temp for 30 min. Water was then added and the mixture was extracted with ethyl acetate; washed with brine; dried over Na₂SO₄, filtered; and concentrated *in vacuo.* The resulting residue was subjected to silica gel chromatography (hexanes:EtOAc 100:0→30:70) to afford N-((S)-1-(7-bromo-3-(4-chloro-1-methyl-3-(methylsulfonamido)-1H-indazol-7-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (2.5 g, 2.83 mmol, 87 % yield). ¹H NMR (500 MHz, CDCl₃) δ ppm 8.18 (d, J=8.24 Hz, 1 H), 7.88 (d, J=1.53 Hz, 1 H), 7.72 (dd, J=8.55, 1.83 Hz, 1 H), 7.33 (s, 1 H), 7.16 (d, J--7.63 Hz, 1 H), 6.57 - 6.83 (m, 4 H), 6.38 (br d, J=5.80 Hz, 2 H), 4.71 - 4.80 (m, 1 H), 4.63 (d, J=6.71 Hz, 2 H), 3.56 (s, 3 H), 3.40 (s, 3 H), 3.18 (dd, J=13.73, 6.10 Hz, 1 H), 2.86 (dd, J=13.58, 7.48 Hz, 1 H), 2.52 - 2.61 (m, 1 H), 2.41 - 2.50 (m, 1 H), 1.42 - 1.50 (m, 1 H), 1.09 - 1.16 (m, 1 H). LCMS: m/z = 883.05 [M+H]⁺.

### Preparation of tert-Butyl (S)-(1-(7-bromo-3-(4-chloro-1-(2,2-difluoroethyl)-3-(N-(4-methoxybenzyl)methylsulfonamido)-1H-indazol-7-yl)-4-oxo-3,4-dihydroquinazolin-2-yl)-2-(3,5-difluorophenyl)ethyl)carbamate

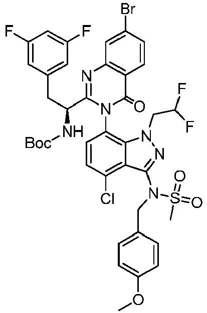

To a stirred solution of (S)-2-((tert-butoxycarbonyl)amino)-3-(3,5-difluorophenyl)propanoic acid (15 g, 49.8 mmol) and 2-amino-4-bromobenzoic acid (10.76 g, 49.8 mmol) in pyridine (150 mL) was added diphenyl phosphite (9.64 mL, 49.8 mmol) at 27 °C. The mixture was flushed with argon and the flask was then sealed. The reaction mixture was heated to 80 °C and stirred at that temperature for 2 hr. The reaction mixture was cooled to 27 °C and to the mixture was added N-(7-amino-4-chloro-1-(2,2-difluoroethyl)-1H-indazol-3-yl)-N-(4-methoxybenzyl)methanesulfonamide. The flask was sealed and the mixture was heated at 80 °C for 16 hr. The progress of the reaction was monitored by TLC (SiO₂, 30% EtOAc/Pet., Rf = 0.4, UV-active). The reaction mixture was allowed to cool to 27 °C and then was concentrated under reduced pressure. The resulting residue was subjected to silca gel column chromatography (Pet.:EtOAc 80:20→70:30) to afford tert-butyl (S)-(1-(7-bromo-3-(4-chloro-1-(2,2-difluoroethyl)-3-(N-(4-methoxybenzyl)methylsulfonamido)-1H-indazol-7-yl)-4-oxo-3,4-dihydroquinazolin-2-yl)-2-(3,5-difluorophenyl)ethyl)carbamate as an off-white solid, 18 g (35%). The isolated material is a mixture of stereoisomers. LCMS: M+H = 907.18 and 909.12; purity = 89%.

### Preparation of (S)-N-(7-(2-(1-amino-2-(3,5-difluorophenyl)ethyl)-7-bromo-4-oxoquinazolin-3(4H)-yl)-4-chloro-1-(2,2-difluoroethyl)-1H-indazol-3-yl)methanesulfonamide

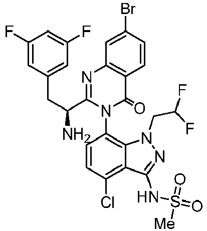

To a stirred solution of tert-butyl (S)-(1-(7-bromo-3-(4-chloro-1-(2,2-difluoroethyl)-3-(N-(4-methoxybenzyl)methylsulfonamido)-1H-indazol-7-yl)-4-oxo-3,4-dihydroquinazolin-2-yl)-2-(3,5-difluorophenyl)ethyl)carbamate (N68085-33-A2, 15 g, 14.70 mmol) in DCM (150 mL) at 27 °C under N₂ atmosphere was added TFA (150 mL, 1947 mmol). The solution was stirred for 10 min. To the reaction mixture was added triflic acid (15 mL, 169 mmol). The solution was stirred for 1 h at 27 °C. The progress of the reaction was monitored by TLC (SiO₂, 5% MeOH/DCM, Rf = 0.4, UV-active). On completion, the solvent was removed under a gentle stream of nitrogen. The residue was dissolved in EtOAc (500 mL), washed with aq saturated NaHCO₃ (2 × 250 mL), brine (150 mL), dried over NaiSOi and filtered. The filtrate was concentrated under reduced pressure to afford an off-white solid. LCMS analysis of the solid found a 75.42%:21.47% ratio of diastereomers. The crude solid subjected to C18 reverse-phase column chromatography (Mobile Phase: A: 0.1% TFA in water and B: 0.1% TFA in MeCN). Pure fractions containing the major diastereomer (atropisomer) were combined concentrated under reduced pressure. The resulting aqueous solution was made basic via the addition of aq. sat. NaHCOs; then was extracted with EtOAc (2 × 500 mL). The combined organic layers were washed with brine (200 mL), dried over Na₂SO₄, filtered and concentrated to afford (S)-N-(7-(2-(1-amino-2-(3,5-difluorophenyl)ethyl)-7-bromo-4-oxoquinazolin-3(4H)-yl)-4-chloro-1-(2,2-difluoroethyl)-1H-indazol-3-yl)methanesulfonamide as an off-white solid, 8.0 g (76%). LCMS: M+H = 687.34, Purity = 96%. This material was further purified to isolate the major enantiomer as follows: (S)-N-(7-(2-(1-amino-2-(3,5-difluorophenyl)ethyl)-7-bromo-4-oxoquinazolin-3(4H)-yl)-4-chloro-1-(2,2-difluoroethyl)-1H-indazol-3-yl)methanesulfonamide (4.5 g, 6.28 mmol) was dissolved in MeOH:MeCN (1:1, 170 mL). The solution was subjected portion-wise to SFC chiral separation as follows: column = (R, R) WHELK-01, 30x250 mm, 5 micron; solvent A = super critical CO₂; solvent B = methanol); eluent composition = 50%A:50%B; flow-rate = 100 g/min; back-pressure = 90 bar; injection volume = 1.1 mL; detection = 214 nm; Stack time = 6.8 min. For each isolated enantiomer, the resulting solution was concentrated under reduced pressure to afford an off-white solid. (S)-N-(7-(2-(1-amino-2-(3,5-difluorophenyl)ethyl)-7-bromo-4-oxoquinazolin-3(4H)-yl)-4-chloro-1-(2,2-difluoroethyl)-1H-indazol-3-yl)methanesulfonamide as was isolated as the peak eluting from 6 min to 8 min and afforded 2.1 g (48%). ¹H NMR (400 MHz, DMSO-d₆) δ = 8.11-8.05 (m, 2H), 7.83-7.78 (m, 1H), 7.47-7.41 (m, 2H), 7.03-6.97 (m, 1H), 6.76-6.69 (m, 2H), 6.41-6.14 (m, 1H), 4.47-4.22 (m, 2H), 3.54-3.49 (m, 1H), 3.25-3.21 (m, 4H), 2.83-2.76 (m, 1H). LCMS: M+H = 687.04, Purity = 99%, Chiral HPLC Purity = 96%.

### Preparation of N-((S)-1-(7-Bromo-3-(4-chloro-1-(2,2-difluoroethyl)-3-(methylsulfonamido)-1H-indazol-7-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

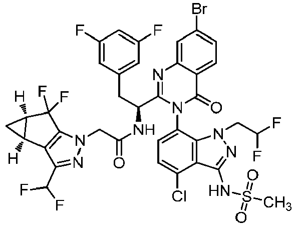

To a solution of (S)-N-(7-(2-(1-amino-2-(3,5-difluorophenyl)ethyl)-7-bromo-4-oxoquinazolin-3(4H)-yl)-4-chloro-1-(2,2-difluoroethyl)-1H-indazol-3-yl)methanesulfonamide (1.75 g, 2.52 mmol), 2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetic acid (0.739 g, 2.77 mmol), HOBt (0.424 g, 2.77 mmol) and EDC.HCl (0.579 g, 3.02 mmol) in DMF (15 mL) at 27 °C under nitrogen atmosphere was added N-methylmorpholine (2.215 mL, 20.15 mmol). The solution was stirred at 27 °C for 36 h. The progress of the reaction was monitored by TLC (SiO₂, 50% EtOAc/Pet. Rf = 0.5, UV-active). The reaction mixture was diluted with ice cold water (50 mL), and stirred for 15 min. The precipitated solid was isolated via filtration, washed with water (50 mL), and dried under vacuum to obtain the crude product. This material was treated with EtOAc (20 mL), stirred for 15 min, and then the solids were isolated via filtration and dried under vacuum to afford N-((S)-1-(7-bromo-3-(4-chloro-1-(2,2-difluoroethyl)-3-(methylsulfonamido)-1H-indazol-7-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide as an off-white solid, 1.6g (64%). ¹H NMR (400 MHz, DMSO-d₆) δ = 10.00 (brs, 1H), 9.23 (d, *J=* 8.1 Hz, 1H), 8.13 (d, *J =* 8.6 Hz, 1H), 7.98 (d, *J =* 2.0 Hz, 1H), 7.85 (dd, *J=* 2.0, 2.1 Hz, 1H), 7.78 (d, *J =* 7.9 Hz, 1H), 7.54 (d, *J =* 7.9 Hz, 1H), 7.07-6.99 (m, 1H), 6.92 (t, *J =* 51.7 Hz, 1H), 6.61(d, *J* = 6.3 Hz, 2H), 6.11 (t, *J=* 54.6 Hz, 1H), 4.72-4.57 (m, 2H), 4.38 (tt, *J* = 107, 2.9 Hz, 1H), 4.31-4.19 (m, 1H), 3.96-3.83 (m, 1H), 3.44-3.37 (m, 1H), 3.19 (s, 3H), 3.00-2.92 (m, 1H), 2.49-2.45 (m, 2H), 1.39-1.31 (m, 1H), 0.87-0.82 (m, 1H). LCMS: M+H = 933.13, LCMS Purity = 95%, HPLC Purity = 96%, Chiral HPLC Purity = 97%.

### Preparation of tert-butyl(S)-(1-(7-bromo-3-(4-chloro-1-(2,2-difluoroethyl)-3-(N-(4-methoxybenzyl) cyclopropanesulfonamido)-1H-indazol-7-yl)-4-oxo-3,4-dihydroquinazolin-2-yl)-2-(3,5-difluorophenyl)ethyl)carbamate

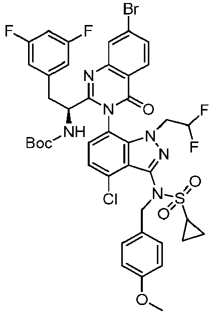

To a stirred solution of (S)-2-((tert-butoxycarbonyl)amino)-3-(3,5-difluorophenyl)propanoic acid (15 g, 49.8 mmol) and 2-amino-4-bromobenzoic acid (12.91 g, 59.7 mmol) in pyridine (150 mL) in a sealed tube at 26 °C was added diphenyl phosphite (35.7 mL, 184 mmol). The reaction mixture was degassed with N₂ bubbling for each addition of reagents. The reaction mixture was heated to 80 °C and stirred for 2 hr. The reaction mixture was cooled to 26 °C, then N-(7-amino-4-chloro-1-(2,2-difluoroethyl)-1H-indazol-3-yl)-N-(4-methoxybenzyl)cyclopropanesulfonamide (N66734-90-A2, 20.49 g, 34.9 mmol) was added. The mixture was heated at 80 °C for 16 h. The progress of the reaction was monitored by TLC (SiO₂, 30% EtOAc/Pet. Rf = 0.3). The reaction mixture was cooled to 26 °C and then was concentrated under reduced pressure. The residue was diluted with water (150 mL) and extracted with ethyl acetate (2 × 500 mL). The combined organic layers were washed with aq. citric acid (5% w/v, 2 × 150 mL), then brine (250 mL); dried over anhydrous Na₂SO₄; filtered; and concentrated under reduced pressure to afford a brown gummy liquid (40 g). The above procedure was repeated, and the crude product of both iterations was combined. This material was then subjected to silica gel column chromatography (pet.:EtOAc, 60:40→55:45) to afforded tert-butyl (S)-(1-(7-bromo-3-(4-chloro-1-(2,2-difluoroethyl)-3-(N-(4-methoxybenzyl)cyclopropanesulfonamido)-1H-indazol-7-yl)-4-oxo-3,4-dihydroquinazolin-2-yl)-2-(3,5-difluorophenyl)ethyl)carbamate (mixture of diastereomers) as a yellow solid (42 g, 98%). LCMS: M+H = 933.88 & 935.88; purity = 76.91%.

### Preparation of (S)-N-(7-(2-(1-amino-2-(3,5-difluorophenyl)ethyl)-7-bromo-4-oxoquinazolin-3(4H)-yl)-4-chloro-1-(2,2-difluoroethyl)-1H-indazol-3-yl)cyclopropanesulfonamide

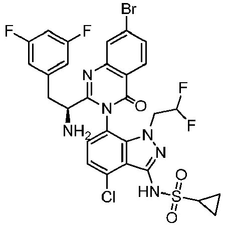

To a stirred solution of tert-butyl (S)-(1-(7-bromo-3-(4-chloro-1-(2,2-difluoroethyl)-3-(N-(4-methoxybenzyl)cyclopropanesulfonamido)-1H-indazol-7-yl)-4-oxo-3,4-dihydroquinazolin-2-yl)-2-(3,5-difluorophenyl)ethyl)carbamate (14 g, 11.53 mmol) in DCM (140 mL) at 27 °C under N2 atmosphere was added TFA (140 mL). The solution was stirred for 10 min. To the solution was added trifluoromethanesulfonic acid (7.16 mL, 81 mmol). The reaction mixture was stirred for 1 h at 27°C. The progress of the reaction was monitored by TLC (SiO₂, 50% EtOAc/pet, Rf = 0.2). The solvent was removed under a gentle stream of nitrogen. The residue was dissolved in EtOAc (500 mL) and the organic layer was washed with aq. saturated NaHCO₃ (2 × 150 mL), brine (50 mL), dried over Na₂SO₄, filtered and concentrated to dryness to the crude compound as an off white solid (12 g). The above procedure was repeated twice more and the additional crude solids (2 × 14 g) were combined with the above. The combined material was dissolved in dichloromethane (500 mL) and concentrated to afford a homogeneous crude solid. This material was washed with pet. ether:EtOAc (80:20) and then dried under vacuum to afford a brown solid (30 g). This material was then subjected to C18 reverse phase chromatography under the following conditions: Column = RediSep Gold HP C18 275 g; Mobile Phase A = Water:MeCN:TFA (950:50:1); Mobile Phase B = Water:MeCN:TFA (50:950:1); flow rate = 80 mL/min; gradient profile (time/%B) = 5/5, 5/10, 5/15, 10/20, 15/30, 20/40, 15/45, 10/50; temperature = ambient. Fractions of the major peak were pooled and concentrated under reduced pressure to remove the non-aqueous solvent. The resulting aq. solution was neutralized via the addition of sat. aq. NaHCO₃ (1000 mL), then was extracted with EtOAc (4 × 500 mL). The combined organics were washed with brine (500 mL), dried over anhydrous Na₂SO₄, filtered and concentrated to afford (S)-N-(7-(2-(1-amino-2-(3,5-difluorophenyl)ethyl)-7-bromo-4-oxoquinazolin-3(4H)-yl)-4-chloro-1-(2,2-difluoroethyl)-1H-indazol-3-yl)cyclopropanesulfonamide (single diastereomer) as an off white solid. The material was then subjected to SFC purification under the following conditions: Column/dimensions = Chiralpak OX-H (30x250 mm), 5µ; Solvent A = liquid CO₂; Solvent B = Methanol with 0.5% diethyl amine; Eluent = A:B (70:30); Flow-rate = 100.0 g/min; Back Pressure = 100.0 bar; Detection = UV (214 nm); injection volume = 1.3 mL (93 mg/injection); 160 injections. Two peaks were collected separately and the major peak was concentrated under reduced pressure to afford (S)-N-(7-(2-(1-amino-2-(3,5-difluorophenyl)ethyl)-7-bromo-4-oxoquinazolin-3(4H)-yl)-4-chloro-1-(2,2-difluoroethyl)-1H-indazol-3-yl)cyclopropanesulfonamide (single stereoisomer) as a pale yellow solid, 7.5 g (20%). ¹H NMR (400 MHz, DMSO-d₆) δ = 8.11 - 8.04 (m, 2H), 7.82-7.78 (m, 1H), 7.47 - 7.39 (m, 2H), 7.02 - 6.95 (m, 1H), 6.76-6.69 (m, 2H), 6.38 - 6.19 (m, 1H), 4.48 - 4.37 (m, 1H), 4.32 - 4.24 (m, 1H), 3.54 - 3.48 (m, 1H), 3.3 -3.20 (m, 1 H), 2.97 - 2.90 (m, 1H), 2.83 - 2.76 (m, 1H), 1.05 - 0.99 (m, 4H). LCMS: M+H = 712.94 and 714.94; purity = 98.37%, chiral HPLC purity = 96 %.

### Preparation of N((S)-1-(7-bromo-3-(4-chloro-3-(cyclopropanesulfonamido)-1-(2,2-difluoroethyl)-1H-indazol-7-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

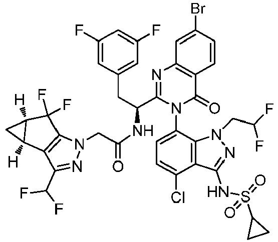

To a stirred solution of (*S*)-N-(7-(2-(1-amino-2-(3,5-difluorophenyl)ethyl)-7-bromo-4-oxoquinazolin-3(4H)-yl)-4-chloro-1-(2,2-difluoroethyl)-1H-indazol-3-yl)cyclopropanesulfonamide (500 mg, 0.700 mmol), 2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetic acid (N68084-15-A1, 185 mg, 0.700 mmol), and HOBt (42.9 mg, 0.280 mmol) in DMF (5 mL) at 27 °C was added N-methylmorpholine (0.308 mL, 2.80 mmol) and N-(3-dimethylaminopropyl)-N'-ethylcarbodiimide hydrochloride (242 mg, 1.261 mmol). The reaction mixture was stirred at 27 °C for 16 h. The progress of the reaction was monitored by TLC (SiO₂, 50% EtOAc/Pet., Rf = 0.3, UV-active). On completion, the reaction mixture was diluted with ice cold water (70 mL) and then stirred for 15 min at 27 °C. The precipitated solids were collected by filtration and then dried under vacuum to obtain the crude compound as an off-white solid. The crude compound was subjected to silica gel chromatography (pet.:EtOAc (98:2→50:50) to afford N-((S)-1-(7-bromo-3-(4-chloro-3-(cyclopropanesulfonamido)-1-(2,2-difluoroethyl)-1H-indazol-7-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide as an off-white solid, 550 mg (80%).¹H NMR (400 MHz, DMSO-d₆) δ = 9.99 (s, 1H), 9.24 (d, *J* = 8.1 Hz, 1H), 8.13 (d, *J =* 8.8 Hz, 1H), 7.97 (d, *J =* 1.8 Hz, 1H), 7.87-7.83 (m, 1H), 7.77 (d, *J=* 7.9 Hz, 1H), 7.54 (d, *J=* 7.9 Hz, 1H), 7.06-6.79 (m, 2H), 6.64-6.58 (m, 2H), 6.23-5.98 (m, 1H), 4.74-4.57 (m, 2H), 4.41-4.35 (m, 1H), 4.29-4.16 (m, 1H), 3.94-3.84 (m, 1H), 3.38-3.34 (m, 1H), 3.02-2.93 (m, 1H), 2.90-2.83 (m, 1H), 2.48-2.35 (m, 2H), 1.37-1.30 (m, 1H), 1.02-0.90 (m, 4H), 0.87-0.82 (m, 1H). LCMS analysis method F: RT = 6.74 mins, (M+H) = 959.0 and 961.0; LCMS Purity = 98%; Chiral HPLC Purity = 98%.

### Preparation of (S)-2-(3,5-bis(difluoromethyl)-1Hpyrazol-1yl)-N-(1-(7-bromo-3-(4-chloro-3-(cyclopropanesulfonamido)-1-(2,2-difluoroethyl)-1H-indazol-7-yl)-4-oxo-3,4-dihydroquinazolin-2-yl)-2-(3,5-difluorophenyl)ethyl)acetamide

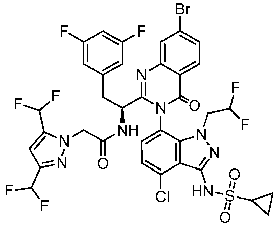

To a solution of (S)-N-(7-(2-(1-amino-2-(3,5-difluorophenyl)ethyl)-7-bromo-4-oxoquinazolin-3(4H)-yl)-4-chloro-1-(2,2-difluoroethyl)-1H-indazol-3-yl)cyclopropanesulfonamide (500 mg, 0.690 mmol), 2-(3,5-bis(difluoromethyl)-1H-pyrazol-1-yl)acetic acid (236 mg, 1.035 mmol) and HOBt (190 mg, 1.242 mmol) in DMF (10 mL) at 27 °C was added N-methylmorpholine (0.152 mL, 1.380 mmol) and N-(3-dimethylaminopropyl)-N'-ethylcarbodiimide hydrochloride (238 mg, 1.242 mmol). Then the reaction mixture was degassed for 10 min with nitrogen gas. The reaction mixture was stirred at 27 °C for 16 h; progress of the reaction was monitored by TLC (SiO₂, 50% EtOAc/Pet. Rf = 0.2). After completion of reaction, the reaction mixture was diluted with ethyl acetate (50 mL) and washed with ice cold water (2x 30 mL), and then brine (20 mL). The organic layer was separated, dried over anhydrous Na₂SO₄, filtered and concentrated to dryness to afford the crude compound as an off white solid (700 mg). This material was subjected to silica gel column chromatography using silica gel (petEtOAc, 100:0→50:50) to afford (S)-2-(3,5-bis(difluoromethyl)-1H-pyrazol-1-yl)-N-(1-(7-bromo-3-(4-chloro-3-(cyclopropanesulfonamido)-1-(2,2-difluoroethyl)-1H-indazol-7-yl)-4-oxo-3,4-dihydroquinazolin-2-yl)-2-(3,5-difluorophenyl)ethyl)acetamide as an off white solid, 500 mg (76%). ¹H NMR (400 MHz, DMSO-d₆) δ = 9.99 - 9.94 (m, 1H), 9.31 - 9.25 (m, 1H), 8.14 (d, *J =* 8.6 Hz, 1H), 7.99 (d, *J =* 1.8 Hz, 1H), 7.88-7.83 (m, 1H), 7.75 (d, *J =* 8.1 Hz, 1H), 7.52 (d, *J=* 7.9 Hz, 1H), 7.07 - 6.82 (m, 4H), 6.65 - 6.57 (m, 2H), 6.19 - 5.99 (m, 1H), 4.94 - 4.81 (m, 2H), 4.45 - 4.38 (m, 1H), 4.31 - 4.19 (m, 1H), 3.97 - 3.87 (m, 1H), 3.39-3.34 (m, 1H), 3.01 - 2.94 (m, 1H), 2.89 - 2.82 (m, 1H), 1.00 - 0.92 (m, 4H). LCMS: M+H = 921.24 and 923.12; purity = 98.3%, chiral HPLC purity = 99.46%.

### Preparation of (S)-N-(1-(7-bromo-3-(4-chloro-3-(cyclopropanesulfonamido)-1-(2,2-difluoroethyl)-1H-indazol-7-yl)-4-oxo-3,4-dihydroquinazolin-2-yl)-2-(3,5-difluorophenyl)ethyl)-2-(5-cyclopropyl-3-(difluoromethyl)-1H-pyrazol-1-yl)acetamide

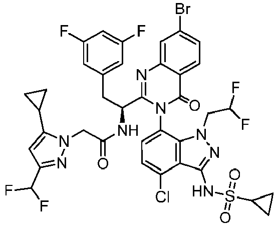

To a solution of (S)-N-(7-(2-(1-amino-2-(3,5-difluorophenyl)ethyl)-7-bromo-4-oxoquinazolin-3(4H)-yl)-4-chloro-1-(2,2-difluoroethyl)-1H-indazol-3-yl)cyclopropanesulfonamide (600 mg, 0.826 mmol), 2-(3-cyclopropyl-5-(difluoromethyl)-1H-pyrazol-1-yl)acetic acid (179 mg, 0.826 mmol) and HOBt (50.6 mg, 0.330 mmol) in DMF (5 mL) at 27 °C was added N-methylmorpholine (0.363 mL, 3.30 mmol) and N-(3-dimethylaminopropyl)-N'-ethylcarbodiimide hydrochloride (285 mg, 1.487 mmol). Then the reaction mixture was degassed for 10 min with nitrogen gas and then stirred at 27 °C for 16 h. The progress of the reaction was monitored by TLC (SiO₂, 50% EtOAc/Pet. Rf= 0.3). The reaction mixture was diluted with ice cold water (70 mL) and then was stirred for 30 min at 27 °C. The precipitated solid was isolated via filtration and then dried under vacuum to afford (S)-N-(1-(7-bromo-3-(4-chloro-3-(cyclopropanesulfonamido)-1-(2,2-difluoroethyl)-1H-indazol-7-yl)-4-oxo-3,4-dihydroquinazolin-2-yl)-2-(3,5-difluorophenyl)ethyl)-2-(5-cyclopropyl-3-(difluoromethyl)-1H-pyrazol-1-yl)acetamide as a pale yellow solid, 550 mg (68%). ¹H NMR (400 MHz, DMSO-d₆) δ = 10.02 - 9.85 (m, 1H), 9.17 - 9.10 (m, 1H), 8.14 (d, *J=* 8.3 Hz, 1H), 7.99 - 7.95 (m, 1H), 7.87 - 7.84 (m, 1H), 7.77 (d, *J=* 7.9 Hz, 1H), 7.52 (d, *J=* 7.9 Hz, 1H), 7.07 - 7.00 (m, 1H), 6.86 - 6.59 (m, 3H), 6.20 - 5.98 (m, 2H), 4.77 - 4.67 (m, 2H), 4.50 - 4.43 (m, 1H), 4.33 - 4.22 (m, 1H), 4.00 - 3.87 (m, 1H), 3.39 - 3.32 (m, 1H), 3.06 - 2.94 (m, 2H), 2.60 - 2.55 (m, 1H), 1.46 - 1.38 (m, 1H), 1.00 - 0.91 (m, 4H), 0.75 - 0.64 (m, 2H), 0.57 - 0.46 (m, 2H). LCMS: M+H = 910.89 and 912.91; purity = 93.59%.

### Preparation of N-((S)-1-(3-(4-chloro-1-methyl-3-(methylsulfonamido)-1H-indazol-7-yl)-4-oxo-7-(4,4,5,5-tetramethyl-1,3,2-dioxaborolan-2-yl)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

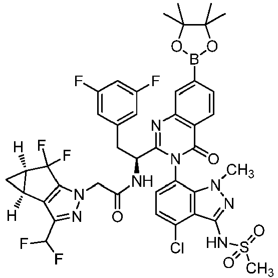

To a round bottom flask equipped with a stir bar was added N-((S)-1-(7-bromo-3-(4-chloro-1-methyl-3-(methylsulfonamido)-1H-indazol-7-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (1.00 g, 1.13 mmol), 4,4,4',4',5,5,5',5'-octamethyl-2,2'-bi(1,3,2-dioxaborolane) (431 mg, 1.70 mmol), potassium acetate (333 mg, 3.39 mmol), and [1,1'-bis(diphenylphosphino)ferrocene]dichloropalladium(II) ("Pd(dppf)Cl₂") (83 mg, 0.113 mmol). The flask was sealed with a rubber septum, and then was placed under an argon atmosphere. To the flask was added dioxane (23 mL). The reaction mixture was degassed with argon, then the reaction mixture was stirred at 60 °C for 16 h. The reaction mixture was concentrated *in vacuo* and adsorbed onto Celite. The resulting powder was subjected to silica gel chromatography (hexanes:EtOAc 100:0→0:100 over 10 column volumes) to afford N-((S)-1-(3-(4-chloro-1-methyl-3-(methylsulfonamido)-1H-indazol-7-yl)-4-oxo-7-(4,4,5,5-tetramethyl-1,3,2-dioxaborolan-2-yl)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (1.2 g, quantitative yield). LCMS: During LCMS analysis both the boronic acid and boronate were observed. Conditions: Wavelength1: 220 nm, Wavelength2: 254 nm, Injection Vol.: 5.00 µl, Stop Time: 4.00, Grad. Time: 3.0, Start %B: 0, End %B: 100, Total Flow: 0.80 ml/min, Solvent A: 95:5 Water:MeCN 0.1% TFA, Solvent B: 5:95 Water:MeCN 0.1% TFA, Column: Acquity UPLC BEH C18 1.7um; Result: retention time (boronic acid): 2.112 min., mass found: 849.15 (M+H); retention time (boronic ester): 2.733 min., mass found: 931.25 (M+H). ¹H NMR (CDCl₃, 500 MHz) δ 8.26 (d, 1H, J=7.6 Hz), 8.11 (s, 1H), 7.95 (d, 1H, J=7.6 Hz), 7.3-7.3 (m, 1H), 7.14 (d, 1H, J=7.9 Hz), 6.7-6.7 (m, 3H), 6.35 (d, 2H, J=6.8 Hz), 4.7-4.8 (m, 1H), 4.1-4.2 (m, 1H), 3.70 (s, 1H), 3.47 (s, 3H), 3.37 (s, 3H), 3.1-3.2 (m, 1H), 2.8-2.9 (m, 1H), 2.6-2.7 (m, 1H), 2.3-2.5 (m, 1H), 1.8-1.9 (m, 2H), 1.24 (s, 12H), 1.1-1.2 (m, 1H)

### Preparation of N-((S)-1-(3-(4-chloro-1-(2,2-difluoroethyl)-3-(methylsulfonamido)-1H-indazol-7-yl)-4-oxo-7-(4,4,5,5-tetramethyl-1,3,2-dioxaborolan-2-yl)-3,4-dihydroquinazolin-2--yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

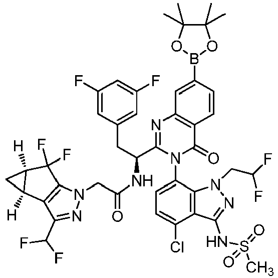

To a dry round-bottom flask equiped with a stir bar was added N-((S)-1-(7-bromo-3-(4-chloro-1-(2,2-difluoroethyl)-3-(methylsulfonamido)-1H-indazol-7-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (500 mg, 0.535 mmol), 4,4,4',4',5,5,5',5'-octamethyl-2,2'-bi(1,3,2-dioxaborolane) (204 mg, 0.803 mmol), potassium acetate (158 mg, 1.606 mmol), and PdCh(dppf) (39.2 mg, 0.054 mmol). The flask was sealed with a septum and then placed under argon atmosphere (vac/fill x 3). To the flask was added 1,4-dioxane (14 mL). The mixture was degassed (vac/fill with argon × 3). The mixture was then stirred at 60 °C for overnight (16 h). The reaction mixture was concentrated under reduced pressure. The resulting residue was adsorbed onto Celite. The resulting powder was subjected to silica gel column chromatography (40g silica gel column, hexanes:EtOAc 100:0→50:50 over 10 column volumes). The fractions containing the product were collected and concentrated *in vacuo* to afford N-((S)-1-(3-(4-chloro-1-(2,2-difluoroethyl)-3-(methylsulfonamido)-1H-indazol-7-yl)-4-oxo-7-(4,4,5,5-tetramethyl-1,3,2-dioxaborolan-2-yl)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide, 520 mg (99%). ¹H NMR (METHANOL-d₄, 500 MHz) δ 8.2-8.3 (m, 2H), 7.97 (d, 1H, J=7.7 Hz), 7.40 (d, 1H, J=8.0 Hz), 7.28 (d, 1H, J=8.0 Hz), 6.5-6.9 (m, 4H), 6.00 (tt, 1H, *J*=4.1, 55.2 Hz), 4.75 (dd, 1H, J=4.8, 9.2 Hz), 4.6-4.7 (m, 2H), 4.38 (dtd, 1H, J=4.2, 13.3, 15.2 Hz), 4.12 (q, 1H, J=7.2 Hz), 3.9-4.0 (m, 1H), 3.3-3.5 (m, 1H), 3.3-3.3 (m, 3H), 3.06 (dd, 1H, J=9.2, 14.0 Hz), 2.4-2.5 (m, 2H), 2.0-2.0 (m, 2H), 1.3-1.4 (m, 2H), 1.22 (s, 12H), 1.0-1.1 (m, 1H)

### Preparation of N-((S)-1-(3-(4-chloro-3-(cyclopropanesulfonamido)-1-(2,2-difluoroethyl)-1H-indazol-7-yl)-4-oxo-7-(4,4,5,5-tetramethyl-1,3,2-dioxaborolan-2-yl)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

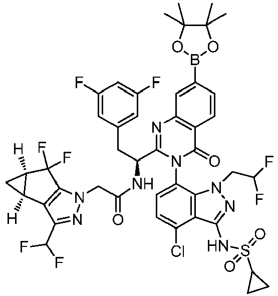

To a dry r.b. flask equipped with a stir bar was added N-((S)-1-(7-bromo-3-(4-chloro-3-(cyclopropanesulfonamido)-1-(2,2-difluoroethyl)-1H-indazol-7-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (300 mg, 0.312 mmol), 4,4,4',4',5,5,5',5'-octamethyl-2,2'-bi(1,3,2-dioxaborolane) (119 mg, 0.469 mmol), potassium acetate (92 mg, 0.937 mmol) and PdCh(dppf) (22.86 mg, 0.031 mmol). The flask was sealed with a septum and then placed under argon atmosphere (vac/fill × 3). To the flask was added dioxane (6.3 mL). The flask was again placed under argon atmosphere (vac/fill × 3). The resulting mixture was stirred at 60 °C for 16 h overnight. Upon cooling to ambient temperature the reaction was concentrated *in vacuo* and the resulting residue was adsorbed onto Celite. The resulting powder was subjected to silica gel column chromatography (hexanes:EtOAc 100:0→0:100 over 10 CV) to afford N-((S)-1-(3-(4-chloro-3-(cyclopropanesulfonamido)-1-(2,2-difluoroethyl)-1H-indazol-7-yl)-4-oxo-7-(4,4,5,5-tetramethyl-1,3,2-dioxaborolan-2-yl)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide, 220 mg (70%). ¹H NMR (METHANOL-d₄, 500 MHz) δ 8.27 (d, 2H, J=6.2 Hz), 8.26 (s, 1H), 7.97 (dd, 1H, J=1.0, 7.9 Hz), 7.41 (d, 1H, J=7.7 Hz), 7.29 (d, 1H, J=7.7 Hz), 6.8-6.8 (m, 1H), 6.70 (br t, 1H, J=54.8 Hz), 6.55 (dd, 2H, J--2.1, 8.0 Hz), 6.01 (t, 1H, J--55.3 Hz), 4.74 (dd, 1H, J=4.8, 9.5 Hz), 4.68 (d, 1H, J=16.4 Hz), 4.59 (d, 1H, J=16.4 Hz), 4.38 (dd, 1H, J=4.2, 15.2 Hz), 4.12 (q, 1H, J=7.2 Hz), 3.91 (dd, 1H, J=3.9, 15.2 Hz), 3.68 (s, 1H), 3.06 (dd, 1H, J--9.4, 14.2 Hz), 2.9-2.9 (m, 1H), 2.4-2.5 (m, 2H), 2.03 (s, 2H), 1.45 (s, 12H), 1.1-1.1 (m, 2H), 1.0-1.0 (m, 3H).

### Preparation of N-((S)-1-(3-(4-chloro-1-methyl-3-(methylsulfonamido)-1H-indazol-7yl)-7-hydrozη-4-oxo-3,4-dihydroquinazolin-2yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

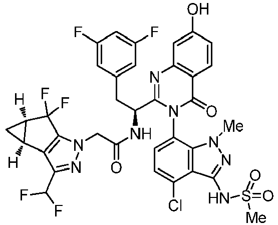

To a stirred solution of (S)-N-(7-(2-(1-amino-2-(3,5-difluorophenyl)ethyl)-7-hydroxy-4-oxoquinazolin-3(4H)-yl)-4-chloro-1-methyl-1H-indazol-3-yl)methanesulfonamide (473 mg, 0.823 mmol), (S)-N-(7-(2-(1-amino-2-(3,5-difluorophenyl)ethyl)-7-hydroxy-4-oxoquinazolin-3(4H)-yl)-4-chloro-1-methyl-1H-indazol-3-yl)methanesulfonamide (473 mg, 0.823 mmol), and diisopropylethylamine ("DIEA", 431 µl, 2.468 mmol) in THF (8.2mL) at room temperature was added HATU (313 mg, 0.823 mmol). The resulting mixture was stirred at room temp for 1h. To the mixture was added ammonia in methanol (2M, 3 mL) (to break down any acyl sulfonamide that may result from over-coupling). The mixture was stirred for 30 min and then was concentrated in vacuo. The resulting residue was combined with a similar crude material prepared by the same procedure but using 25 mg of (S)-N-(7-(2-(1-amino-2-(3,5-difluorophenyl)ethyl)-7-hydroxy-4-oxoquinazolin-3(4H)-yl)-4-chloro-1-methyl-1H-indazol-3-yl)methanesulfonamide where all other reagents and solvents were scaled accordingly. The combined crude material was subjected to silica gel column chromatography (hexanes:EtOAc 100:0→0:100 over 10 CV) to afford N-((S)-1-(3-(4-chloro-1-methyl-3-(methylsulfonamido)-1H-indazol-7-yl)-7-hydroxy-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide, 472 mg (70%).¹H NMR (METHANOL-d₄, 500 MHz) δ 8.1-8.2 (m, 1H), 7.3-7.3 (m, 1H), 7.19 (d, 1H, J=1.5 Hz), 7.11 (br d, 2H, J=8.2 Hz), 6.7-6.8 (m, 2H), 6.5-6.6 (m, 2H), 3.61 (s, 3H), 2.4-2.5 (m, 3H), 1.3-1.4 (m, 2H), 1.2-1.3 (m, 6H), 1.1-1.2 (m, 1H), 1.0-1.0 (m, 1H).

### Preparation of N-((S)-1-(7-bromo-3-(4-chloro-3-(N-(4-methoxybenzyl)methylsulfonamido)-1-methyl-1H-indazol-7-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

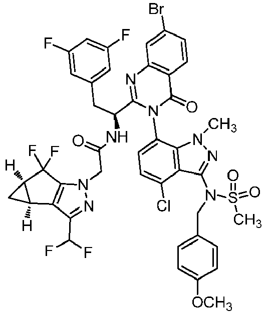

1-(chloromethyl)-4-methoxybenzene (0.276 mL, 2.036 mmol) was added to a stirred solution of N-((S)-1-(7-bromo-3-(4-chloro-1-methyl-3-(methylsulfonamido)-1H-indazol-7-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (1.5 g, 1.697 mmol) and cesium carbonate (0.553 g, 1.697 mmol) in N,N-Dimethylformamide (DMF) (10 mL) and the resulting mixture was stirred at room temp for 16 h. Water was then added and the mixture was extracted with ethyl acetate, washed with brine, dried (Na₂SO₄), filtered and concentrated. The residue was then subjected to silica gel column chromatography (hexanes:EtOAc 95:5→70:30) to afford N-((S)-1-(7-bromo-3-(4-chloro-3-(N-(4-methoxybenzyl)methylsulfonamido)-1-methyl-1H-indazol-7-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide, 1.4 g (82%). LCMS analysis conditions: Wavelength1: 220 nm; Wavelength2: 254 nm; Injection Vol.: 5.00 µl; Stop Time: 4.50 min; Grad. Time: 3.50 min; Start %B: 0; End %B: 100; Total Flow: 0.80 ml/min; Solvent A: 95:5 Water:MeCN with 0.1% TFA; Solvent B: 5:95 Water:MeCN with 0.1% TFA; Column = Acquity UPLC BEH C18, 2.1 × 100 mm, 1.7 µm. LCMS analysis result: retention time: 3.536 min, M+H: 1003.05.

### Preparation of N-((S)-1-(3-(4-chloro-3-(N-(4-methoxybenzyl)methylsulfonamido)-1-methyl-1H-indazol-7-yl)-4-oxo-7-(4,4,5,5-tetramethyl-1,3,2-dioxaborolan-2-yl)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

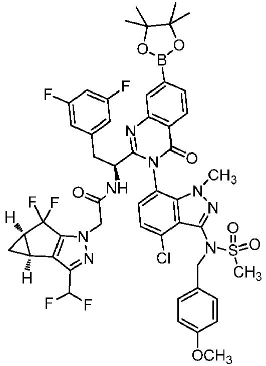

N-((S)-1-(7-bromo-3-(4-chloro-3-(N-(4-methoxybenzyl)methylsulfonamido)-1-methyl-1H-indazol-7-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (954 mg, 0.950 mmol), 4,4,4',4',5,5,5',5'-octamethyl-2,2'-bi(1,3,2-dioxaborolane) (362 mg, 1.425 mmol), potassium acetate (280 mg, 2.85 mmol) and PdCl₂(dppf) (69.5 mg, 0.095 mmol) were combined dry and degassed with Ar. Then they were taken up in dioxane (19 mL) and degassed again with argon and the resulting mixture was stirred at 60 °C overnight (16 h). The reaction mixture was concentrated, adsorbed onto Celite and, the resulting powder was subjected to silica gel column chromatography (hexanes:EtOAc 100:0→0:100 over 10 CVs) to afford N-((S)-1-(3-(4-chloro-3-(N-(4-methoxybenzyl)methylsulfonamido)-1-methyl-1H-indazol-7-yl)-4-oxo-7-(4,4,5,5-tetramethyl-1,3,2-dioxaborolan-2-yl)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide, 709 mg (71%). Under LCMS analysis both the boronic ester and the boronic acid are observed. However, 1H-NMR indicates that the product is entirely the boronic ester. LCMS analysis conditions: Wavelength1: 220 nm; Wavelength2: 254 nm; Injection Vol.: 5.00 µl; Stop Time: 2.50 min; Grad. Time: 1.50 min; Start %B: 0; End %B: 100; Total Flow: 0.80 ml/min; Solvent A: 95:5 Water:MeCN with 0.1% TFA; Solvent B: 5:95 Water:MeCN with 0.1% TFA; Column = Acquity UPLC BEH C18, 2.1 × 50 mm, 1.7um. LCMS analysis result: retention time: 1.495 min, M+H: 969.15; retention time: 1.760 min, M+H: 1051.25.

### Preparation of N-((S)-1-(3-(4-chloro-3-(N-(4-methoxybenzyl)methylsulfonamido)-1-methyl-1H-indazol-7-yl)-7-hydroxy-4-oxo-3, 4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

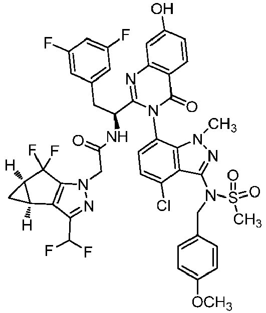

To a 20 mL scintillation vial charged with a solution ofN-((S)-1-(3-(4-chloro-3-(N-(4-methoxybenzyl)methylsulfonamido)-1-methyl-1H-indazol-7-yl)-4-oxo-7-(4,4,5,5-tetramethyl-1,3,2-dioxaborolan-2-yl)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (709 mg, 0.674 mmol) in THF (6.7 mL) was added aq. H₂O₂ (30%, 303 µL, 2.97 mmol). The mixture was stirred at room temperature for 1 h. The mixture was diluted with EtOAc and then washed with aq sat. sodium metabisulfite solution. The organic phase isolated, then concentrated in vacuo. The resulting residue was dissolved/suspended in DCM and then adsorbed onto Celite. The resulting powder was subjected to silica gel column chromatography (DCM:MeOH 100:0→90:10 over 10 CVs) to afford N-((S)-1-(3-(4-chloro-3-(N-(4-methoxybenzyl)methylsulfonamido)-1-methyl-1H-indazol-7-yl)-7-hydroxy-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide, 630 mg (99%). LCMS analysis conditions: Wavelength1: 220 nm; Wavelength2: 254 nm; Injection Vol.: 5.00 µl; Stop Time: 2.50 min; Grad. Time: 1.50 min; Start %B: 0; End %B: 100; Total Flow: 0.80 ml/min; Solvent A: 95:5 Water:MeCN with 0.1% TFA; Solvent B: 5:95 Water:MeCN with 0.1% TFA; Column = Acquity UPLC BEH, 2.1 × 50 mm, 1.7 µm. LCMS analysis result = retention time: 1.534 min, M+H: 941.10.

### Preparation of Example 1: N-((S)-1-((3P)-3-(4-chloro-1-methyl-3-(methylsulfonamido)-1H-indazol-7-yl)-4-oxo-7-(pyridin-2-ylmethoxy)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

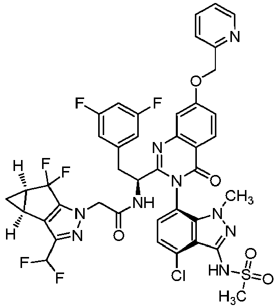

The title compound was prepared according to General Procedure A using (pyridin-2-yl)methanol as the coupling partner. The experiment afforded the title compound, N-((S)-1-((3P)-3-(4-chloro-1-methyl-3-(methylsulfonamido)-1H-indazol-7-yl)-4-oxo-7-(pyridin-2-ylmethoxy)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method F: retention time = 1.31 min.; observed ion = 912.4 (M+H). 1H NMR (500 MHz, METHANOL-d4) δ ppm 8.60 - 8.66 (m, 1 H), 8.23 (d, J=9.24 Hz, 1 H), 7.95 (td, J=7.75, 1.79 Hz, 1 H), 7.70 (d, J=8.05 Hz, 1 H), 7.45 (ddd, J=7.60, 4.92, 1.19 Hz, 1 H), 7.35 - 7.41 (m, 2 H), 7.28 (d, J=7.75 Hz, 1 H), 7.13 (d, J=7.75 Hz, 1 H), 6.55 - 6.84 (m, 4 H), 5.44 (s, 2 H), 4.81 - 4.85 (m, 1 H), 4.52 (s, 2 H), 3.59 (s, 3 H), 3.41 - 3.48 (m, 1 H), 3.24 (s, 3 H), 3.07 (dd, J=14.01, 9.24 Hz, 1 H), 2.44 (ddd, J=11.10, 7.67, 3.87 Hz, 2 H), 1.33 - 1.41 (m, 1 H), 0.98 - 1.03 (m, 1 H)

### Preparation of Example 2: N-((S)-1-((3P)-3-(4-chloro-1-methyl-3-(methylsulfonamido)-1H-indazol-7-yl)-7-(cyclopentylmethoxy)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

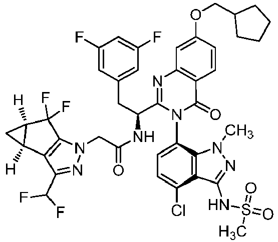

The title compound was prepared according to General Procedure A using cyclopentylmethanol as the coupling partner. The experiment afforded the title compound, N-((S)-1-((3P)-3-(4-chloro-1-methyl-3-(methylsulfonamido)-1H-indazol-7-yl)-7-(cyclopentylmethoxy)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method F: retention time = 1.61 min.; observed ion = 903.4 (M+H). NMR (500 MHz, METHANOL-d4) δ ppm 8.19 (d, J=8.64 Hz, 1 H), 7.31 (d, J=2.68 Hz, 1 H), 7.28 (d, J=7.75 Hz, 1 H), 7.24 (dd, J=8.79, 2.53 Hz, 1 H), 7.13 (d, J=7.75 Hz, 1 H), 6.59 - 6.82 (m, 4 H), 4.83 (dd, J=8.94, 5.36 Hz, 1 H), 4.49 - 4.57 (m, 2 H), 4.11 (d, J=6.85 Hz, 2 H), 3.60 (s, 3 H), 3.43 - 3.49 (m, 1 H), 3.24 (s, 3 H), 3.08 (dd, J=14.01, 8.94 Hz, 1 H), 2.47 - 2.54 (m, 1 H), 2.44 (ddd, J=11.18, 7.60, 4.17 Hz, 2 H), 1.91 - 1.99 (m, 2 H), 1.65 - 1.80 (m, 4 H), 1.45 - 1.53 (m, 2 H), 1.34 - 1.41 (m, 1 H), 0.99 - 1.04 (m, 1 H)

### Preparation of Example 3: N-((S)-1-((3P)-3-(4-chloro-1-methyl-3-(methylsulfonamido)-1H-indazol-7-yl)-7-(cyclohexylmethoxy)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

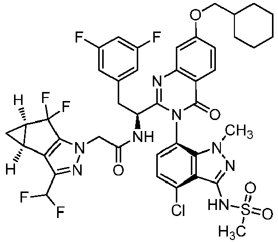

The title compound was prepared according to General Procedure A using cyclohexylmethanol as the coupling partner. The experiment afforded the title compound, N-((S)-1-((3P)-3-(4-chloro-1-methyl-3-(methylsulfonamido)-1H-indazol-7-yl)-7-(cyclohexylmethoxy)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method F: retention time = 1.66 min.; observed ion = 917.4 (M+H). 1H NMR (500 MHz, METHANOL-d4) δ ppm 8.18 (d, J=8.94 Hz, 1 H), 7.26 - 7.31 (m, 2 H), 7.23 (dd, J=8.79, 2.53 Hz, 1 H), 7.14 (d, J=8.05 Hz, 1 H), 6.58 - 6.83 (m, 4 H), 4.83 (dd, J=9.09, 5.22 Hz, 1 H), 4.53 (d, J=2.38 Hz, 2 H), 4.03 (d, J=6.26 Hz, 2 H), 3.60 (s, 3 H), 3.43 - 3.50 (m, 1 H), 3.24 (s, 3 H), 3.08 (dd, J=14.01, 9.24 Hz, 1 H), 2.44 (ddd, J=11.18, 7.60, 3.87 Hz, 2 H), 1.74 - 1.99 (m, 6 H), 1.25 - 1.45 (m, 4 H), 1.16 - 1.25 (m, 2 H), 0.99 - 1.05 (m, 1 H)

### Preparation of Example 4: N-((S)-1-((3P)-3-(4-chloro-1-methyl-3-(methylsulfonamido)-1H-indazol-7-yl)-7-(2-(1,1-dioxidothiomorpholino)ethoxy)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

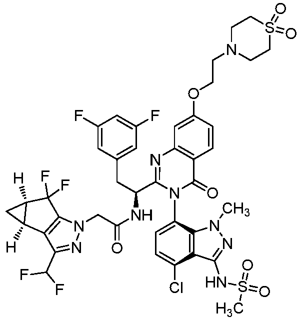

The title compound was prepared according to General Procedure A using 4-(2-hydroxyethyl)-1λ⁶-thiomorpholine-1,1-dione as the coupling partner. The experiment afforded the title compound, N-((S)-1-((3P)-3-(4-chloro-1-methyl-3-(methylsulfonamido)-1H-indazol-7-yl)-7-(2-(1,1-dioxidothiomorpholino)ethoxy)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method F: retention time = 1.22 min.; observed ion = 982.3 (M+H). 1H NMR (500 MHz, METHANOL-d4) δ ppm 8.21 (d, J=8.94 Hz, 1 H), 7.36 (d, J=2.38 Hz, 1 H), 7.26 - 7.30 (m, 2 H), 7.14 (d, J=7.75 Hz, 1 H), 6.59 - 6.82 (m, 4 H), 4.83 (dd, J=9.09, 5.22 Hz, 1 H), 4.58 - 4.63 (m, 1 H), 4.52 (s, 2 H), 4.36 - 4.40 (m, 2 H), 3.60 (s, 3 H), 3.44 - 3.50 (m, 1 H), 3.20 - 3.27 (m, 6 H), 3.15 - 3.19 (m, 4 H), 3.13 (t, J=5.22 Hz, 2 H), 3.09 (dd, J=14.01, 8.94 Hz, 1 H), 2.45 (ddd, J=11.10, 7.67, 3.87 Hz, 2 H), 1.35 - 1.41 (m, 1 H), 0.99 - 1.04 (m, 1H)

### Preparation of Example 5: N-((S)-1-((3P)-3-(4-chloro-1-methyl-3-(methylsulfonamido)-1H-indazol-7-yl)-4-oxo-7-(2-phenoxyethoxy)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

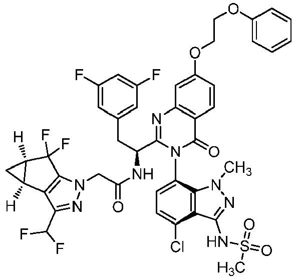

The title compound was prepared according to General Procedure A using 2-phenoxyethan-1-ol as the coupling partner. The experiment afforded the title compound, N-((S)-1-((3P)-3-(4-chloro-1-methyl-3-(methylsulfonamido)-1H-indazol-7-yl)-4-oxo-7-(2-phenoxyethoxy)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method F: retention time = 1.49 min.; observed ion = 941.4 (M+H). 1H NMR (500 MHz, METHANOL-d4) δ ppm 8.22 (br d, J=8.94 Hz, 1 H), 7.40 (br s, 1 H), 7.26 - 7.36 (m, 3 H), 7.11 - 7.20 (m, 1 H), 7.06 - 7.11 (m, 1 H), 7.03 (br d, J=8.35 Hz, 1 H), 6.95 - 7.01 (m, 1 H), 6.76 - 6.91 (m, 2 H), 6.57 - 6.71 (m, 2 H), 4.80 - 4.85 (m, 1 H), 4.40 - 4.64 (m, 4 H), 3.98 - 4.06 (m, 1 H), 3.74 - 3.91 (m, 3 H), 3.61 (s, 2 H), 3.42 - 3.52 (m, 1 H), 3.24 (s, 3 H), 3.02 - 3.14 (m, 1 H), 2.34 - 2.52 (m, 2 H), 1.33 - 1.40 (m, 1 H), 1.01 (br d, J=1.79 Hz, 1 H)

### Preparation of Example 6: N-((S)-1-((3P)-3-(4-chloro-1-methyl-3-(methylsulfonamido)-1H-indazol-7-yl)-7-(3,3-dimethylbutoxy)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

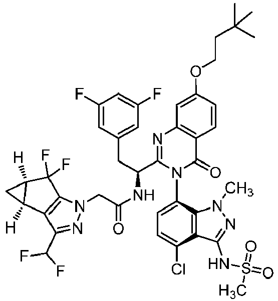

The title compound was prepared according to General Procedure A using 3,3-dimethylbutan-1-ol as the coupling partner. The experiment afforded the title compound, N-((S)-1-((3P)-3-(4-chloro-1-methyl-3-(methylsulfonamido)-1H-indazol-7-yl)-7-(3,3-dimethylbutoxy)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method F: retention time = 1.62 min.; observed ion = 905.4 (M+H). 1H NMR (500 MHz, METHANOL-d4) δ ppm 8.19 (d, J=8.94 Hz, 1 H), 7.31 (d, J=2.38 Hz, 1 H), 7.29 (d, J=7.75 Hz, 1 H), 7.23 (dd, J=8.94, 2.38 Hz, 1 H), 7.15 (d, J=8.05 Hz, 1 H), 6.58 - 6.83 (m, 4 H), 4.82 (dd, J=8.94, 5.07 Hz, 1 H), 4.54 (d, J=2.68 Hz, 2 H), 4.30 (t, J=7.00 Hz, 2 H), 3.60 (s, 3 H), 3.43 - 3.49 (m, 1 H), 3.24 (s, 3 H), 3.08 (dd, J=14.01, 9.24 Hz, 1 H), 2.44 (ddd, J=11.33, 7.60, 3.73 Hz, 2 H), 1.86 (t, J=7.15 Hz, 2 H), 1.34 - 1.41 (m, 1H), 1.09 (s, 9 H), 0.99 - 1.04 (m, 1H)

### Preparation of Example 7: N-((S)-1-((3P)-3-(4-chloro-1-methyl-3-(methylsulfonamido)-1H-indazol-7-yl)-7-(2-(1-methyl-1H-pyrazol-5-yl)ethoxy)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

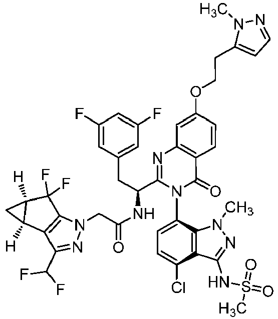

The title compound was prepared according to General Procedure A using 2-(1-methyl-1H-pyrazol-5-yl)ethan-1-ol as the coupling partner. The experiment afforded the title compound, N-((S)-1-((3P)-3-(4-chloro-1-methyl-3-(methylsulfonamido)-1H-indazol-7-yl)-7-(2-(1-methyl-1H-pyrazol-5-yl)ethoxy)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method F: retention time = 1.3 min.; observed ion = 929.4 (M+H). 1H NMR (500 MHz, METHANOL-d4) δ ppm 8.20 (d, J=8.94 Hz, 1 H), 7.43 (d, J=1.79 Hz, 1 H), 7.34 (d, J=2.38 Hz, 1 H), 7.23 - 7.31 (m, 2 H), 7.14 (d, J=7.75 Hz, 1 H), 6.59 - 6.82 (m, 4 H), 6.28 (d, J=2.09 Hz, 1 H), 4.82 (dd, J=9.09, 5.22 Hz, 1 H), 4.52 (d, J=1.49 Hz, 2 H), 4.47 - 4.51 (m, 2 H), 3.93 (s, 3 H), 3.60 (s, 3 H), 3.44 - 3.49 (m, 1 H), 3.29 - 3.32 (m, 2 H), 3.24 (s, 3 H), 3.08 (dd, J=14.01, 9.24 Hz, 1 H), 2.39 - 2.47 (m, 2 H), 1.34 - 1.41 (m, 1 H), 0.98 - 1.04 (m, 1 H)

### Preparation of Example 8: N-((S)-1-((3P)-3-(4-chloro-1-methyl-3-(methylsulfonamido)-1H-indazol-7-yl)-7-cyclobutoxy-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

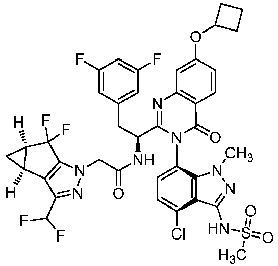

The title compound was prepared according to General Procedure A using cyclobutanol as the coupling partner. The experiment afforded the title compound, N-((S)-1-((3P)-3-(4-chloro-1-methyl-3-(methylsulfonamido)-1H-indazol-7-yl)-7-cyclobutoxy-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method F: retention time = 1.49 min.; observed ion = 875.4 (M+H). 1H NMR (500 MHz, METHANOL-d4) δ ppm 8.18 (d, J=9.24 Hz, 1 H), 7.27 (d, J=8.05 Hz, 1 H), 7.18 (s, 1 H), 7.18 (d, J=5.07 Hz, 1 H), 7.13 (d, J=7.75 Hz, 1 H), 6.58 - 6.82 (m, 4 H), 4.93 - 4.97 (m, 1 H), 4.83 (dd, J=9.09, 5.22 Hz, 1 H), 4.54 (d, J=2.98 Hz, 2 H), 3.59 (s, 3 H), 3.43 - 3.48 (m, 1 H), 3.23 (s, 3 H), 3.08 (dd, J=14.01, 9.24 Hz, 1 H), 2.58 - 2.66 (m, 2 H), 2.44 (ddd, J=11.18, 7.60, 3.87 Hz, 2 H), 2.22 - 2.32 (m, 2 H), 1.93 - 2.02 (m, 1 H), 1.80 - 1.91 (m, 1 H), 1.35 - 1.40 (m, 1 H), 0.99 - 1.04 (m, 1 H)

### Preparation of Example 9: N-((S)-1-((3P)-3-(4-chloro-1-methyl-3-(methylsulfonamido)-1H-indazol-7-yl)-7-(cyclopentyloxy)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

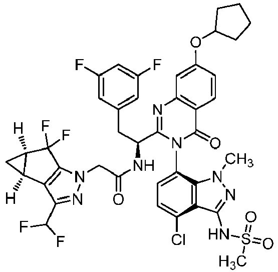

The title compound was prepared according to General Procedure A using cyclopentanol as the coupling partner. The experiment afforded the title compound, N-((S)-1-((3P)-3-(4-chloro-1-methyl-3-(methylsulfonamido)-1H-indazol-7-yl)-7-(cyclopentyloxy)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method F: retention time = 1.54 min.; observed ion = 889.4 (M+H). 1H NMR (500 MHz, METHANOL-d4) δ ppm 8.17 (d, J=8.94 Hz, 1 H), 7.27 - 7.31 (m, 2 H), 7.19 (dd, J=8.94, 2.38 Hz, 1 H), 7.14 (d, J=8.05 Hz, 1 H), 6.58 - 6.82 (m, 4 H), 5.05 - 5.10 (m, 1 H), 4.83 (dd, J=9.09, 5.22 Hz, 1 H), 4.54 (d, J=2.98 Hz, 2 H), 3.60 (s, 3 H), 3.43 - 3.48 (m, 1 H), 3.24 (s, 3 H), 3.09 (dd, J=13.86, 9.09 Hz, 1 H), 2.40 - 2.47 (m, 2 H), 2.06 - 2.15 (m, 2 H), 1.92 - 2.00 (m, 2 H), 1.83 - 1.91 (m, 2 H), 1.72 - 1.80 (m, 2 H), 1.35 - 1.40 (m, 1 H), 0.99 - 1.03 (m, 1 H)

### Preparation of Example 10: N-((S)-1-((3P)-3-(4-chloro-1-methyl-3-(methylsulfonamido)-1H-indazol-7-yl)-4-oxo-7-((tetrahydro-2H-pyran-4-yl)oxy)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

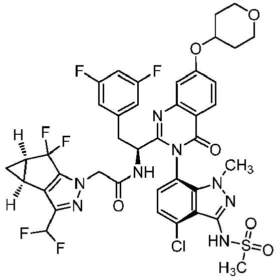

The title compound was prepared according to General Procedure A using oxan-4-ol as the coupling partner. The experiment afforded the title compound, N-((S)-1-((3P)-3-(4-chloro-1-methyl-3-(methylsulfonamido)-1H-indazol-7-yl)-4-oxo-7-((tetrahydro-2H-pyran-4-yl)oxy)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method F: retention time = 1.35 min.; observed ion = 905.4 (M+H). 1H NMR (500 MHz, METHANOL-d4) δ ppm 8.21 (d, J=8.94 Hz, 1 H), 7.35 (d, J=2.68 Hz, 1 H), 7.25 - 7.31 (m, 2 H), 7.14 (d, J=7.75 Hz, 1 H), 6.57 - 6.83 (m, 4 H), 4.82 (dd, J=9.09, 5.22 Hz, 1 H), 4.53 (d, J=1.19 Hz, 1 H), 4.00 - 4.07 (m, 2 H), 3.66 - 3.74 (m, 2 H), 3.60 (s, 3 H), 3.43 - 3.49 (m, 1 H), 3.24 (s, 3 H), 3.08 (dd, J=13.86, 9.09 Hz, 1 H), 2.40 - 2.47 (m, 2 H), 2.14 - 2.22 (m, 2 H), 1.81 - 1.91 (m, 2 H), 1.34 - 1.40 (m, 1 H), 0.98 - 1.04 (m, 1 H). 3 protons were not observed on the pyran ring (possibly hidden due to solvent peaks)

### Preparation of Example 11: N-((S)-1-((3P)-3-(4-chloro-1-methyl-3-(methylsulfonamido)-1H-indazol-7-yl)-4-oxo-7-(pyrimidin-5-ylmethoxy)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

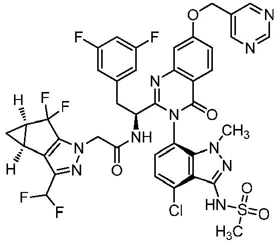

The title compound was prepared according to General Procedure A using (pyrimidin-5-yl)methanol as the coupling partner. The experiment afforded the title compound, N-((S)-1-((3P)-3-(4-chloro-1-methyl-3-(methylsulfonamido)-1H-indazol-7-yl)-4-oxo-7-(pyrimidin-5-ylmethoxy)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method E: retention time = 1.58 min.; observed ion = 913 (M+H). 1H NMR (500 MHz, METHANOL-d4) δ ppm 9.21 (s, 1 H), 9.02 (s, 2 H), 8.25 (d, J=8.94 Hz, 1 H), 7.47 (d, J=2.68 Hz, 1 H), 7.38 (dd, J=8.94, 2.68 Hz, 1 H), 7.28 (d, J=7.75 Hz, 1 H), 7.14 (d, J=7.75 Hz, 1 H), 6.58 - 6.83 (m, 4 H), 5.46 (s, 2 H), 4.83 - 4.86 (m, 1 H), 4.51 (s, 2 H), 3.61 (s, 3 H), 3.45 - 3.50 (m, 1 H), 3.24 (s, 3 H), 3.09 (dd, J=13.86, 9.09 Hz, 1 H), 2.41 - 2.48 (m, 2 H), 1.35 - 1.41 (m, 1 H), 0.99 - 1.04 (m, 1 H)

### Preparation of Example 12: N-((S)-1-((3P)-3-(4-chloro-1-methyl-3-(methylsulfonamido)-1H-indazol-7-yl)-7-(cyclobutylmethoxy)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

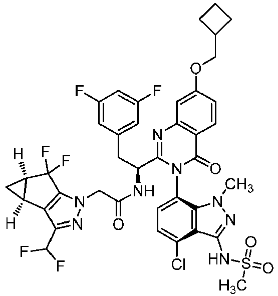

The title compound was prepared according to General Procedure A using cyclobutylmethanol as the coupling partner. The experiment afforded the title compound, N-((S)-1-((3P)-3-(4-chloro-1-methyl-3-(methylsulfonamido)-1H-indazol-7-yl)-7-(cyclobutylmethoxy)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method F: retention time = 1.55 min.; observed ion = 889.4 (M+H). 1H NMR (500 MHz, METHANOL-d4) δ ppm 8.19 (d, J=8.64 Hz, 1 H), 7.31 (d, J=2.38 Hz, 1 H), 7.28 (d, J=7.75 Hz, 1 H), 7.24 (dd, J=8.79, 2.53 Hz, 1 H), 7.14 (d, J=7.75 Hz, 1 H), 6.58 - 6.83 (m, 4 H), 4.83 (dd, J=8.94, 5.07 Hz, 1 H), 4.53 (d, J=2.09 Hz, 2 H), 4.19 (dd, J=6.41, 1.04 Hz, 2 H), 3.60 (s, 3 H), 3.46 (dd, J=13.86, 5.22 Hz, 1 H), 3.24 (s, 3 H), 3.08 (dd, J=13.86, 9.09 Hz, 1 H), 2.87 - 2.96 (m, 1 H), 2.44 (ddd, J=11.18, 7.60, 3.87 Hz, 2 H), 2.18 - 2.29 (m, 2 H), 1.95 - 2.12 (m, 4 H), 1.34 - 1.41 (m, 1 H), 1.02 (dtd, J=5.74, 3.91, 3.91, 2.24 Hz, 1 H)

### Preparation of Example 13: N-((S)-1-(7-(2-(1H-pyrazol-1-yl)ethoxy)-(3P)-3-(4-chloro-1-methyl-3-(methylsulfonamido)-1H-indazol-7-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

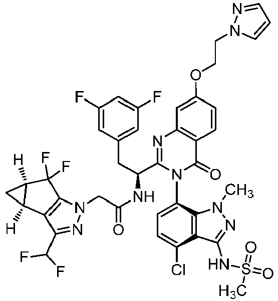

The title compound was prepared according to General Procedure A using 2-(1H-pyrazol-1-yl)ethan-1-ol as the coupling partner. The experiment afforded the title compound, N-((S)-1-(7-(2-(1H-pyrazol-1-yl)ethoxy)-(3P)-3-(4-chloro-1-methyl-3-(methylsulfonamido)-1H-indazol-7-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method F: retention time = 1.31 min.; observed ion = 915.4 (M+H). 1H NMR (500 MHz, METHANOL-d4) δ ppm 8.21 (d, J=8.94 Hz, 1 H), 7.35 (d, J=2.68 Hz, 1 H), 7.25 - 7.31 (m, 2 H), 7.14 (d, J=7.75 Hz, 1 H), 6.57 - 6.83 (m, 4 H), 4.82 (dd, J=9.09, 5.22 Hz, 1 H), 4.53 (d, J=1.19 Hz, 1 H), 4.00 - 4.07 (m, 2 H), 3.66 - 3.74 (m, 2 H), 3.60 (s, 3 H), 3.43 - 3.49 (m, 1 H), 3.24 (s, 3 H), 3.08 (dd, J=13.86, 9.09 Hz, 1 H), 2.40 - 2.47 (m, 2 H), 2.14 - 2.22 (m, 2 H), 1.81 - 1.91 (m, 2 H), 1.34 - 1.40 (m, 1 H), 0.98 - 1.04 (m, 1 H)

### Preparation of N-((S)-1-((3P)-3-(4-chloro-1-methyl-3-(N-(methylsulfonyl)acetamido)-1H-indazol-7-yl)-7-hydroxy-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

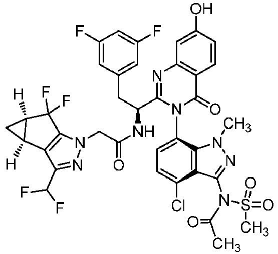

To a solution ofN-((S)-1-((3P)-3-(4-chloro-1-methyl-3-(methylsulfonamido)-1H-indazol-7-yl)-4-oxo-7-(4,4,5,5-tetramethyl-1,3,2-dioxaborolan-2-yl)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (1.12 g, 1.203 mmol) in Tetrahydrofuran (THF) (10 mL) was added acetic anhydride (0.125 mL, 1.323 mmol). The solution was stirred at room temperature for 24 h. To the solution was added additional acetic anhydride (0.125 mL, 1.323 mmol) and the solution was stirred at room temperature for 4 days. To the solution was added acetic anhydride (0.063 mL, 0.66 mmol) and the solution was stirred for 7 days. To the reaction was added aq. hydrogen peroxide (0.614 mL, 6.01 mmol) and the mixture was then stirred at room temperature for 3 hrs. The mixture was diluted with EtOAc and then was washed with aq. sat. sodium metabisulfite (50 mL). The organic layer was washed with brine, dried (MgSO₄), filtered and then concentrated in vacuo to afford the crude product. The crude product was purified via silica gel chromatography (40 g RediSep Gold column, 30-75% EtOAc in Hexanes) to afford the product N-((S)-1-((3P)-3-(4-chloro-1-methyl-3-(N-(methylsulfonyl)acetamido)-1H-indazol-7-yl)-7-hydroxy-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (947 mg, 1.097 mmol, 91 % yield) as a light yellow solid. The sample was analyzed using the following LCMS analysis method: Column = Acquity BEH C18, 2.1 x 100 mm, 1.7 µm particles; Solvent A = 0.1% formic acid in water:acetonitrile (95:5); Solvent B = 0.1% formic acid in water: acetonitrile (5:95); Flow rate = 0.80 mL/min.; Start % B = 0; End % B = 100%; Gradient time = 3.5 min. followed by a 1 min. hold at 100% B; Detection = 220 and 254 nm. LCMS retention time = 2.927 min.; observed ion = 863.10.

### Preparation of Example 14: N-((S)-1-((3P)-3-(4-chloro-1-methyl-3-(methylsulfonamido)-1H-indazol-7-yl)-4-oxo-7-(4,4,4-trifluoro-3,3-dimethylbutoxy)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

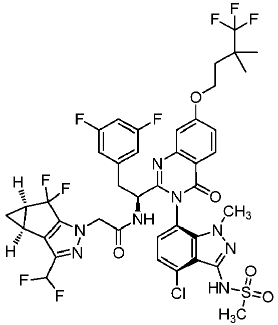

To a mixture of N-((S)-1-((3P)-3-(4-chloro-1-methyl-3-(N-(methylsulfonyl)acetamido)-1H-indazol-7-yl)-7-hydroxy-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (30 mg, 0.035 mmol) and R-OH (0.104 mmol) was added a solution of triphenylphosphine (27.3 mg, 0.104 mmol) in THF (0.25 mL). The solution was stirred for 5 min. To the solution was added diisopropyl (E)-diazene-1,2-dicarboxylate (0.021 mL, 0.104 mmol) as a solution in THF (0.25 mL). The solution was stirred at ambient temp for 18h. To the solution was added ammonia in methanol (2M, 1 mL). The solution was stirred for 3h. The solution was concentrated under a stream of nitrogen gas and the resulting sticky residue was dissolved in DMF (2 mL) and subjected to HPLC purification to afford the title compound, N-((S)-1-((3P)-3-(4-chloro-1-methyl-3-(methylsulfonamido)-1H-indazol-7-yl)-4-oxo-7-(4,4,4-trifluoro-3,3-dimethylbutoxy)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method F: retention time = 1.63 min.; observed ion = 959.3 (M+H). 1H NMR (500 MHz, METHANOL-d4) δ ppm 8.21 (d, J=8.64 Hz, 1 H), 7.32 (d, J=2.38 Hz, 1 H), 7.22 - 7.30 (m, 2 H), 7.14 (d, J=7.75 Hz, 1 H), 6.59 - 6.82 (m, 4 H), 4.54 (d, J=2.09 Hz, 2 H), 4.37 (t, J=6.85 Hz, 2 H), 3.60 (s, 3 H), 3.43 - 3.48 (m, 1 H), 3.24 (s, 3 H), 3.08 (dd, J=14.16, 9.09 Hz, 1 H), 2.44 (ddd, J=11.40, 7.82, 4.02 Hz, 2 H), 2.15 (t, J=6.85 Hz, 2 H), 1.35 - 1.41 (m, 1H), 1.31 (s, 6 H), 1.02 (ddt, J=5.51, 3.80, 2.05, 2.05 Hz, 1 H)

### Preparation of Example 15: N-((S)-1-((3P)-3-(4-chloro-1-methyl-3-(methylsulfonamido)-1H-indazol-7-yl)-7-(2-(difluoromethoxy)ethoxy)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

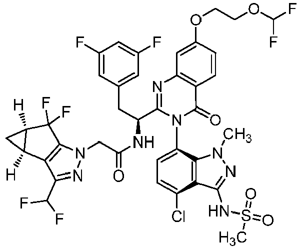

To a mixture of N-((S)-1-((3P)-3-(4-chloro-1-methyl-3-(N-(methylsulfonyl)acetamido)-1H-indazol-7-yl)-7-hydroxy-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (30 mg, 0.035 mmol) and 2-(difluoromethoxy)ethan-1-ol (11.69 mg, 0.104 mmol) was added a solution of triphenylphosphine (27.3 mg, 0.104 mmol) in THF (0.25 mL). The solution was stirred at room temperature for 5 min. To the solution was added a solution of diisopropyl (E)-diazene-1,2-dicarboxylate (0.021 mL, 0.104 mmol) in THF (0.25 mL). The solution was stirred at room temperature for 18h. To the solution was added ammonia in MeOH (2M, 1 mL) and the solution was stirred for 1h. The solution was concentrated under a stream of nitrogen gas and the resulting sticky residue was dissolved in DMF (2 mL) and subjected to HPLC purification to afford the title compound, N-((S)-1-((3P)-3-(4-chloro-1-methyl-3-(methylsulfonamido)-1H-indazol-7-yl)-7-(2-(difluoromethoxy)ethoxy)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide. The sample was analyzed using LCMS Method F: retention time = 1.43 min.; observed ion = 915.2 (M+H). 1H NMR (500 MHz, METHANOL-d4) δ ppm 8.22 (d, J=8.64 Hz, 1 H), 7.36 (d, J=2.38 Hz, 1 H), 7.29 (dd, J=8.79, 2.53 Hz, 2 H), 7.14 (d, J=8.05 Hz, 1 H), 6.37 - 6.82 (m, 5 H), 4.53 (d, J=1.19 Hz, 2 H), 4.41 - 4.46 (m, 2 H), 4.27 - 4.35 (m, 2 H), 3.61 (s, 3 H), 3.44 - 3.50 (m, 1 H), 3.24 (s, 3 H), 3.09 (dd, J=13.86, 9.09 Hz, 1 H), 2.44 (ddd, J=11.33, 7.60, 4.02 Hz, 2 H), 1.34 - 1.40 (m, 1H), 0.98 - 1.04 (m, 1 H)

### Alternate preparation of N-(7-amino-4-chloro-1-methyl-1H-indazol-3-yl)-N-(4-methoxybenzyl)methanesulfonamide.

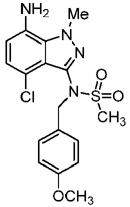

### Synthesis Scheme:

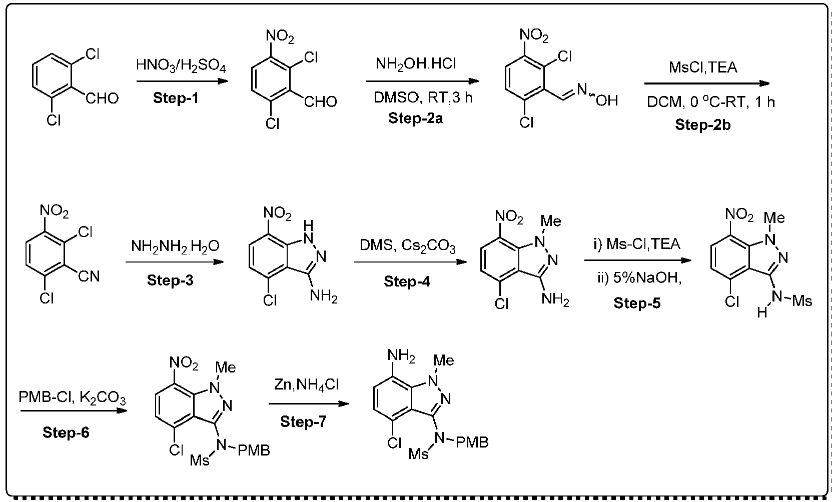

### Step 1: Preparation of 2,6-dichloro-3-nitrobenzaldehyde

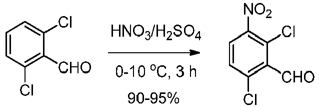

To a solution of sulfuric acid (H₂SO₄) (5.63 L, 4.5 V) in a round-bottom flask at 0-5 °C was added 2,6-dichlorobenzaldehyde (1.25 kg, 7.10 mol, 1.0 equiv.) in portions at below 15 °C. The reaction mass was stirred at 0-5 °C for 30 min. A solution of freshly prepared nitration mixture [Prepared from Conc. H₂SO₄ (0.425 L, 0.34 V) and 70% HNO₃ (0.85 kg, 13.49 mol, 1.30 equiv.) at 0 °C] was added to the above reaction mixture at below 10 °C [Note: Reaction is slightly exothermic (3-6 °C); so that addition is preferred at lower temperature]. The reaction mixture was stirred at 5-10 °C for 2-3 h. After completion of the reaction (monitored by TLC), it was quenched with ice cold water (18.75 L, 15 V) at below 25 °C. Then the reaction mass was allowed warm to room temperature and stirred for 2 h. The solids were isolated by filtration and then were washed with water (2.5 L, 2.0 V). Bulk residual water was removed from the solids by maintaining vacuum filtration for 60-90 min. The crude wet solid was initially dried under air atmosphere; then in a hot air oven at 50-55 °C for 10-12 h (until moisture content is not more than 5.0 %) to get the dried title product, 2,6-dichloro-3-nitrobenzaldehyde (1.44 kg, 92% yield) as a yellow solid. ¹H NMR (400 MHz, CDCl₃): *δ* 10. 44 (s, 1H), 7.88 (d, *J=* 8.4 Hz, 1H), 7.56 (d, *J=* 8.8 Hz, 1H).

### Step 2: Preparation of 2,6-dichloro-3-nitrobenzonitrile

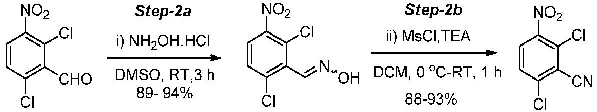

(Step-2a) To a solution of DMSO (5.9 L, 5.0 V)) in a round-bottom flask was added 2,6-dichloro-3-nitrobenzaldehyde (1.17 kg, 5.31 mol, 1.0 equiv.) at room temperature. After being stirred for 30 min at room temperature, hydroxylamine hydrochloride (0.63 kg, 9.04 mol, 1.70 equiv.) was added and the reaction mass was stirred at room temperature for 3 h. After completion of the reaction (monitored by TLC), the reaction mass was quenched by the addition of ice-cold water (18.0 L, 15.0 V) added at a rate sufficient to maintain the temperature below 30 °C (Observation: Solids formed upon water addition). The reaction mass was stirred at room temperature for 60-90 min. The solids were isolated by filtration; washed with water (2.5 L, 2.0 V); followed by washing with a mixture of acetone and hexanes (6.0 L, 1: 1 ratio). Bulk residual water was removed from the solids by maintaining vacuum filtration for 60-90 min. The wet solid was initially air dried and then finally dried in a hot air oven at 50-55 °C for 10-12 h (until moisture content was not more than 1.0 %) to get the dried target product, 2,6-dichloro-3-nitrobenzaldehyde oxime (1.22 kg, 92% yield) as an off-white solid. The crude product (which contains 10-20% of 2,6-dichloro-3-nitrobenzonitrile) was used directly in the next step without further purification. (Step-2b) To a stirred solution of the crude oxime (preparation described above, 1.13 kg, 4.80 mol, 1.0 equiv.) in DCM (9.04 L, 8.0 V) at 0-5 °C was added triethylamine ("TEA", 1.02 kg, 10.09 mol, 2.1 equiv.). After being stirred for 5 min, methanesulfonyl chloride (0.60 kg, 5.29 mol, 1.1 equiv.) was added (Observation: An exotherm is noted during the addition) slowly at 15 °C. Then the reaction mass was stirred at room temperature for 30-45 min. After completion of the reaction (progress of reaction was monitored by TLC; mobile phase: 20% ethyl acetate in hexanes), the reaction mass was diluted with water (6.78 L, 6.0 V); the organic layer was separated; and the aqueous layer was extracted with DCM (3.4 L, 3.0 V). The combined organic layers were washed with brine (5.65 L, 5.0 V); dried over Na₂SO₄; and concentrated under vacuum. The resulting crude solids were triturated with hexanes (4.50 L, 4.0 V) at room temperature. The wet material was dried in a hot air oven at 50-55 °C for 5- 6 h to get the dried product, 2,6-dichloro-3-nitrobenzonitrile (0.95 kg, 91% yield) as a yellow solid. ¹H NMR (400 MHz, CDCl₃): *δ* 8.07 (d, *J=* 8.8 Hz, 1H), 7.63 (d, *J=* 8.8 Hz, 1H).

### Step 3: Preparation of 4-chloro-7-nitro-1H-indazol-3-amine

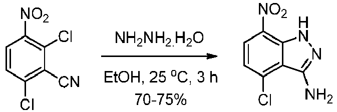

To a stirred solution of 2,6-dichloro-3-nitrobenzonitrile (750.0 g, 3.45 mol, 1.0 equiv.) in ethanol (7.5 L, 10.0 V) at 15-20 °C. was slowly added hydrazine hydrate (519.0 g, 10.36 mol, 3.0 equiv.) while maintaining the reaction mass below 25 °C (Observation: Addition is slightly exothermic and solid formation will begin upon addition). The reaction mixture temperature was slowly raised to room temperature and then the mixture was stirred for 3 h (Observation: the quantity of solids will increase during this time). After completion of the reaction (monitored by TLC), the mixture was diluted with water (7.5 L, 10.0 V) and further stirred for 1 h at room temperature. The solids were isolated via filtration and then were washed with water (2.25 L, 3.0 V). The wet solid was washed with a 1:1 ratio mixture of acetone (1.875 L, 2.5 V) and hexanes (1.875 L, 2.5 V). Bulk residual water was removed from the solids by maintaining vacuum filtration for 60-90 min. The wet solid was finally dried in a hot air oven for 7-8 h at 50 °C (until moisture content reaches below 1.5%) to get the dried product, 4-chloro-7-nitro-1*H*-indazol-3-amine (549.0 g, 75% yield) as a brick red-colored solid. ¹H NMR (400 MHz, CDCl₃): *δ* 10.36 (bs, 1H), 8.20 (d, *J=* 8.4 Hz, 1H), 7.07 (d, *J=* 8.40 Hz, 1H), 4.73 (bs, 2H).

### Step 4: Preparation of 4-chloro-1-methyl-7-nitro-1H-indazol-3-amine

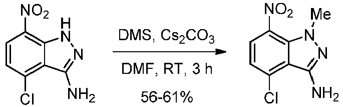

To a stirred solution of 4-chloro-7-nitro-1*H*-indazol-3-amine (500 g, 0.42 mol, 1.0 equiv.) in DMF (5.0 L, 10.0 V) at 5-10 °C was slowly added cesium carbonate (Cs₂CO₃) (1.91 kg, 5.88 mol, 2.5 equiv.) while maintaining the reaction mass below 10 °C. After being stirred for 5-10 min, dimethyl sulphate (326.3 g, 2.59 mol, 1.1 equiv.) was added while maintaining the reaction mass below 10 °C (Note: Slow addition is preferred for obtaining more favorable regio-selectivity). Then, the reaction temperature was slowly raised to room temperature and stirring was continued an additional 2 h at the same temperature. After completion of the reaction (monitored by TLC), the reaction mass was quenched by the addition of ice-cold water (15.0 L, 30.0 V) and the resulting mixture was then stirred for 6-8 h at room temperature. The solids were isolated via filtration and were then washed with water (1.5 L, 3.0 V). The wet solid was washed with IPA (1.5 L, 3.0 V) followed by hexanes (1.0 L, 2.0 V). Bulk residual water was removed from the solids by maintaining vacuum filtration for 60-90 min. The wet solid was dried in a hot air oven for 7-8 h at 50 °C (until moisture content is below 1.0%). The isolated material, 4-chloro-1-methyl-7-nitro-1*H-*indazol-3-amine (319.0 g, 60% yield), was used in the next step without further purification. ¹H NMR (400 MHz, CDCl₃): *δ* 7.97 (d, *J=* 8.32 Hz, 1H), 6.97 (d, *J* = 8.24 Hz, 1H), 4.63 (bs, 2H), 3.96 (s, 3H).

### Step 5: Preparation of N-(4-chloro-1-methyl-7-nitro-1H-indazol-3-yl)methanesulfonamide

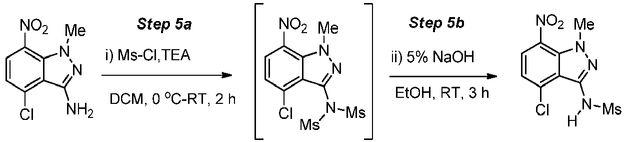

(Step 5a) To a solution of 4-chloro-1-methyl-7-nitro-1*H*-indazol-3-amine (625.0 g, 2.76 mol, 1.0 equiv.) in DCM (6.25 L, 10.0 V) at 0-5 °C. was added triethylamine (TEA) (837.0 g, 8.27 mol, 3.0 equiv.); followed by the addition of 4-dimethylaminopyridine (DMAP) (20.60 g, 0.165 mol, 0.06 equiv.). The reaction mass was stirred for 5-10 min., then methanesulfonyl chloride (MsCl) (790.0 g, 6.89 mol, 2.5 equiv.) added slowly while maintaining the reaction mass below 10 °C. The reaction mixture was allowed to warm to room temperature and was then stirred for 1.5-2.0 h. After completion of the reaction (monitored by TLC), the mixture was diluted with water (6.25 L, 10.0 V) and then stirred at room temperature for 15 min. The organic layer was separated, and the aqueous layer was extracted with DCM (6.25 L, 10.0 V). The combined organic layers were washed with brine (1.25 L, 2.0 V), dried over Na₂SO₄ and concentrated to get the crude solids. The solids were triturated with hexanes (1.25 L, 2.0 V) at room temperature to obtain the intermediate, N-(4-chloro-1-methyl-7-nitro-1H-indazol-3-yl)-N-(methylsulfonyl)methanesulfonamide, which was used directly in the next step. (ii) To a stirred solution of N-(4-chloro-1-methyl-7-nitro-1H-indazol-3-yl)-N-(methylsulfonyl)methanesulfonamide (prepared above) in ethanol (10.5 L, 20.0 V) at room temperature was added slowly an aq. 5% NaOH solution (4.38 L, 7.0 V) [Note: Slow addition is preferred via dropping funnel]. The reaction mass was stirred at the same temperature for 3 h. After completion of the reaction (monitored by TLC) [Sample preparation for TLC analysis: ~1.0 ml of sample acidified with aq. 2.0 N HCl to reach the pH: 2-3, extract it with ethyl acetate and analyze the organic layer by TLC], the reaction mass was cooled to 0-5 °C and the pH was adjusted to 2-3 by the addition of aq. 2.0 N HCl (3.13 L, 5.0 V) while maintain the reaction temperature below 10 °C [Note: Precipitation occurred upon addition of HCl and increased with stirring]. The reaction mixture was warmed to room temperature and then stirred for 1.5-2.0 h. Solids obtained were isolated via filtration and were then washed with water (1.25 L, 2.0 V); followed by washing with hexanes (1.25 L, 2.0 V). Bulk residual water was removed from the solids by maintaining vacuum filtration for 60-90 min. The wet material was dried in a hot air oven at 50 °C for 6-7 h (Until the moisture content is below 1.0%) to get the dried product, *N*-(4-chloro-1-methyl-7-nitro-1*H*-indazol-3-yl)methanesulfonamide (640.0 g, 76%) as a yellow solid. ¹H NMR (400 MHz, CDCl₃): *δ* 8.05 (d, *J=* 8.32 Hz, 1H), 7.32 (bs, 1H), 7.17 (d, *J* = 8.28 Hz, 1H), 4.15 (s, 3H), 3.45 (s, 3H).

### Step 6: Preparation of N-(4-chloro-1-methyl-7-nitro-1H-indazol-3-yl)-N-(4-methoxybenzyl)methanesulfonamide

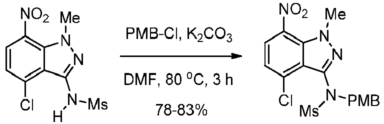

To a mixture of *N*-(4-chloro-1-methyl-7-nitro-1*H*-indazol-3-yl)methanesulfonamide (635.0 g, 2.08 mol, 1.0 equiv.) and 1-(chloromethyl)-4-methoxybenzene (359.0 g, 2.30 mol, 1.1 equiv.) in DMF (6.35 L, 10.0 V) at room temperature was added potassium carbonate (374.7 g, 2.70 mol, 1.3 equiv.). The reaction mixture was heated to 80-90 °C and maintained at that temperature for 3 h. After completion of the reaction (monitored by TLC), the mixture was poured into ice cold water (19.05 L, 30.0 V) [Note: Slow quenching with vigorous stirring is preferred to avoid clumping as the product precipitates]. The resulting solids were isolated via filtration and washed with water (1.90 L, 3.0 V); then the solids were washed with hexanes (1.27 L, 2.0 V). Bulk residual water was removed from the solids by maintaining vacuum filtration for 60-90 min. The isolated solid was dissolved in Ethyl acetate (12.7 L, 20.0 V) and charcoal was added (63.5 g). The mixture was heated to 60-70 °C and then stirred for 30-45 min. at that temperature. The mixture was filtered while still hot (40-50 °C) through a pad of Celite and the Celite pad was then extracted with ethyl acetate (3.17 L, 5.0 V). The combined filtrates were concentrated to dryness under reduced pressure at below 50 °C. Ethyl acetate (0.635 L, 1.0 V) was added to the solids at room temperature. The resultant solid suspension was stirred for 30 min. The solids were isolated via filtration and then were washed with hexanes (1.27 L, 2.0 V). Residual water was removed from the solids by maintaining vacuum filtration for 45-60 min. to afford the product N-(4-chloro-1-methyl-7-nitro-1H-indazol-3-yl)-N-(4-methoxybenzyl) methane sulfonamide (705.0 g, 80% yield) as a yellow solid. ¹H NMR (400 MHz, CDCl₃): *δ* 7.99 (d, *J=* 8.24 Hz, 1H), 7.27 (d, *J* = 8.68 Hz, 2H), 7.19 (d, *J=* 8.24 Hz, 1H), 6.80 (d, *J=* 8.44 Hz, 2H), 4.95-4.76 (m, 2H), 4.17 (s, 3H), 3.76 (s, 3H), 3.01 (s, 3H).

### Step 7: Preparation of N-(7-Amino-4-chloro-1-methyl-1H-indazol-3-yl)-N-(4-methoxybenzyl)methanesulfonamide

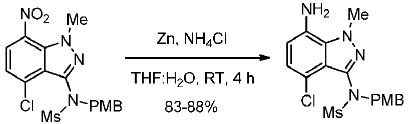

To a stirred suspension of zinc powder (540.0 g, 8.23 mol, 10.0 equiv.) in a mixture of THF (3.50 L, 10.0 V) and water (7.0 L, 20.0 V) at room temperature was added ammonium chloride (NH₄Cl) (449.0 g, 8.23 mol, 10.0 equiv.). To the mixture was added *N-*(4-chloro-1-methyl-7-nitro-1*H*-indazol-3-yl)-*N*-(4-methoxybenzyl)methanesulfonamide (350 g, 0.823 mol, 1.0 equiv.) in THF (7.0 L, 20.0 V). The reaction mixture was stirred at room temperature for 3-4 h. After completion of the reaction (monitored by in-process TLC/HPLC), the mixture was diluted with ethyl acetate (3.5 L, 10.0 V) and water (1.12 L, 2.5 V). The mixture was stirred for 15 min. The reaction mass was filtered through a pad of Celite bed washing with ethyl acetate (1.75 L, 5.0 V). The bi-phasic filtrate was collected, and the phases were separated. The aqueous layer was extracted with ethyl acetate (3.50 L, 10.0 V). The combined organic layers were washed with brine (3.50 L, 10 V), dried over Na₂SO₄, and then concentrated in *vacuo* to afford a crude solid. To the crude product was added MTBE (3.25 L, 10 V) and the suspension was stirred for 30 min at room temperature. The solids were isolated by filtration. Bulk residual water was removed from the solids by maintaining vacuum filtration for 30-45 min. The wet product was dried in a hot air oven (50 °C) for 2 h to afford the title product, *N-*(7-amino-4-chloro-1-methyl-1*H*-indazol-3-yl)-*N-*(4-methoxybenzyl)methanesulfonamide (276.0 g, 85% yield) as off-white solid. ¹H NMR (400 MHz, CDCl₃): *δ* 7.29-7.26 (m, 2H), 6.86-6.79 (m, 2H), 6.42 (d, *J=* 7.80 Hz, 1H), 4.99-4.70 (m, 2H), 4.25 (s, 3H), 3.77 (s, 5H), 2.98 (s, 3H).

### Alternate preparation of N-(7-amino-4-chloro-1-(2,2-difluoroethyl)-1H-indazol-3-yl)-N-(4-methoxybenzyl)methanesulfonamide:

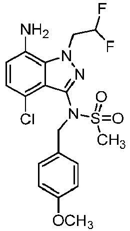

### Synthesis Scheme:

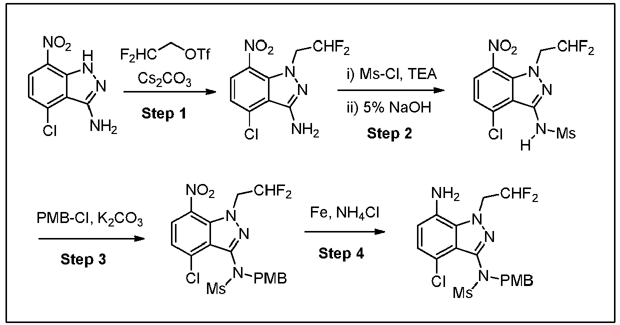

### Step 1: Preparation of 4-chloro-1-(2,2-difluoroethyl)-7-nitro-1H-indazol-3-amine

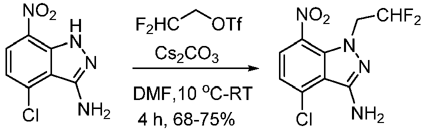

To a stirred solution of 4-chloro-7-nitro-1*H*-indazol-3-amine (180 g, 0.85 mol, 1.0 equiv.) in DMF (1.8 L, 10.0 V) at 10-15 °C was added cesium carbonate (Cs₂CO₃) (551 g, 1.70 mol, 2.0 equiv.) at a rate necessary to maintaining the reaction mass below 20 °C. The mixture was stirred for 5-10 min, then to the stirred mixture at 10-15 °C was added 2,2-difluoroethyl trifluoromethanesulfonate (133 mL, 0.93 mol, 1.1 equiv.) at a rate necessary to maintain the reaction mass below 20 °C (Note: Slow addition is preferred to obtain more favorable regio-selectivity). The reaction mass was allowed to slowly warm to room temperature and was then stirred at the same temperature for 3 h. After completion of the reaction (monitored by TLC), the reaction mass was quenched by the addition of ice-cold water (5.4 L, 30.0 V) and the resulting mixture was allowed to warm to room temperature with stirring for 6-8 h. The solids were isolated via filtration and were then washed with water (540 mL, 3.0 V). The wet solid was washed with hexanes (0.9 L, 5.0 V). Bulk residual water was removed from the solids by maintaining vacuum filtration for 60-90 min. The wet solid was dried in a hot air oven for 7-8 h at 50 °C (until the moisture content was below 1.0%). The isolated material, 4-chloro-1-(2,2-difluoroethyl)-7-nitro-1*H*-indazol-3-amine (160 g, 71% yield), was used in the next step without further purification. ¹H NMR (400MHz, CDCl₃): *δ* 8.05 (d, *J=* 8.4 Hz, 1H), 7.07 (d, *J=* 8.4 Hz, 1H), 6.00 (tt, *J₁* = 3.9 Hz, *J₂ =* 7.7 Hz, 1H), 4.76 - 4.84 (m, 4H).

### Step 2: Preparation of N-(4-chloro-1-(2,2-difluoroethyl)-7-nitro-1H-indazol-3-yl)methane sulfonamide

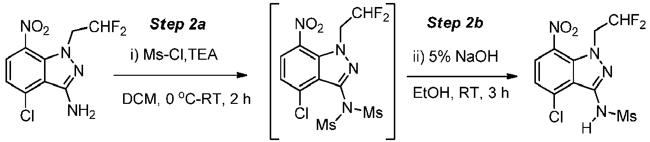

Step 2a: To a solution of 4-chloro-1-(2,2-difluoroethyl)-7-nitro-1*H*-indazol-3-amine (170.0 g, 0.96 mol, 1.0 equiv.) in DCM (1.7 L, 10.0 V) at 0-5 °C was added triethyl amine (264 mL, 2.88 mol, 3.0 equiv.), followed by 4-dimethylaminopyridine (3.4 g, 0.048 mol, 0.05 equiv.). The reaction mass was stirred for 5-10 min., then methanesulfonyl chloride (120 mL, 2.4 mol, 2.5 equiv.) was added slowly while maintaining the reaction mass below 10 °C. The reaction mixture was allowed to warm to room temperature and then was stirred for 1.5-2.0 h. After completion of the reaction (monitored by TLC), the mixture was diluted with water (1.7 L, 10.0 V) and then stirred at room temperature for 15 min. The organic layer was separated, and the aqueous layer was extracted with DCM (1.7 L, 10.0 V). The combined organic layers were washed with 10% brine solution (340 mL, 2.0 V), dried over Na₂SO₄ and concentrated to afford the product as a crude solid. The solids were triturated with hexanes (340 mL, 2.0 V) at room temperature to obtain *N*-(4-chloro-1-(2,2-difluoroethyl)-7-nitro-1*H*-indazol-3-yl)-N-(methylsulfonyl) methanesulfonamide which was used directly in the next step. Step 2b: To a stirred solution of *N*-(4-chloro-1-(2,2-difluoroethyl)-7-nitro-1H-indazol-3-yl)-*N*-(methylsulfonyl) methanesulfonamide (entirety of material prepared above) in ethanol (1.7 L, 10.0 V) at room temperature was added slowly aq. 5% NaOH solution (1.19 L, 7.0 V) [Note: Slow addition is preferred via dropping funnel]. The reaction mass was stirred at the same temperature for 3 h. After completion of the reaction [Sample preparation for TLC analysis: an aliquot of reaction solution (~1 mL) was acidified with aq. 2.0 N HCl to reach the pH 2-3; then the mixture was extracted with ethyl acetate and organic layer was analyzed by TLC], the reaction mass was cooled to 0-5 °C and the pH was adjusted to 2-3 by the addition of aq. 2.0 N HCl (~850 mL, 5.0 V) at below 10 °C [Note: Precipitation occurred upon addition of HCl and the solids increased gradually with stirring]. The reaction mixture was warmed to room temperature and then stirred for 1.5-2.0 h. Solids obtained were isolated via filtration and were then washed with water (340 mL, 2.0 V); followed by washing with hexanes (340 mL, 2.0 V). Bulk residual water was removed from the solids by maintaining vacuum filtration for 60-90 min. The wet material was dried in a hot air oven at 50 °C for 6-7 h (until the moisture content was below 1.0%) to afford the dried product, *N*-(4-chloro-1-(2,2-difluoroethyl)-7-nitro-1*H*-indazol-3-yl)methanesulfonamide (170.0 g, 75%) as a yellow solid. ¹H NMR (400MHz, CDCl₃): *δ* 8.15 *(d, J=* 8.3 Hz, 1H), 7.52 (bs, 1H), 7.24 *(d, J=* 8.3 Hz, 1H), 6.04 (tt, *J₁* = 3.7 Hz, *J₂* = 7.9 Hz, 1H), 5.02 (td, *J₁* = 3.9 Hz, *J₂ =* 14.3 Hz, 2H), 3.42 (s, 4H).

### Step 3: Preparation of N-(4-chloro-1-(2,2-difluoroethyl)-7-nitro-1H-indazol-3-yl)-N-(4-methoxy benzyl)methanesulfonamide

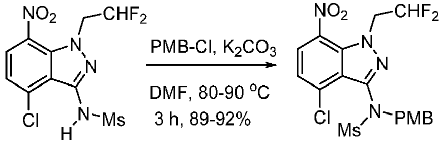

To a mixture of *N*-(4-chloro-1-(2,2-difluoroethyl)-7-nitro-1*H*-indazol-3-yl)methane sulfonamide (160.0 g, 0.45 mol, 1.0 equiv.) and 1-(chloromethyl)-4-methoxybenzene (67.6 mL, 0.5 mol, 1.1 equiv.) in DMF (1.6 L, 10.0 V) at room temperature was added potassium carbonate (93.8 g, 0.59 mol, 1.3 equiv.). The reaction mixture was heated to 80-90 °C and maintained at the same temperature for 3 h. After completion of the reaction (monitored by TLC), the mixture was poured into ice cold water (4.8 L, 60.0 V) [Note: Slow quenching with vigorous stirring is preferred to avoid clumping as the product precipitates]. The resulting solids were isolated via filtration and washed with water (480 mL, 3.0 V); then the solids were washed with hexanes (320 mL, 2.0 V). Bulk residual water was removed from the solids by maintaining vacuum filtration for 1-2 h. The isolated solid was dissolved in ethyl acetate (1.6 L, 10.0 V) and charcoal was added (16.0 g). The mixture was heated to 60-70 °C and then stirred for 30-45 min. at that temperature. The mixture was filtered while hot (40-50 °C) through a pad of Celite and the Celite pad was then extracted with ethyl acetate (800 mL, 5.0 V). The combined filtrates were concentrated to dryness under reduced pressure at below 50 °C. To the resulting solids at room temperature was added ethyl acetate (160 mL, 1.0 V). The suspension was stirred for 30 min. The solids were isolated via filtration and then were washed with hexanes (320 mL, 2.0 V). Residual water was removed from the solids by maintaining vacuum filtration for 45-60 min. to afford the product *N*-(4-chloro-1-(2,2-difluoroethyl)-7-nitro-1*H*-indazol-3-yl)-*N*-(4-methoxybenzyl)methanesulfonamide (180.0 g, 92% yield) as a yellow solid. ¹H NMR (400MHz, CDCl₃): *δ* 8.06 (d, *J=* 8.4 Hz, 1H), 7.52 (bs, 1H), 7.27 - 7.21 (m, 4H), 6.77 (d, *J=* 8.3 Hz, 2H), 6.01 (tt, *J₁* = 3.8 Hz, *J₂ =* 7.9 Hz, 1H), 5.12 - 4.78 (m, 4H), 3.74 (s, 3H), 3.02 (s, 3H).

### Step 4: Preparation of N-(7-amino-4-chloro-1-(2,2-difluoroethyl)-1H-indazol-3-yl)-N-(4-methoxybenzyl)methanesulfonamide

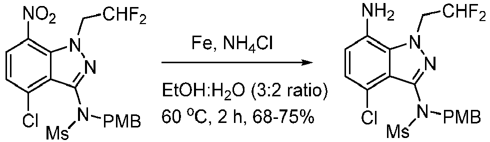

To a stirred suspension of iron powder (76.5 g, 1.37 mol, 5.0 equiv.) in a mixture of EtOH (650 mL, 5.0 V) and water (780 mL, 6.0 V) at room temperature was added ammonium chloride (118.0 g, 2.18 mol, 8.0 equiv.). To the mixture was added *N*-(4-chloro-1-(2,2-difluoroethyl)-7-nitro-1*H*-indazol-3-yl)-*N*-(4-methoxybenzyl)methanesulfonamide (130 g, 0.27 mol, 1.0 equiv.) in EtOH (520 mL, 4.0 V). The reaction mixture was heated to 60 °C and then stirred for 2 h. After completion of the reaction (monitored by in-process TLC/HPLC), the mixture was cooled to room temperature and diluted with ethyl acetate (1.3 L, 10.0 V) and water (390 mL, 3.0 V). The mixture was stirred for 15 min. The mixture was filtered through a pad of Celite and the Celite pad was then extracted with ethyl acetate (650 mL, 5.0 V). The bi-phasic filtrate was partitioned, and the organic phase was reserved while the aqueous layer was extracted with ethyl acetate (650 mL, 5.0 V). The combined organic layers were washed with brine (1.3 L, 10 V), dried over Na₂SO₄, and then concentrated *in vacuo* to afford a crude solid. To the crude product was added MTBE (650 mL, 5.0 V) and the suspension was stirred for 30 min. at room temperature. The solids were isolated via filtration. Bulk residual water was removed from the solids by maintaining vacuum filtration for 30-45 min. The wet product was dried in a hot air oven (50 °C) for 2 h to afford the title compound *N*-(7-amino-4-chloro-1-(2,2-difluoroethyl)-1*H*-indazol-3-yl)-*N-*(4-methoxy benzyl)methanesulfonamide (100.0 g, 70% yield) as off-white solid. ¹H NMR (400MHz, CDCl₃): *δ* 7.21 (d, *J=* 8.5 Hz, 2H), 6.87 (d, *J=* 8.4 Hz, 1H), 6.78 (d, *J=* 8.5 Hz, 2H), 6.52 (d, *J* = 8.3 Hz, 1H), 6.01 (tt, *J₁* = 3.8 Hz, *J₂* = 7.7 Hz, 1H), 4.98-4.69 (m, 4H), 3.75 (s, 3H), 2.98 (s, 3H).

### Alternate preparation of N-(7-amino-4-chloro-1-(2,2-difluoroethyl)-1H-indazol-3-yl)-N-(4-methoxybenzyl)cyclopropanesulfonamide

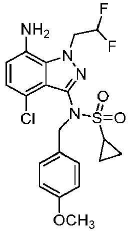

### Synthesis Scheme:

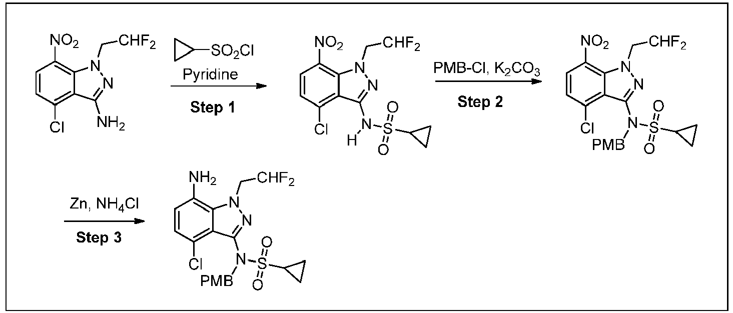

### Step 1: Preparation of N-(4-chloro-1-(2,2-difluoroethyl)-7-nitro-1H-indazol-3-yl)cyclopropanesulfonamide

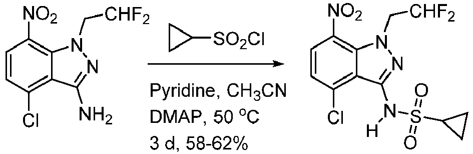

To a stirred solution of 4-chloro-1-(2,2-difluoroethyl)-7-nitro-1*H*-indazol-3-amine (150.0 g, 0.54 mol, 1.0 equiv.) in acetonitrile (600 mL, 4.0 V) at room temperature was added pyridine (600 mL, 4.0 V), followed by the addition of 4-dimethylaminopyridine (30.0 g, 0.27 mol, 0.5 equiv.). The reaction mass was stirred for 5-10 min., then cyclopropylsulfonyl chloride (114 mL, 1.08 mol, 2.0 equiv.) was added at room temperature. The reaction mixture was heated to 50 °C and then stirred at that temperature for 3 days. After completion of the reaction (monitored by TLC), the mixture was cooled to room temperature and diluted with water (1.5 L, 10.0 V) and ethyl acetate (1.5 L, 10.0 V), then stirred at room temperature for 15 min. The organic layer was separated, and the aqueous layer was extracted with EtOAc (300 mL, 2.0 V). The combined organic layers were washed with aq. 1.0 N HCl (600 mL, 4.0 V), followed by 10% brine solution (1.5 L, 10.0 V). The organic layer was dried over Na₂SO₄, filtered, and then concentrated under reduced pressure to afford *N*-(4-chloro-1-(2,2-difluoroethyl)-7-nitro-1*H*-indazol-3-yl)cyclopropanesulfonamide (124.0 g, 61%) as a viscous liquid. ¹H NMR (400MHz, CDCl₃): *δ* 8.11 *(d, J=* 8.5 Hz, 1H), 7.25 *(d, J=* 8.5 Hz, 1H), 6.04 (tt, *J₁* = 3.8 Hz, *J₂ =* 7.7 Hz, 1H), 5.05 (td, *J₁* = 3.8 Hz, *J₂ =* 14.4 Hz, 2H), 3.06 - 3.00 (m, 1H), 1.65 - 1.42 (m, 2H), 1.19 - 1.13 (m, 2H).

### Step 2: Preparation of N-(4-chloro-1-(2,2-difluoroethyl)-7-nitro-1H-indazol-3-yl)-N-(4-methoxybenzyl)cyclopropanesulfonamide

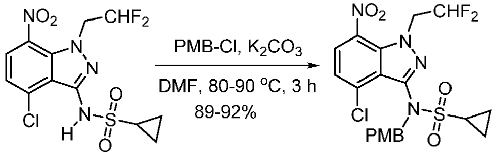

To a mixture of *N*-(4-chloro-1-(2,2-difluoroethyl)-7-nitro-1*H*-indazol-3-yl)cyclopropanesulfonamide (100.0 g, 0.20 mol, 1.0 equiv.) and 1-(chloromethyl)-4-methoxybenzene (39.2 mL, 0.22 mol, 1.1 equiv.) in DMF (1.0 L, 10.0 V) at room temperature was added potassium carbonate (128 g, 0.33 mol, 1.3 equiv.). The reaction mixture was heated to 80-90 °C and maintained at that temperature for 3 h. After completion of the reaction (monitored by TLC), the mixture was poured into ice cold water (3.0 L, 30.0 V) [Note: Slow quenching with vigorous stirring is preferred to avoid clumping as the product precipitates]. The resulting solids were isolated via filtration and washed with water (300 mL, 3.0 V); then the solids were washed with hexanes (300 mL, 3.0 V). Bulk residual water was removed from the solids by maintaining vacuum filtration for 1-2 h. The wet solid was dissolved in ethyl acetate (500 mL, 5.0 V) and charcoal was added (10.0 g). The mixture was heated to 60-70 °C and then stirred for 30-45 minutes at that temperature. The mixture was filtered while hot (40-50 °C) through a pad of Celite and the Celite pad was extracted with ethyl acetate (500 mL, 5.0 V). The combined filtrates were concentrated to dryness under reduced pressure at below 50 °C to afford *N*-(4-chloro-1-(2,2-difluoroethyl)-7-nitro-1*H*-indazol-3-yl)-*N-*(4-methoxy- benzyl)cyclopropanesulfonamide (122.0 g, 92% yield) as a yellow solid. ¹H NMR (400MHz, CDCl₃): *δ* 8.05 (d, *J=* 8.6 Hz, 1H), 7.26-7.22 (m, 3H), 6.73 (d, *J=* 8.5 Hz, 2H), 5.98 (tt, *J₁* = 3.7 Hz, *J₂ =* 7.8 Hz, 1H), 5.09-4.88 (m, 4H), 3.72 (s, 3H), 2.65-2.60 (m, 1H), 1.15-1.06 (m, 2H), 0.89 - 0.86 (m, 2H).

### Step 3: Preparation of N-(7-amino-4-chloro-1-(2,2-difluoroethyl)-1H-indazol-3-yl)-N-(4-methoxybenzyl)cyclopropanesulfonamide

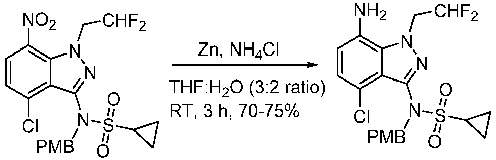

To a stirred suspension of zinc powder (156.0 g, 2.4 mol, 10.0 equiv.) in a mixture of THF (1.2 L, 10.0 V) and water (2.4 L, 20.0 V) at room temperature was added ammonium chloride (129.0 g, 2.40 mol, 10.0 equiv.). To the mixture was added *N*-(4-chloro-1-(2,2-difluoroethyl)-7-nitro-1*H*-indazol-3-yl)-*N-*(4-methoxybenzyl)cyclopropanesulfonamide (120 g, 0.2 mol, 1.0 equiv.) in THF (2.4 L, 20.0 V). The reaction mixture was stirred at room temperature for 2 h. After completion of the reaction (monitored by in-process TLC/HPLC), the mixture was diluted with ethyl acetate (1.2 L, 10.0 V) and water (360 mL, 3.0 V). The mixture was stirred for 15 min. The mixture was filtered through Celite and the Celite pad was extracted with ethyl acetate (600 mL, 5.0 V). The bi-phasic filtrate was partitioned, and the organic phase was reserved while the aqueous layer was extracted with ethyl acetate (600 mL, 5.0 V). The combined organic layers were washed with 10% brine solution (1.2 L, 10 V), dried over Na₂SO₄, filtered, and then concentrated *in vacuo* to afford a crude solid. To the crude product was added MTBE (600 mL, 5.0 V) and the suspension was stirred for 30-45 min. at room temperature. The solids were isolated by filtration and then bulk residual water was removed from the solids by maintaining vacuum filtration for 30-45 min. The wet product was dried in a hot air oven (50 °C) for 2 h to afford the product, *N-*(7-amino-4-chloro-1-(2,2-difluoroethyl)-1*H*-indazol-3-yl)-*N*-(4-methoxybenzyl)cyclopropanesulfonamide (81.0 g, 73% yield) as off-white solid. ¹H NMR (400MHz, CDCl₃): *δ* 7.25 (d, *J* = 8.5 Hz, 2H), 6.93 (d, *J* = 8.4 Hz, 1H), 6.75 (d, *J* = 8.3 Hz, 2H), 6.57 (d, *J* = 8.4 Hz, 1H), 6.03 (tt, *J₁* = 3.7 Hz, *J₂ =* 7.9 Hz, 1H), 4.80-4.95 (m, 4H), 3.74 (s, 3H), 2.67-2.61 (m, 1H), 1.14 (d, *J=* 2.4 Hz, 2H), 0.96 (d, *J* = 2.3 Hz, 2H).

### Alternate preparation of N-(7-amino-4-chloro-1-(2,2,2-trifluoroethyl)-1H-indazol-3-yl)-N-(4-methoxybenzyl)methanesulfonamide

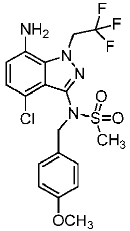

### Synthesis Scheme:

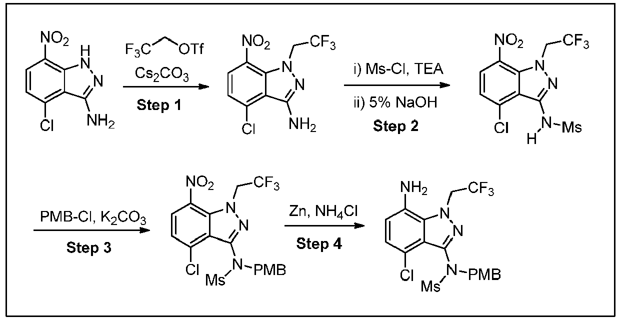

### Step 1: Preparation of 4-chloro-7-nitro-1-(2,2,2-trifluoroethyl)-1H-indazol-3-amine

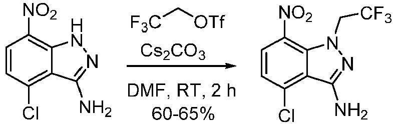

To a stirred solution of 4-chloro-7-nitro-1*H*-indazol-3-amine (50 g, 0.23 mol, 1.0 equiv.) in DMF (500 mL, 10.0 V) at 10-15 °C was added cesium carbonate (Cs₂CO₃) (153.3 g, 0.47 mol, 2.0 equiv.) at a rate sufficient to maintain the reaction mass below 20 °C. The mixture was stirred for 5-10 min, then to the stirred mixture at 10-15 °C was added 2,2,2-trifluoroethyl trifluoromethanesulfonate (60.18 g, 0.26 mol, 1.1 equiv.) at a rate sufficient to maintain the reaction mass below 20 °C (Note: slow addition is preferred for obtaining more favorable regio-selectivity). The reaction mass was allowed to slowly warm to room temperature and was then stirred at the same temperature for 2 h. After completion of the reaction (monitored by TLC), the reaction mass was quenched via the addition of ice-cold water (1.5 L, 30.0 V) and the resulting mixture was allowed to warm to room temperature with stirring for 6-8 h. The solids were isolated via filtration and were then washed with water (150 mL, 3.0 V). The wet solid was washed with hexanes (250 mL, 5.0 V) and then bulk residual water was removed from the solids by maintaining vacuum filtration for 60-90 min. The wet solid was dried in a hot air oven for 7-8 h at 50 °C (until the moisture content was below 1.0%). The isolated material, 4-chloro-7-nitro-1-(2,2,2-trifluoroethyl)-1*H*-indazol-3-amine (45.0 g, 60% yield), was used directly in the next step without further purification. ¹H-NMR (400 MHz, CDCl₃): *δ* 8.09 (d, *J=* 8.40 Hz, 1H), 7.12 (d, *J=* 8.40 Hz, 1H), 5.14 (q, *J=* 8.52 Hz, 2H), 4.77 (bs, H).

### Step 2: Preparation of N-(4-chloro-7-nitro-1-(2,2,2-trifluoroethyl)-1H-indazol-3-yl)methanesulfonamide

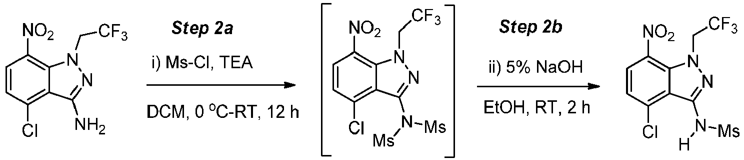

(Step 2a): To a solution of 4-chloro-7-nitro-1-(2,2,2-trifluoroethyl)-1*H*-indazol-3-amine (20.0 g, 0.068 mol, 1.0 equiv.) in DCM (200 mL, 10.0 V) at 0-5 °C. was added triethylamine (29.0 mL, 0.204 mol, 3.0 equiv.), followed by the addition of 4-dimethylaminopyridine (415 mg, 0.03 mol, 0.05 equiv.). The reaction mass was stirred for 5-10 min., then to the mixture was added methanesulfonyl chloride (13.25 mL, 0.17 mol, 2.5 equiv) at a rate sufficient to maintain the reaction mass below 10 °C. The reaction mixture was allowed to warm to room temperature with stirring for 12 h. After completion of the reaction (monitored by TLC), the mixture was diluted with water (200 mL, 10.0 V) and then stirred at room temperature for 15 min. The organic layer was separated, and the aqueous layer was extracted with DCM (200 mL, 10.0 V). The combined organic layers were washed with 10% brine solution (60 mL, 3.0 V), dried over Na₂SO₄, filtered, and concentrated to afford the crude solids. The solids were triturated with hexanes (60 mL, 3.0 V) at room temperature to obtain the intermediate, *N*-(4-chloro-7-nitro-1-(2,2,2-trifluoroethyl)-1*H*-indazol-3-yl)-*N-*(methylsulfonyl)methanesulfonamide, which was used directly in the next step. (Step 2b): To a stirred solution of *N*-(4-chloro-7-nitro-1-(2,2,2-trifluoroethyl)-1*H-*indazol-3-yl)-*N*-(methylsulfonyl)methanesulfonamide (entirety of the material prepared above) in ethanol (200 mL, 10.0 V) at room temperature was added slowly aq. 5% NaOH solution (140 mL, 7.0 V) [Note: Slow addition is preferred via dropping funnel]. The reaction mass was stirred at the same temperature for 2 h. After completion of the reaction [Sample preparation for TLC analysis: An aliquot of the reaction solution (~1.0 ml) was acidified by the addition of aq. 2.0 N HCl to reach pH 2-3; then the mixture was extracted with ethyl acetate and the organic phase was analyzed by TLC], the reaction mass was cooled to 0-5 °C and the pH was adjusted to 2-3 by the addition of aq. 2.0 N HCl (100 mL, 5.0 V) while maintain the temperature below 10 °C [Note: Precipitation occurred upon addition of HCl and increased with stirring]. The reaction mixture was warmed to room temperature and then stirred for 1.5-2.0 h. The solids were isolated via filtration and were then washed with water (60 mL, 3.0 V), followed by washing with hexanes (60 mL, 3.0 V). Bulk residual water was removed from the solids by maintaining vacuum filtration for 60-90 min. The wet material was dried in a hot air oven at 50 °C for 6-7 h (until the moisture content was below 1.0%) to afford *N*-(4-chloro-7-nitro-1-(2,2,2-trifluoroethyl)-1*H*-indazol-3-yl)methanesulfonamide (22.1 g, 87%) as a yellow solid. ¹H NMR (400 MHz, CDCl₃): *δ* 8.19 (d, *J=* 8.40 Hz, 1H), 7.56 (bs, 1H), 7.30 (d, *J=* 8.40 Hz, 1H), 5.34 (q, *J=* 8.30 Hz, 2H), 3.46 (s, 3H).

### Step 3: Preparation of N-(4-chloro-7-nitro-1-(2,2,2-trifluoroethyl)-1H-indazol-3-yl)-N-(4-methoxybenzyl)methanesulfonamide

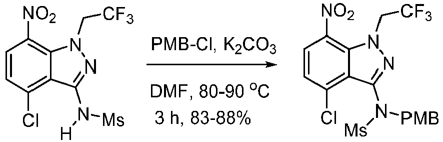

To a mixture of *N*-(4-chloro-7-nitro-1-(2,2,2-trifluoroethyl)-1*H*-indazol-3-yl)methanesulfonamide (50.0 g, 0.134 mol, 1.0 equiv.) and 1-(chloromethyl)-4-methoxybenzene (23.0 g, 0.147 mol, 1.1 equiv.) in DMF (500 mL, 10.0 V) at room temperature was added potassium carbonate (27.8 g, 0.201 mol, 1.5 equiv.). The reaction mixture was heated to 80-90 °C and maintained at that temperature for 3 h. After completion of the reaction (monitored by TLC), the mixture was poured into ice cold water (2.0 L, 40.0 V) [Note: Slow quenching with vigorous stirring is preferred to avoid clumping as the product precipitates]. The resulting solids were isolated via filtration and washed with water (150 mL, 3.0 V); then the solids were washed with hexanes (150 mL, 3.0 V). Bulk residual water was removed from the solids by maintaining vacuum filtration for 1-2 h. The solids were dissolved in ethyl acetate (500 mL, 10.0 V) and to the solution was added charcoal (5.0 g). The mixture was heated to 60-70 °C and then stirred at that temperature for 30-45 min. The mixture was filtered while hot (40-50 °C) through a pad of Celite and the Celite pad was extracted with ethyl acetate (250 mL, 5.0 V). The combined filtrate was concentrated to dryness under reduced pressure at below 50 °C. The solids were combined with ethyl acetate (50 mL, 1.0 V) at room temperature. The resulting suspension was stirred for 30 min. The solids were isolated via filtration and then were washed with hexanes (100 mL, 2.0 V). Residual water was removed from the solids by maintaining vacuum filtration for 45-60 min. to afford *N*-(4-chloro-7-nitro-1-(2,2,2-trifluoroethyl)-1*H*-indazol-3-yl)-*N-*(4-methoxybenzyl)methanesulfonamide (56.0 g, 85% yield) as a yellow solid. ¹H NMR (400 MHz, CDCl₃): *δ* 8.12 (d, *J=* 8.36 Hz, 1H), 7.31 (d, *J=* 8.36 Hz, 1H), 7.22 (d, *J=* 8.44 Hz, 2H), 6.77 (d, *J=* 8.44 Hz, 2H), 5.50-5.25 (m, 2H), 4.94-4.79 (m, 2H), 3.75 (s, 3H), 3.02 (s, 3H).

### Step 4: Preparation of N-(7-amino-4-chloro-1-(2,2,2-trifluoroethyl)-1H-indazol-3-yl)-N-(4-methoxybenzyl)methanesulfonamide

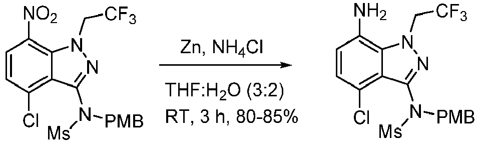

To a stirred suspension of zinc powder (66.31 g, 1.01 mol, 10.0 equiv.) in THF (500 mL, 10.0 V) and water (1.0 L, 20.0 V) at room temperature was added ammonium chloride (54.78 g, 1.01 mol, 10.0 equiv.). To the mixture was added a solution of N-(4-chloro-7-nitro-1-(2,2,2-trifluoroethyl)-1*H*-indazol-3-yl)-*N*-(4-methoxybenzyl)methanesulfonamide (50.0 g, 0.101 mol, 1.0 equiv.) in THF (1.0 L, 20.0 V). The reaction mixture was stirred at room temperature for 3 h. After completion of the reaction (monitored by in-process TLC/HPLC), the mixture was diluted with ethyl acetate (1.0 L, 20.0 V) and water (250 mL, 5.0 V). The mixture was stirred for 15 min. The mixture was filtered through a pad of Celite and the Celite pad was extracted with ethyl acetate (250 mL, 5.0 V). The bi-phasic filtrate was partition and the organic layer was reserved while the aqueous layer was extracted with ethyl acetate (500 mL, 10.0 V). The combined organic layers were washed with 10% brine solution (500 mL, 10.0 V), dried over Na₂SO₄, filtered, and then concentrated *in vacuo* to afford a crude solid. To the crude product was added MTBE (250 mL, 5.0 V) and the resulting suspension was stirred for 30 min. at room temperature. The solids were isolated by filtration and then bulk residual water was removed from the solids by maintaining vacuum filtration for 30-45 min. The wet product was dried in a hot air oven (50 °C) for 2 h to afford the title product *N-*(7-amino-4-chloro-1-(2,2,2-trifluoroethyl)-1*H*-indazol-3-yl)-*N-*(4-methoxybenzyl)methanesulfonamide (39.0 g, 83% yield) as off-white solid. ¹H NMR (400 MHz, CDCl₃): *δ* 7.25 (d, *J=* 8.48 Hz, 2H), 6.98 (d, *J=* 7.80 Hz, 1H), 6.79 (d, *J=* 8.48 Hz, 2H), 6.66 (d, *J=* 7.84 Hz, 1H), 5.35-4.75 (m, 4H), 3.77 (s, 3H), 3.56 (bs, 2H), 2.98 (s, 3H).

### IUPAC Chemical Names:

The IUPAC chemical names for each example are listed below. At this time these names are not recognized by common software such tools such as ChemDraw or JChem. Therefore, the chemical names used throughout the Examples section above were generated with ChemDraw with P/M nomenclature manually inserted. The chemical names can be converted to chemical structures using ChemDraw after the P/M nomenclature-e.g., "(3P)-"-is removed.

| Example | IUPAC Name |
|---|---|
| Example 1 | N-[(1S)-1-[(3P)-3-(4-chloro-3-methanesulfonamido-1-methyl-1H-indazol-7-yl)-4-oxo-7-[(pyridin-2-yl)methoxy]-3,4-dihydroquinazolin-2-yl]-2-(3,5-difluorophenyl)ethyl]-2-[(2S,4R)-9-(difluoromethyl)-5,5-difluoro-7,8-diazatricyclo[4.3.0.0²,⁴]nona-1(6),8-dien-7-yl]acetamide |
| Example 2 | N-[(1S)-1-[(3P)-3-(4-chloro-3-methanesulfonamido-1-methyl-1H-indazol-7-yl)-7-(cyclopentylmethoxy)-4-oxo-3,4-dihydroquinazolin-2-yl]-2-(3,5-difluorophenyl)ethyl]-2-[(2S,4R)-9-(difluoromethyl)-5,5-difluoro-7,8-diazatricyclo[4.3.0.0²,⁴]nona-1(6),8-dien-7-yl]acetamide |
| Example 3 | N-[(1S)-1-[(3P)-3-(4-chloro-3-methanesulfonamido-1-methyl-1H-indazol-7-yl)-7-(cyclohexylmethoxy)-4-oxo-3,4-dihydroquinazolin-2-yl]-2-(3,5-difluorophenyl)ethyl]-2-[(2S,4R)-9-(difluoromethyl)-5,5-difluoro-7,8-diazatricyclo[4.3.0.0²,⁴]nona-1(6),8-dien-7-yl]acetamide |
| Example 4 | N-[(1S)-1-[(3P,3P)-3-(4-chloro-3-methanesulfonamido-1-methyl-1H-indazol-7-yl)-7-[2-(1,1-dioxo-1λ⁶-thiomorpholin-4-yl)ethoxy]-4-oxo-3,4-dihydroquinazolin-2-yl]-2-(3,5-difluorophenyl)ethyl]-2-[(2S,4R)-9-(difluoromethyl)-5,5-difluoro-7,8-diazatricyclo[4.3.0.0²,⁴]nona-1(6),8-dien-7-yl]acetamide |
| Example 5 | N-[(1S)-1-[(3P,3P)-3-(4-chloro-3-methanesulfonamido-1-methyl-1H-indazol-7-yl)-4-oxo-7-(2-phenoxyethoxy)-3,4-dihydroquinazolin-2-yl]-2-(3,5-difluorophenyl)ethyl]-2-[(2S,4R)-9-(difluoromethyl)-5,5-difluoro-7,8-diazatricyclo[4.3.0.0²,⁴]nona-1(6),8-dien-7-yl]acetamide |
| Example 6 | N-[(1S)-1-[(3P)-3-(4-chloro-3-methanesulfonamido-1-methyl-1H-indazol-7-yl)-7-(3,3-dimethylbutoxy)-4-oxo-3,4-dihydroquinazolin-2-yl]-2-(3,5-difluorophenyl)ethyl]-2-[(2S,4R)-9-(difluoromethyl)-5,5-difluoro-7,8-diazatricyclo[4.3.0.0²,⁴]nona-1(6),8-dien-7-yl]acetamide |
| Example 7 | N-[(1S)-1-[(3P,3P)-3-(4-chloro-3-methanesulfonamido-1-methyl-1H-indazol-7-yl)-7-[2-(1-methyl-1H-pyrazol-5-yl)ethoxy]-4-oxo-3,4-dihydroquinazolin-2-yl]-2-(3,5-difluorophenyl)ethyl]-2-[(2S,4R)-9-(difluoromethyl)-5,5-difluoro-7,8-diazatricyclo[4.3.0.0²,⁴]nona-1(6),8-dien-7-yl]acetamide |
| Example 8 | N-[(1S)-1-[(3P)-3-(4-chloro-3-methanesulfonamido-1-methyl-1H-indazol-7-yl)-7-cyclobutoxy-4-oxo-3,4-dihydroquinazolin-2-yl]-2-(3,5-difluorophenyl)ethyl]-2-[(2S,4R)-9-(difluoromethyl)-5,5-difluoro-7,8-diazatricyclo[4.3.0.0²,⁴]nona-1(6),8-dien-7-yl]acetamide |
| Example 9 | N-[(1S)-1-[(3P)-3-(4-chloro-3-methanesulfonamido-1-methyl-1H-indazol-7-yl)-7-(cyclopentyloxy)-4-oxo-3,4-dihydroquinazolin-2-yl]-2-(3,5-difluorophenyl)ethyl]-2-[(2S,4R)-9-(difluoromethyl)-5,5-difluoro-7,8-diazatricyclo[4.3.0.0²,⁴]nona-1(6),8-dien-7-yl]acetamide |
| Example 10 | N-[(1S)-1-[(3P)-3-(4-chloro-3-methanesulfonamido-1-methyl-1H-indazol-7-yl)-7-(oxan-4-yloxy)-4-oxo-3,4-dihydroquinazolin-2-yl]-2-(3,5-difluorophenyl)ethyl]-2-[(2S,4R)-9-(difluoromethyl)-5,5-difluoro-7,8-diazatricyclo[4.3.0.0²,⁴]nona-1(6),8-dien-7-yl]acetamide |
| Example 11 | N-[(1S)-1-[(3P,3P)-3-(4-chloro-3-methanesulfonamido-1-methyl-1H-indazol-7-yl)-4-oxo-7-[(pyrimidin-5-yl)methoxy]-3,4-dihydroquinazolin-2-yl]-2-(3,5-difluorophenyl)ethyl]-2-[(2S,4R)-9-(difluoromethyl)-5,5-difluoro-7,8-diazatricyclo[4.3.0.0²,⁴]nona-1(6),8-dien-7-yl]acetamide |
| Example 12 | N-[(1S)-1-[(3P)-3-(4-chloro-3-methanesulfonamido-1-methyl-1H-indazol-7-yl)-7-(cyclobutylmethoxy)-4-oxo-3,4-dihydroquinazolin-2-yl]-2-(3,5-difluorophenyl)ethyl]-2-[(2S,4R)-9-(difluoromethyl)-5,5-difluoro-7,8-diazatricyclo[4.3.0.0²,⁴]nona-1(6),8-dien-7-yl]acetamide |
| Example 13 | N-[(1S)-1-[(3P,3P)-3-(4-chloro-3-methanesulfonamido-1-methyl-1H-indazol-7-yl)-4-oxo-7-[2-(1H-pyrazol-1-yl)ethoxy]-3,4-dihydroquinazolin-2-yl]-2-(3,5-difluorophenyl)ethyl]-2-[(2S,4R)-9-(difluoromethyl)-5,5-difluoro-7,8-diazatricyclo[4.3.0.0²,⁴]nona-1(6),8-dien-7-yl]acetamide |
| Example 14 | N-[(1S)-1-[(3P)-3-(4-chloro-3-methanesulfonamido-1-methyl-1H-indazol-7-yl)-4-oxo-7-(4,4,4-trifluoro-3,3-dimethylbutoxy)-3,4-dihydroquinazolin-2-yl]-2-(3,5-difluorophenyl)ethyl]-2-[(2S,4R)-9-(difluoromethyl)-5,5-difluoro-7,8-diazatricyclo[4.3.0.0²,⁴]nona-1(6),8-dien-7-yl]acetamide |
| Example 15 | N-[(1S)-1-[(3P)-3-(4-chloro-3-methanesulfonamido-1-methyl-1H-indazol-7-yl)-7-[2-(difluoromethoxy)ethoxy]-4-oxo-3,4-dihydroquinazolin-2-yl]-2-(3,5-difluorophenyl)ethyl]-2-[(2S,4R)-9-(difluoromethyl)-5,5-difluoro-7,8-diazatricyclo[4.3.0.0²,⁴]nona-1(6),8-dien-7-yl]acetamide |

### Biological Methods:

HIV cell culture assay - MT-2 cells, 293T cells and the proviral DNA clone of NL₄₋₃ virus were obtained from the NIH AIDS Research and Reference Reagent Program. MT-2 cells were propagated in RPMI 1640 media supplemented with 10% heat inactivated fetal bovine serum (FBS), 100 mg/ml penicillin G and up to 100 units/mL streptomycin. The 293T cells were propagated in DMEM media supplemented with 10% heat inactivated FBS, 100 mg/mL penicillin G and 100 mg/mL streptomycin. A recombinant NL₄₋₃ proviral clone, in which a section of the nef gene was replaced with the Renilla luciferase gene, was used to make the reference virus used in these studies. The recombinant virus was prepared through transfection of the recombinant NL₄₋₃ proviral clone into 293T cells using Transit-293 Transfection Reagent from Mirus Bio LLC (Madison, WI). Supernatent was harvested after 2-3 days and the amount of virus present was titered in MT-2 cells using luciferase enzyme activity as a marker by measuring luciferase enzyme activity. Luciferase was quantitated using the EnduRen Live Cell Substrate from Promega (Madison, WI). Antiviral activities of compounds toward the recombinant virus were quantified by measuring luciferase activity in MT-2 cells infected for 4-5 days with the recombinant virus in the presence of serial dilutions of the compound.

The 50% effective concentration (EC₅₀) was calculated by using the exponential form of the median effect equation where (Fa) = 1/[1+ (ED₅₀/drug conc.)m] (Johnson VA, Byington RT. Infectivity Assay. In Techniques in HIV Research. ed. Aldovini A, Walker BD. 71-76. New York: Stockton Press.1990). The 50% inhibitory concentration (EC₅₀) was calculated by using the exponential form of the median effect equation where percent inhibition = 1/[1 + (EC₅₀/drug concentration)m], where *m* is a parameter that reflects the slope of the concentration-response curve.

Compound cytotoxicity and the corresponding CC₅₀ values were determined using the same protocol as described in the antiviral assay except that uninfected cells were used. Cytotoxicity was assessed on day 4 in uninfected MT2 cells by using a XTT (2,3-bis[2-Methoxy-4-nitro-5-sulfophenyl]-2H-tetrazolium-5-carboxyanilide inner salt)-based colorimetric assay (Sigma-Aldrich, St Louis, Mo).

| Example | EC₅₀ nM | CC₅₀ µM |
|---|---|---|
| Example 2 | 0.18 | >1 |
| Example 3 | 0.47 | >1 |
| Example 4 | 0.080 | >1 |
| Example 5 | 0.36 | >1 |
| Example 6 | 0.18 | >1 |
| Example 7 | .074 | >1 |
| Example 9 | 0.22 | >1 |
| Example 10 | 0.036 | >1 |
| Example 11 | 0.066 | >1 |
| Example 12 | 0.15 | >1 |
| Example 13 | 0.032 | >1 |
| Example 14 | 0.23 | > 0.5 |
| Example 15 | 0.051 | > 0.5 |

The disclosure is not limited to the foregoing illustrative examples and the examples should be considered in all respects as illustrative and not restrictive, reference being made to the appended claims, rather than to the foregoing examples.

## Claims

1. A compound of Formula I, or a pharmaceutically acceptable salt thereof: 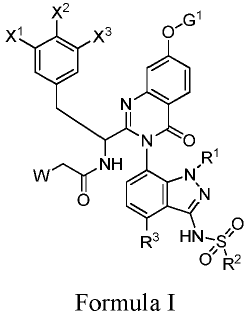 wherein:
G¹ is C₆-C₈alkyl optionally substituted with 1-3 fluorines; or G¹ is one of the following: 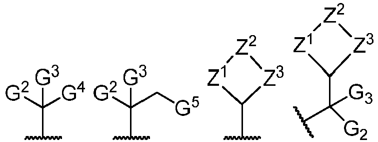
Z¹ is -C₁-C₂alkylene;
Z² is -O-, -S(O₂)-, or -CH₂-;
Z³ is -C₁-C₂alkylene;
G² and G³ are independently selected from H and -CH₃;
G⁴ is phenyl, pyridine, pyrimidine or pyrazine;
G⁵ is G⁴, -OG⁴, -O(C₁-C₂ alkyl optionally substituted with 1-3 fluorines), or G⁵ is one of the following: 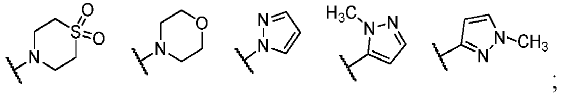
R¹ is hydrogen, C₁-C₃alkyl optionally substituted with 1-3 fluorines, or cyclopropyl optionally substituted with 1-2 fluorines;
R² is C₁-C₂alkyl optionally substituted with 1-3 fluorines, or C₃-C₄ cycloalkyl optionally substituted with 1-2 fluorines;
R³ is hydrogen, Cl, F, CH₃, or OCH₃;
W is selected from: 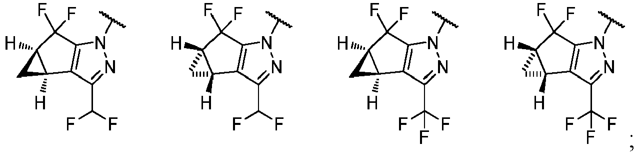
X¹, X² and X³ are independently selected from H, F, and Cl, or one of the group X¹, X² and
X³ is selected from -CN, -OCH₃, -CH₃, -CH₂F, -CHF₂, and -CF₃.

2. A compound or salt according to Claim 1 wherein W is the following: 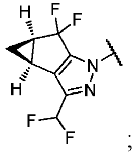 or

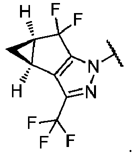

3. A compound or salt according to claim 1 or claim 2 wherein R¹ is -CH₃, -CH₂CHF₂, or -CH₂CF₃; R² is -CH₃ or cyclopropyl; and R³ is H, Cl or CH₃; suitably wherein R¹ is -CH₃; R² is -CH₃; and R³ is Cl.

4. A compound or salt according to any of Claims 1-3 wherein X¹, X², and X³ are independently selected from H or F; suitably wherein X¹ is F, X² is H, and X³ is F.

5. A compound or salt according to any of Claims 1-4 wherein G² and G³ are H.

6. A compound or salt according to any of Claims 1-5 wherein G₁ is the following: 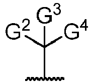 wherein G⁴ is pyridine or pyrimidine; or wherein G₁ is the following: 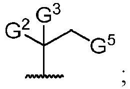 or wherein G₁ is the following: 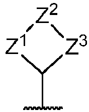 wherein Z² is -O- or -(CH₂)-; or wherein G₁ is the following: 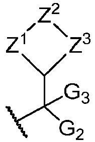 wherein Z² is -(CH₂)-.

7. A compound or salt according to any of Claims 1-4 wherein G¹ is C₆-C₈alkyl optionally substituted with 1-3 fluorines.

8. A compound or salt according to any of Claims 1-4 wherein G¹ is one of the following:

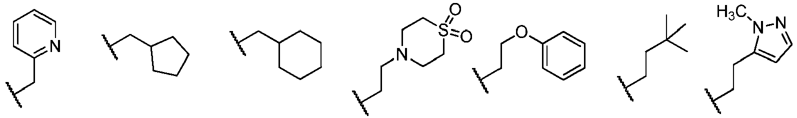

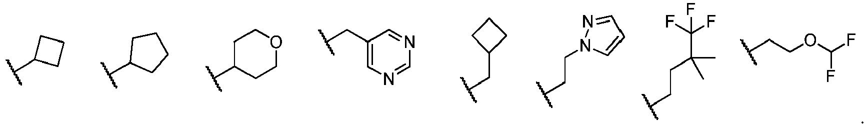

9. A compound or salt according to any of Claims 1-8 wherein the stereochemistry is as depicted below:

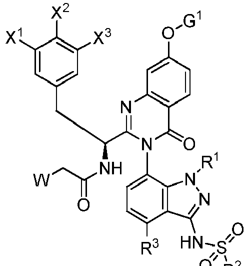

10. A compound or salt according to any of Claims 1-8 wherein the stereochemistry is as depicted below:

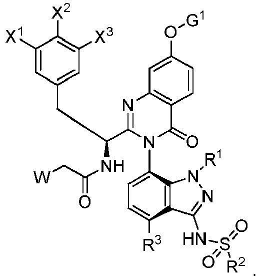

11. A compound or salt according to Claim 1, selected from the group consisting of: 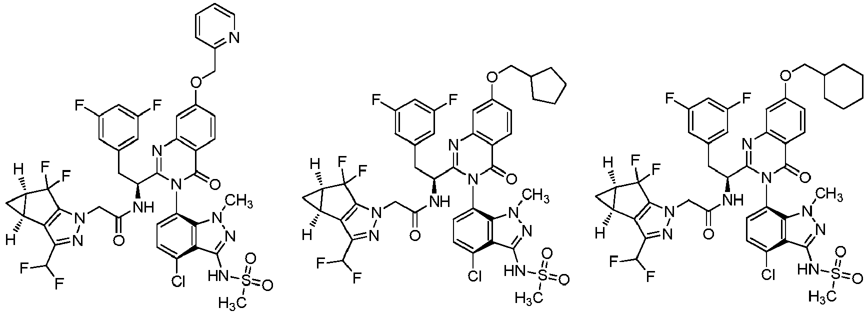 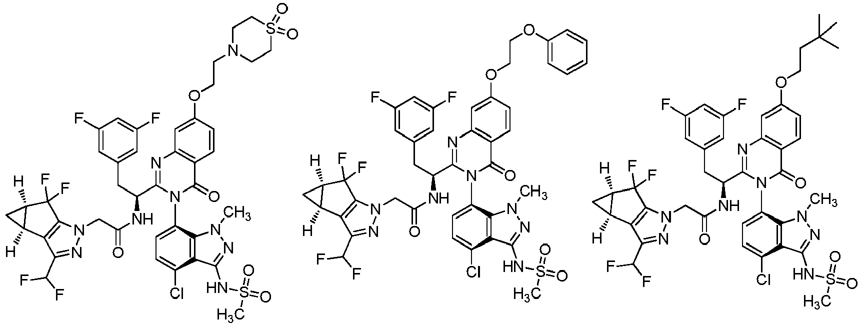 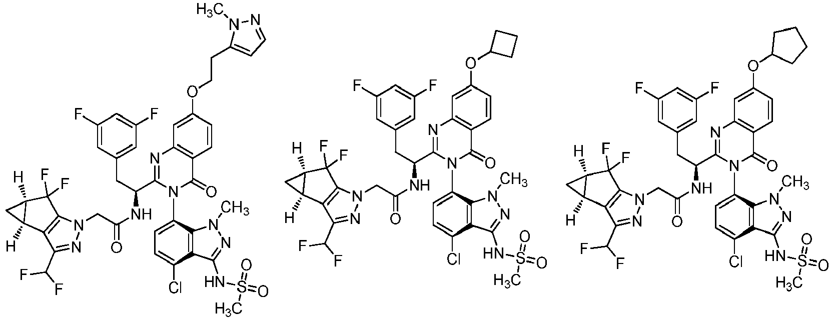 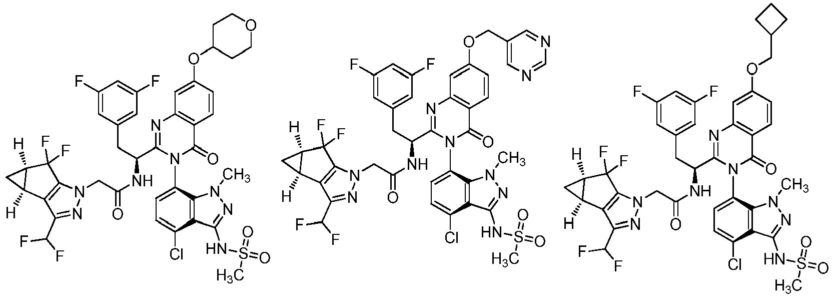 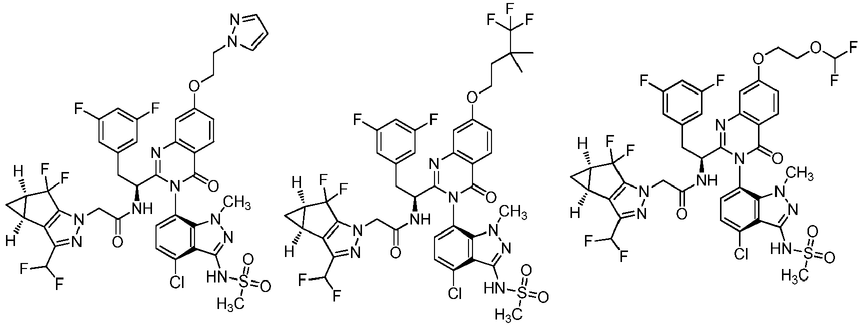 and pharmaceutically acceptable salts thereof.

12. A pharmaceutical composition comprising a compound or salt according to any of Claims 1-11.

13. A composition according to Claim 12 further comprising a pharmaceutically acceptable carrier, excipient, and/or diluent.

14. A compound or pharmaceutically acceptable salt thereof according to any of Claims 1-11 for use in therapy

15. A compound or pharmaceutically acceptable salt thereof according to any of Claims 1-11 for use in treating HIV infection.

## Patentansprüche

1. Verbindung der Formel I, oder ein pharmazeutisch akzeptables Salz davon: 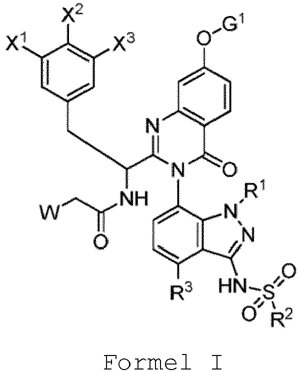 worin:
G¹ ist C₆-C₈-Alkyl, optional substituiert mit 1-3 Fluoratomen; oder G¹ ist eines der folgenden: 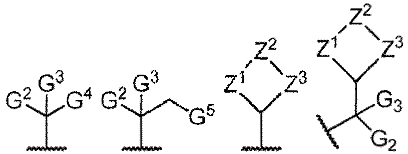
Z¹ ist -C₁-C₂-Alkylen;
Z² ist -O-, -S(O₂)-, oder -CH₂-;
Z³ ist -C₁-C₂-Alkylen;
G² und G³ sind jeweils unabhängig voneinander ausgewählt aus H und -CH₃;
G⁴ ist Phenyl, Pyridin, Pyrimidin oder Pyrazin;
G⁵ ist G⁴, -OG⁴, -O(C₁-C₂-Alkyl optional substituiert mit 1-3 Fluoratomen), oder G⁵ ist eines der folgenden: 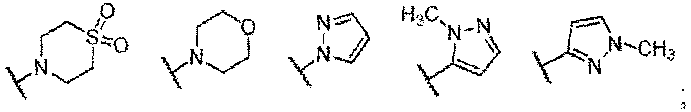
R¹ ist Wasserstoff, C₁-C₃-Alkyl optional substituiert mit 1-3 Fluoratomen, oder Cyclopropyl optional substituiert mit 1-2 Fluoratomen;
R² ist C₁-C₂-Alkyl optional substituiert mit 1-3 Fluoratomen, oder C₃-C₄-Cycloalkyl optional substituiert mit 1-2 Fluoratomen;
R³ ist Wasserstoff, Cl, F, CH₃, oder OCH₃;
W ist ausgewählt aus: 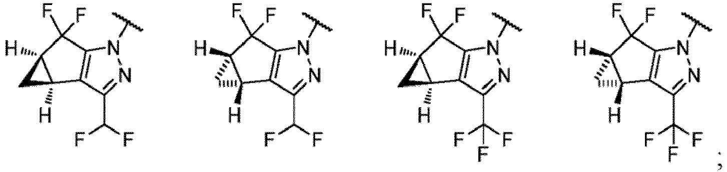
X¹, X² und X³ sind jeweils unabhängig ausgewählt aus H, F und Cl, oder eines aus der Gruppe X¹, X² und X³ ist ausgewählt aus -CN, -OCH₃, -CH₃, -CH₂F, -CHF₂ und -CF₃.

2. Verbindung oder Salz gemäß Anspruch 1, worin W ist: 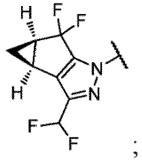 oder

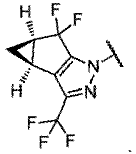

3. Verbindung oder Salz gemäß Anspruch 1 oder Anspruch 2, worin R¹ ist -CH₃, -CH₂CHF₂, oder -CH₂CF₃; R² ist -CH₃ oder Cyclopropyl; und R³ ist H, Cl oder CH₃; vorzugsweise worin R¹ ist -CH₃; R² ist -CH₃; und R³ ist Cl.

4. Verbindung oder Salz gemäß mindestens einem der Ansprüche 1-3, worin X¹, X² und X³ jeweils unabhängig voneinander ausgewählt sind aus H oder F; vorzugsweise worin X¹ F ist, X² H ist und X³ F ist.

5. Verbindung oder Salz gemäß mindestens einem der Ansprüche 1-4, worin G² und G³ H sind.

6. Verbindung oder Salz gemäß mindestens einem der Ansprüche 1-5, worin G¹ ist: 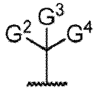 worin G⁴ ist Pyridin oder Pyrimidin; oder worin G¹ ist: 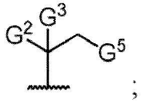 oder worin G¹ ist: 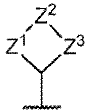 worin Z² ist -O- oder -(CH₂)-; oder worin G¹ ist: 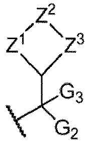 worin Z² ist -(CH₂)-.

7. Verbindung oder Salz gemäß mindestens einem der Ansprüche 1-4, worin G¹ ist C₆-C₈-Alkyl optional substituiert mit 1-3 Fluoratomen.

8. Verbindung oder Salz gemäß mindestens einem der Ansprüche 1-4, worin G¹ eines der folgenden ist:

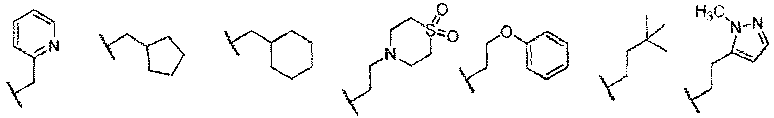

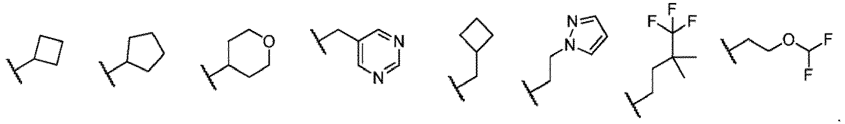

9. Verbindung oder Salz gemäß mindestens einem der Ansprüche 1-8, worin die Stereochemie wie unten dargestellt ist:

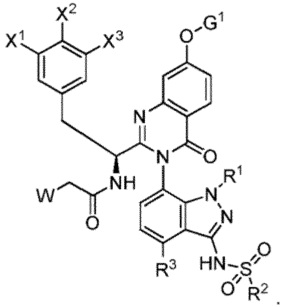

10. Verbindung oder Salz gemäß mindestens einem der Ansprüche 1-8, worin die Stereochemie wie unten dargestellt ist:

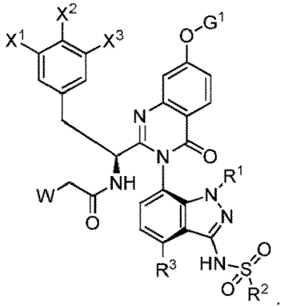

11. Verbindung oder Salz gemäß Anspruch 1, ausgewählt aus der Gruppe, bestehend aus: 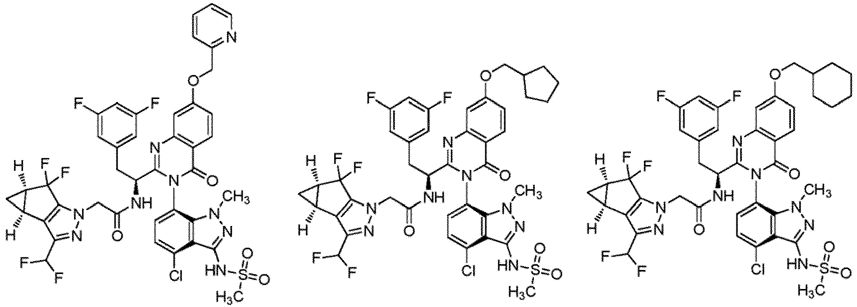 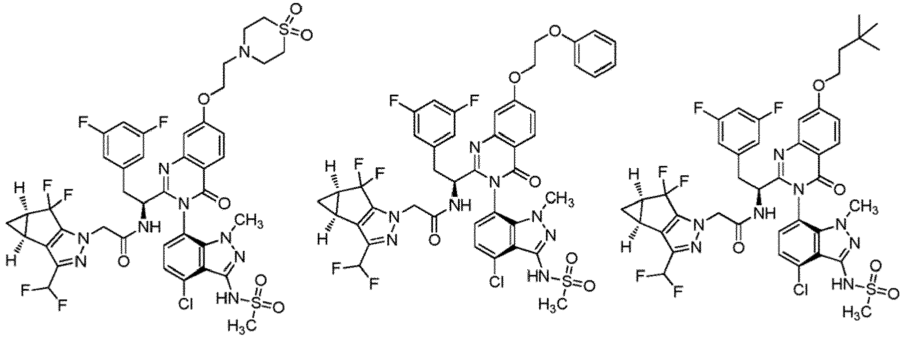 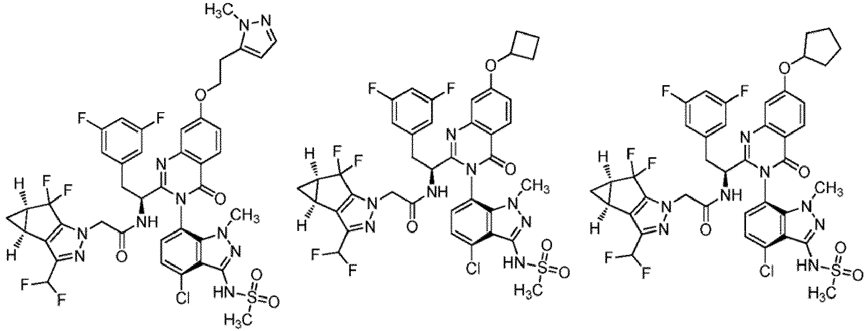 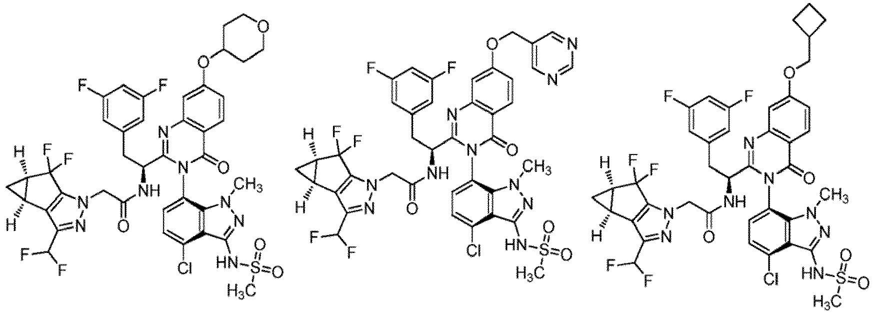 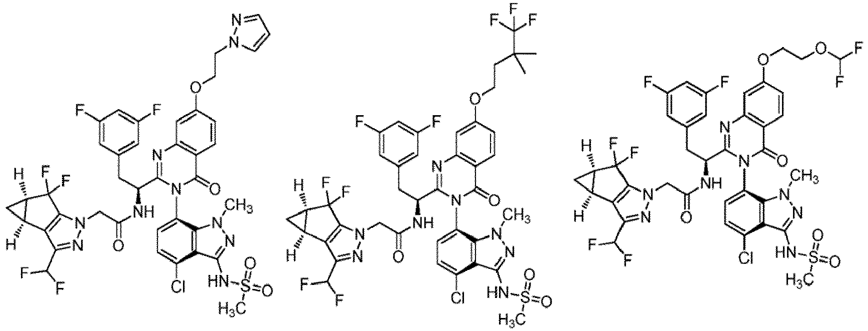 und pharmazeutisch akzeptable Salze davon.

12. Pharmazeutische Zusammensetzung umfassend eine Verbindung oder ein Salz gemäß mindestens einem der Ansprüche 1-11.

13. Zusammensetzung gemäß Anspruch 12, ferner umfassend einen pharmazeutisch akzeptablen Träger, Hilfsstoff, und/oder Verdünnungsmittel.

14. Verbindung oder pharmazeutisch akzeptables Salz davon gemäß mindestens einem der Ansprüche 1-11 zur Verwendung in der Therapie.

15. Verbindung oder pharmazeutisch akzeptables Salz davon gemäß mindestens einem der Ansprüche 1-11 zur Verwendung bei der Behandlung einer HIV-Infektion.

## Revendications

1. Composé de formule I, ou un sel pharmaceutiquement acceptable de celui-ci : 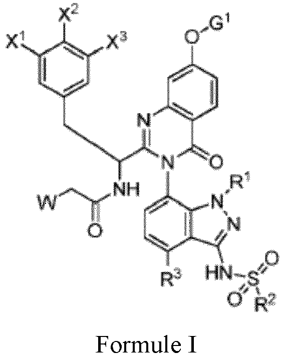 dans lequel :
G¹ est un alkyle en C₆-C₈ optionnellement substitué avec 1-3 fluors ; ou G¹ est l'un des suivants : 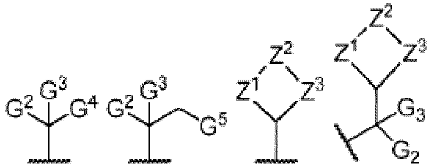
Z¹ est un alkylène en C₁-C₂ ;
Z² est -O-, -S(O₂)-, ou -CH₂- ;
Z³ est un alkylène en C₁-C₂ ;
G² et G³ sont sélectionnés indépendamment parmi H et -CH₃ ;
G⁴ est un phényle, une pyridine, une pyrimidine ou une pyrazine ;
G⁵ est G⁴, -OG⁴, un -O(alkyle en C₁-C₂ optionnellement substitué avec 1-3 fluors), ou
G⁵ est l'un des suivants : 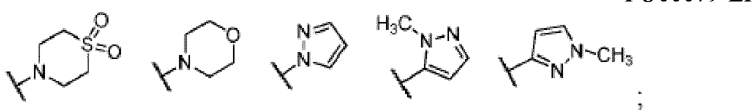
R¹ est un hydrogène, un alkyle en C₁-C₃ optionnellement substitué avec 1-3 fluors, ou un cyclopropyle optionnellement substitué avec 1-2 fluors ;
R² est un alkyle en C₁-C₂ optionnellement substitué avec 1-3 fluors, ou un cycloalkyle en C₃-C₄ optionnellement substitué avec 1-2 fluors ;
R³ est un hydrogène, Cl, F, CH₃ ou OCH₃ ;
W est sélectionné parmi : 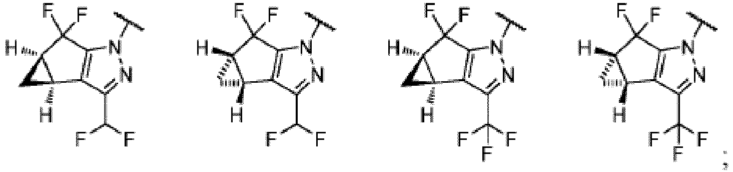
X¹, X² et X³ sont sélectionnés indépendamment parmi H, F, et Cl, ou l'un des groupes
X¹, X² et X³ est sélectionné parmi -CN, -OCH₃, -CH₃, -CH₂F, -CHF₂, et -CF₃.

2. Composé ou sel selon la revendication 1, dans lequel W est le suivant : 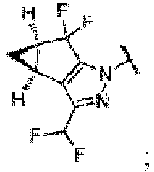 ou

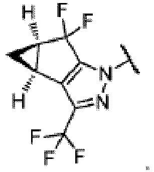

3. Composé ou sel selon la revendication 1 ou la revendication 2 dans lequel R¹ est -CH₃, -CH₂CHF₂, ou -CH₂CF₃ ; R² est -CH₃ ou un cyclopropyle ; et R³ est H, Cl ou CH₃ ; de manière appropriée dans lequel R¹ est -CH₃ ; R² est -CH₃ ; et R³ est Cl.

4. Composé ou sel selon l'une quelconque des revendications 1-3, dans lequel X¹, X², et X³ sont sélectionnés indépendamment parmi H ou F ; de manière appropriée dans lequel X¹ est F, X² est H, et X³ est F.

5. Composé ou sel selon l'une quelconque des revendications 1-4, dans lequel G² et G³ sont H.

6. Composé ou sel selon l'une quelconque des revendications 1-5, dans lequel G₁ est le suivant : 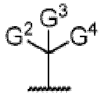 dans lequel G⁴ est une pyridine ou une pyrimidine ; ou dans lequel G₁ est le suivant : 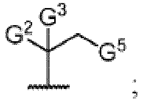 ou dans lequel G₁ est le suivant : 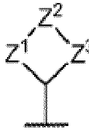 dans lequel Z² est -O- ou -(CH₂)- ; ou dans lequel G₁ est le suivant : 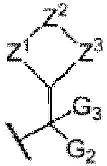 dans lequel Z² est -(CH₂)-.

7. Composé ou sel selon l'une quelconque des revendications 1-4, dans lequel G¹ est un alkyle en C₆-C₈ optionnellement substitué avec 1-3 fluors.

8. Composé ou sel selon l'une quelconque des revendications 1-4, dans lequel G¹ est l'un des suivants :

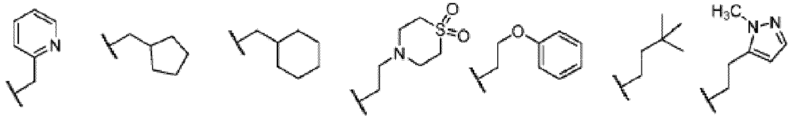

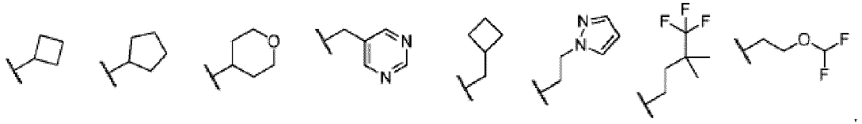

9. Composé ou sel selon l'une quelconque des revendications 1-8, dans lequel la stéréochimie est telle que représentée ci-dessous :

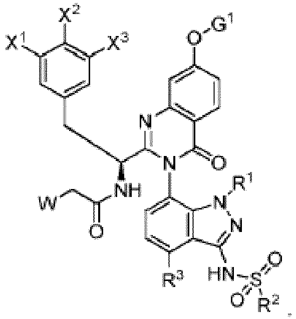

10. Composé ou sel selon l'une quelconque des revendications 1-8, dans lequel la stéréochimie est telle que représentée ci-dessous :

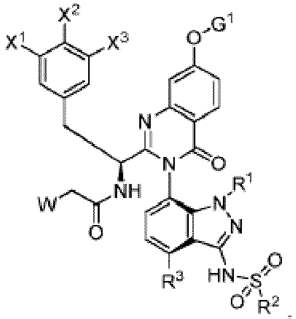

11. Composé ou sel selon la revendication 1, sélectionné dans le groupe constitué de : 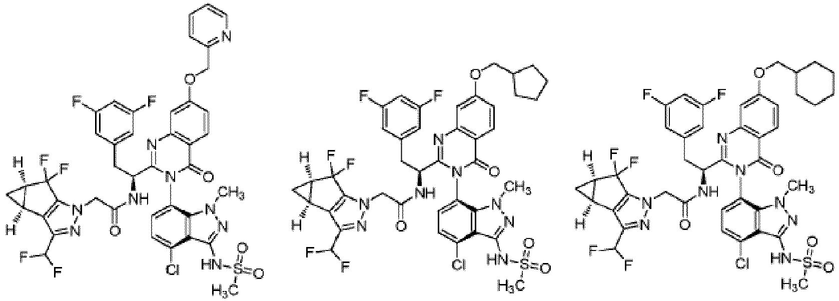 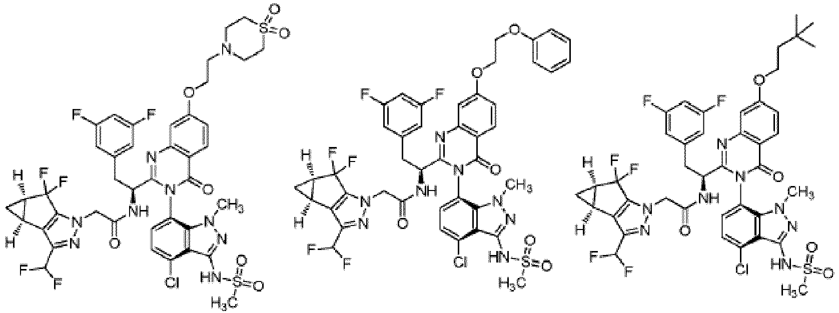 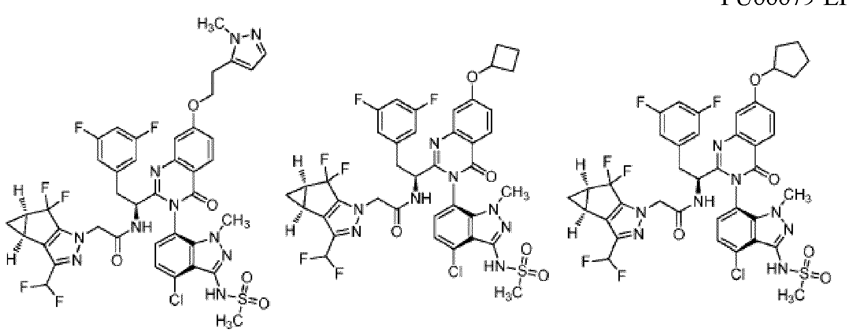 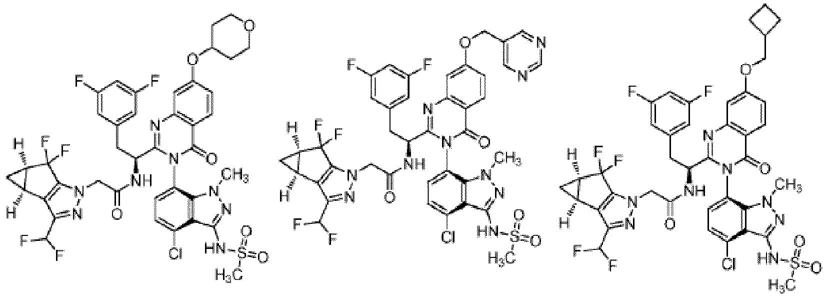 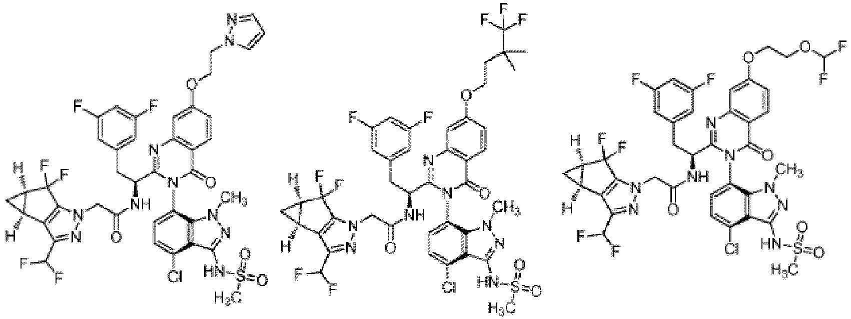 et les sels pharmaceutiquement acceptables de ceux-ci.

12. Composition pharmaceutique comprenant un composé ou un sel selon l'une quelconque des revendications 1-11.

13. Composition selon la revendication 12 comprenant en outre un support, excipient et/ou diluant pharmaceutiquement acceptable.

14. Composé ou sel pharmaceutiquement acceptable de celui-ci selon l'une quelconque des revendications 1-11 pour une utilisation en thérapie.

15. Composé ou sel pharmaceutiquement acceptable de celui-ci selon l'une quelconque des revendications 1-11 pour une utilisation dans le traitement d'une infection par le VIH.
